# EUROPEAN PATENT SPECIFICATION

(11) **EP 2 152 865 B1**
(45) Date of publication and mention of the grant of the patent: **15.04.2015**
(21) Application number: 07776985.9
(22) Date of filing: 11.05.2007
(51) Int. Cl.: C12N 9/20

(54) **METHODS OF TREATMENT AND PREVENTION OF NEURODEGENERATIVE DISEASES AND DISORDERS**
VERFAHREN ZUR BEHANDLUNG UND VORBEUGUNG NEURODEGENERATIVER KRANKHEITEN UND STÖRUNGEN
MÉTHODES DE TRAITEMENT ET DE PRÉVENTION DE MALADIES ET DE TROUBLES NEURODÉGÉNÉRATIFS

(43) Date of publication of application: 17.02.2010
(73) Proprietor: THOMAS JEFFERSON UNIVERSITY, Philadelphia Pennsylvania 19107 (US); Rowan University, Glassboro, NJ 08028 (US)
(72) Inventor: SHI, Yi, Boothwyn, PA 19061 (US); NAGELE, Robert, Turnersville, NJ 08012 (US)
(74) Representative: Crease, Devanand John
(86) International application number: PCT/US2007/011365
(87) International publication number: WO 2008/140449

(56) References cited:
- WO-A1-97/21676
- WO-A1-03/015786
- WO-A1-03/042218
- WO-A1-2007/029081
- WO-A2-2006/116381
- WO-A2-2006/118805
- US-A- 6 071 899
- US-A1- 2005 033 052
- US-A1- 2006 241 130
- OIJEN ET AL.: 'Lipoprotein-Associated Phospholipase A2 Is Associated with Risk of Dementia' ANNALS OF NEUROLOGY vol. 59, no. 1, January 2006, pages 139 - 144, XP008124236
- SADOWSKI ET AL.: 'Blocking the apoliprotein E/amyloid-B interaction as a potential therapeutic approach for Alzheimer's disease' PNAS vol. 103, no. 49, 05 December 2006, pages 18787 - 18792, XP008124235

## Description

### FIELD OF THE INVENTION

The present invention relates generally to pharmaceutical compositions for use in the treatment and/or prevention of neurodegenerative diseases and disorders, and more particularly to treatment and/or prevention of neurodegenerative disease associated with abnormal blood barrier permeability using agents that inhibit the expression and/or activity of Lp-PLA₂ protein.

### BACKGROUND

Lipoprotein-Associated Phospholipase A₂ (Lp-PLA₂), also previously known in the art as Platelet Activating Factor Acetyl Hydrolase (PAF acetyl hydrolase) is a member of the super family of phospholipase A₂ enzymes that are involved in hydrolysis of lipoprotein lipids or phospholipids. It is secreted by several cells that play a major role in the systemic inflammatory response to injury, including lymphocytes, monocytes, macrophage, T Lymphocytes and mast cells.

During the conversion of LDL to its oxidised form, Lp-PLA₂ is responsible for hydrolysing the sn-2 ester of oxidatively modified phosphatidylcholine to give lysophosphatidylcholine and an oxidatively modified fatty acid. Lp-PLA₂ hydrolyzes the sn2 position of a truncated phospholipid associated with oxidized LDL. As a result, there is a generation of two inflammatory cell homing mediators (non-esterified fatty acids (NEFA) and LYSO PC). Both NEFA and LYSO PCs are chemoattractants for circulating monocytes, play a role in the activation of macrophages and increase oxidative stress as well as affecting the functional and the immediate responses of T lymphocytes. Lp-PLA₂ is bound in humans and pigs to the LDL molecule via lipoprotein B, and once in the arterial wall the oxidized LDL is susceptible to hydrolysis by LpPLA₂.

Both of these products of Lp-PLA₂ action are potent chemoattractants for circulating monocytes. As such, this enzyme is thought to be responsible for the accumulation of cells loaded with cholesterol ester in the arteries, causing the characteristic 'fatty streak' associated with the early stages of atherosclerosis, and inhibition of the Lp-PLA₂ enzyme may be useful in preventing the build up of this fatty streak (by inhibition of the formation of lysophosphatidylcholine), and useful in the treatment of atherosclerosis.

In addition, it is proposed that Lp-PLA₂ plays a direct role in LDL oxidation. This is due to the polyunsaturated fatty acid-derived lipid peroxide products of Lp-PLA₂ action contributing to and enhancing the overall oxidative process. In keeping with this idea, Lp-PLA₂ inhibitors inhibit LDL oxidation. Lp-PLA₂ inhibitors may therefore have a general application in any disorder that involves lipid peroxidation in conjunction with the enzyme activity, for example in addition to conditions such as atherosclerosis and diabetes other conditions such as rheumatoid arthritis, myocardial infarction and reperfusion injury.

The clinical management of numerous neurological disorders has been frustrated by the progressive nature of degenerative, traumatic, or destructive neurological diseases and the limited efficacy and serious side-effects of available pharmacological agents. Conditions such as Huntington's disease, Alzheimer's disease, Parkinson's disease, severe seizure disorders (e.g., epilepsy and familial dysautonomia), have eluded most conventional pharmacological attempts to alleviate or cure the conditions.

Dementia, a progressive brain dysfunction, leads to a gradually increasing restriction of daily activities. The most well-known type of dementia is Alzheimer's disease. It affects about 10% of the population over 65-years old and about 40% of the population over 80-years old. Approximately 4.5 million people in the United States are currently afflicted with this disorder; and the number is expected to rise sharply in the coming years, because this demographic population is the fastest growing segment in our society. By 2050, if no preventative treatments become available, the number of Americans with Alzheimer's disease is expected to triple (from 4.6 to 14 million) and the cost of caring for people with the disease in US will be over $100 billion annually. This epidemic has enormous implications for society, in terms of both human suffering and monetary cost.

Vascular dementia is defined as the loss of cognitive function resulting from ischemic, ischemic-hypoxic, or hemorrhagic brain lesions as a result of cardiovascular diseases and cardiovascular pathologic changes. See, e. g., G. C. Roman, Med. Clin. North. Am., 86, pp. 477-99 (2002). Vascular dementia is a chronic and progressive disorder. The symptoms of vascular dementia include cognitive loss, headaches, insomnia and memory loss. Vascular dementia can be caused by multiple lacunes, and hypoperfusion lesions such as border zone infarcts and ischemic periventricular leukoencephalopathy (Binswanger's disease). See, G. C. Roman, supra. In Asian countries such as China, Japan and Korea, vascular dementia is observed in over 60% of patients with dementia.

Treatment of vascular dementia typically involves control of risk factors (i.e., hypertension, diabetes, high fat diet, smoking, hyperfibrinogenemia, hyperhomocystinemia, orthostatic hypotension, cardiac arrhythmias). See, G. C. Roman, supra. Researchers have also investigated whether hormone replacement therapy and estrogen replacement therapy could delay the onset of dementia in women. See, E. Hogervorst et al., Cochrane Database Syst. Rev., 3, CD003799 (2002). However, such hormone replacement therapy has negative side effects.

Moreover, although aspirin is widely prescribed for vascular dementia, there is very limited evidence that aspirin is actually effective in treating vascular dementia patients. See, P. S. Williams et al., Cochrane Database Syst. Rev., 2, CD001296 (2000). Nimodipine has been implicated as a drug demonstrating short-term benefits in vascular dementia patients, but has not been justified as a long-term anti-dementia drug. See, J. M. Lopez-Arrieta and J. Birks, Cochrane Database Syst. Rev., 3, CD000147 (2002). In addition, clinical efficacy data of piracetam does not support the use of this drug in the treatment of dementia or cognitive impairment. L. Flicker and G. (irimley Evans, Cochrane Database Syst. Rev., 2, CD001011 (2001).

To date, no treatment can prevent vascular dementia or stop Alzheimer's disease. For people in the early and middle stages of the disease, current drugs (Cognex, Aricept, Exelon, Razadyne, Namenda) can reduce some symptoms for a limited time. Since inflammation in the brain can contribute to Alzheimer's disease, nonsteroidal anti-inflammatory drugs (NSAIDs, Vioxx, Aleve, Celebrex) have been tested in clinical trials and showed no benefits.

### SUMMARY

The present invention relates to a pharmaceutical composition comprising 1-(N-(2-(diethylamino)ethyl)-N-(4-(4-trifluoromethylphenyl)benzyl)-aminocarbonylmethyl)-2-(4-fluorobenzyl)thio-5,6-trimethylenepyrimidin-4-one (SB480848) or a pharmaceutically acceptable salt thereof, and a pharmaceutically acceptable carrier for use in the treatment and/or prevention of a neurodegenerative disorder or disease, wherein the neurodegenerative disorder is Vascular Dementia.

Also described are methods for treatment and/or prevention of neurodegenerative disease and disorders by inhibition of Lp-PLA2, for example inhibition of expression and/or activity of Lp-PLA2 protein. Neurodegenerative diseases amenable to treatment and/or prevention by these methods are associated with abnormal blood brain barrier (BBB) function, for example neurodegenerative disease with a permeable BBB, and include for example, but are not limited to, Alzheimer's Disease, Huntington's Disease, Parkinson's Disease, Vascular dementia and the like.

The methods described herein comprise administering to a subject in need of treatment and/or prevention of a neurodegenerative disease, a pharmaceutical composition comprising an agent which inhibits Lp-PLA2, for example an agent which inhibits the expression of Lp-PLA₂ and/or the activity of Lp-PLA₂ protein. It is not intended that the methods be limited to any particular stage of the disease (e.g. early or advanced).

As described herein methods to prevent BBB leakage are provided by inhibition of Lp-PLA₂, for example inhibition of expression of Lp-PLA₂ and/or inhibition of protein activity of Lp-PLA₂. Accordingly, some methods inhibit Lp-PLA₂ by blocking enzyme activity and some methods inhibit Lp-PLA₂ by reducing and/or downregulating the expression of Lp-PLA₂ RNA. Prevention and/or reduction of BBB leakage or BBB permeability may lead to prevention and/or reduction of symptoms associated with neurodegenerative diseases or disorders.

Also described are methods of treating and/or preventing a neurodegenerative disorder or disease in a subject, wherein the methods comprise administering to the subject in need thereof a pharmaceutical composition comprising an agent that inhibits the activity and/or expression of the Lp-PLA₂ protein. Such a neurodegenerative disease or disorder can be a neurodegenerative disease or disorder and can be, for example but without limitation, Alzheimer's Disease, Vascular Dementia, Parkinson's Disease and Huntington's Disease. The neurodegenerative disease or disorder may be associated with an abnormal blood brain barrier. The subject administered an agent that inhibits the activity or expression of the Lp-PLA₂ protein may be a human.

Also described are methods of treating and/or preventing a subject with or at risk of vascular dementia comprising administering to the subject a pharmaceutical composition comprising an agent which inhibits the activity and/or expression of Lp-PLA₂ protein, wherein inhibition of the Lp-PLA₂ protein reduced or stops a symptom of vascular dementia. The vascular dementia may be associated with Alzheimer's disease.

Also described are methods of treating and/or preventing a disease or disorder associated with an abnormal blood brain barrier in a subject in need thereof, comprising administering to the subject a pharmaceutical composition comprising an agent which inhibits the expression and/or activity of the Lp-PLA₂ protein. The abnormal BBB may be a permeable blood brain barrier, and the disease or disorder may be a neurodegenerative disease or disorder. Such neurodegenerative diseases and disorders may be, for example but are not limited to vascular dementia, Alzheimer's disease, Parkinson's disease and Huntington's disease and the like.

Also described are methods of decreasing beta amyloid, and referred to as "Aβ" accumulation in the brain of a subject, comprising administering to the subject a pharmaceutical composition comprising an agent which inhibits the expression and/or activity of Lp-PLA₂ protein, wherein inhibition of the Lp-PLA₂ protein reduces or stops a symptom of beta amyloid accumulation in the brain. The beta amyloid may be Abeta-42.

Where the neurodegenerative disease results in a decline in cognitive function, for example where the neurodegenerative disease is for example, Alzheimer's Disease and/or vascular dementia, the method of treatment and/or prevention of the disease using the methods as described herein can further comprise assessing the cognitive function of the subject after administration of the pharmaceutical composition comprising an agent that inhibits the expression and/or activity of Lp-PL_{A}2.

In the methods as described herein for the treatment and/or prevention of neurodegenerative diseases, and/or treatment and/or prevention of abnormal BBB in a subject, the method can further comprise monitoring the treatment by assessing cerebral blood flow or blood-brain barrier function. Such methods can be performed by one of ordinary skill in the art, for example but are not limited to assessing presence of tau and Aβ-42 in the CSF.

The methods as described herein for the treatment and/or prevention of neurodegenerative diseases are applicable to subjects, for example mammalian subjects. The subject may be a human.

### BRIEF DESCRIPTION OF FIGURES

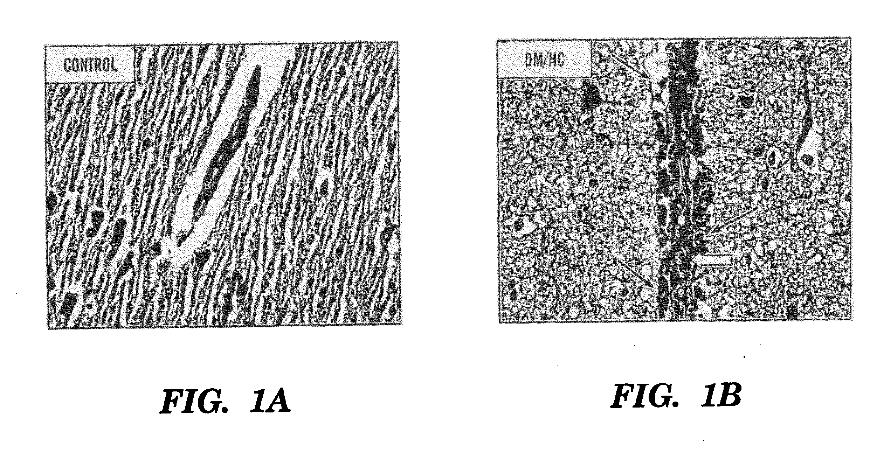

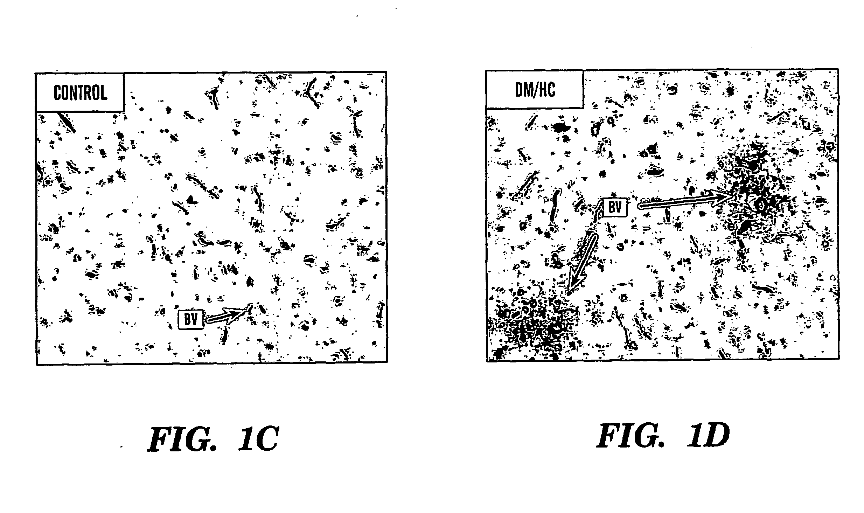

Figure 1 shows evidence of DM/HC-induced hemorrhage and BBB leakage. Panel A shows no hemorrhage in the control brain, as compared to the brain in the DM/HC brain in panel B, with black arrows showing inflammatory cells outside the blood vessel, and white arrow showing increased adhesion of inflammatory cells on the surface of endothelial cells. Panel C shows no vascular permeability and intact BBB in the control animal, as compared to BBB leakage in the brain of DM/HC animal as shown in panel D, with arrows in C and D showing the border of the immunoglobulin (Ig)-positive "leak cloud" surrounding small blood vessels.

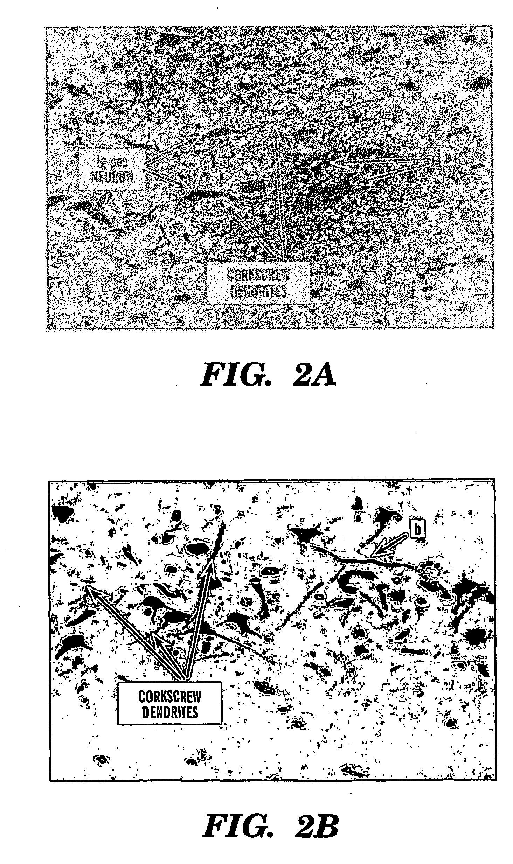

Figure 2 shows that leaked Ig in DM/HC brains binds to neurons and induces dendrite collapse. The Left and Right panels show Ig positive (Ig-pos) neurons with characteristic dendrite collapse as revealed by the "corkscrew morphology" of the dendrite trunks emerging from the neuronal cell body.

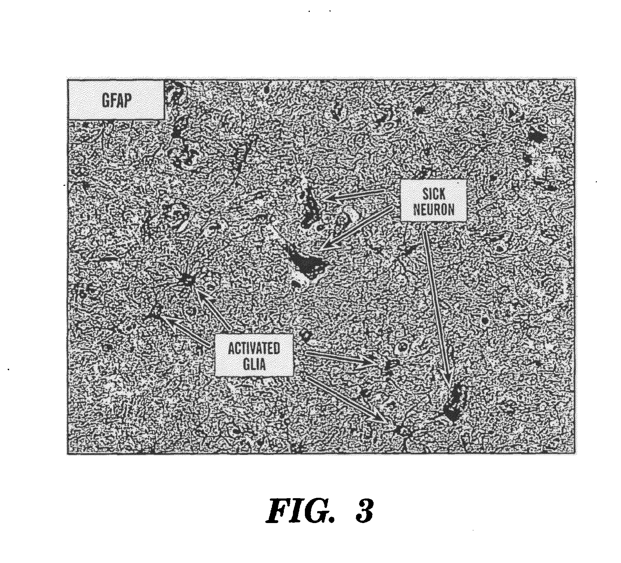

Figure 3 shows evidence of DM/HC-induced astrocyte activation, and shows GFAP immunostaining as a marker for activated astrocytes in the vicinity of "sick neurons" in the brain of DM/HC treated animals.

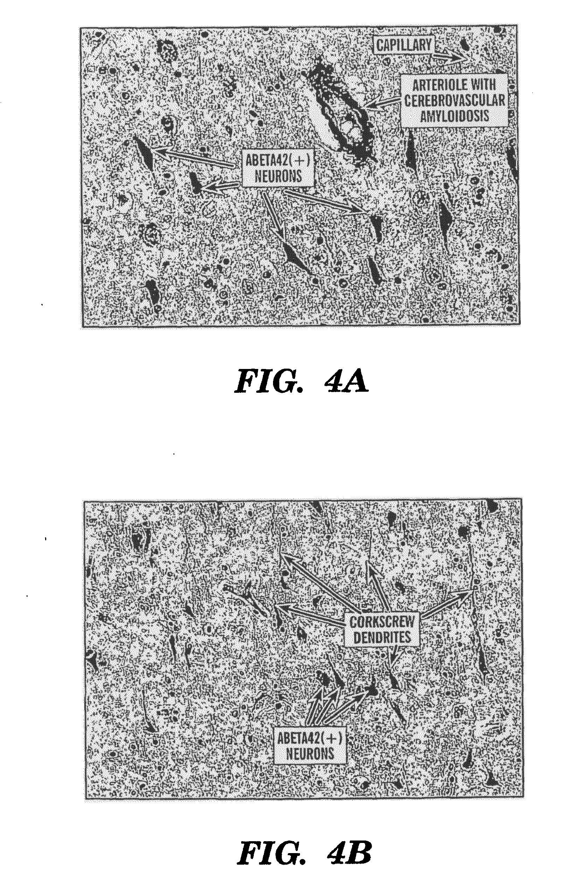

Figure 4 shows evidence of DM/HC-induced accumulation of Abeta-42 in neurons and in the local cerebral microvasculature. Panel A shows neurons that are intensely immunopositive for Abeta-42 and an arteriole exhibiting the deposition of Abeta-42 in the vascular smooth muscle layer ( cerebrovascular amyloidosis), and Panel B shows that neurons immunopositive for Abeta-42 have "corkscrew dendrites" indicative of dendrite retraction.

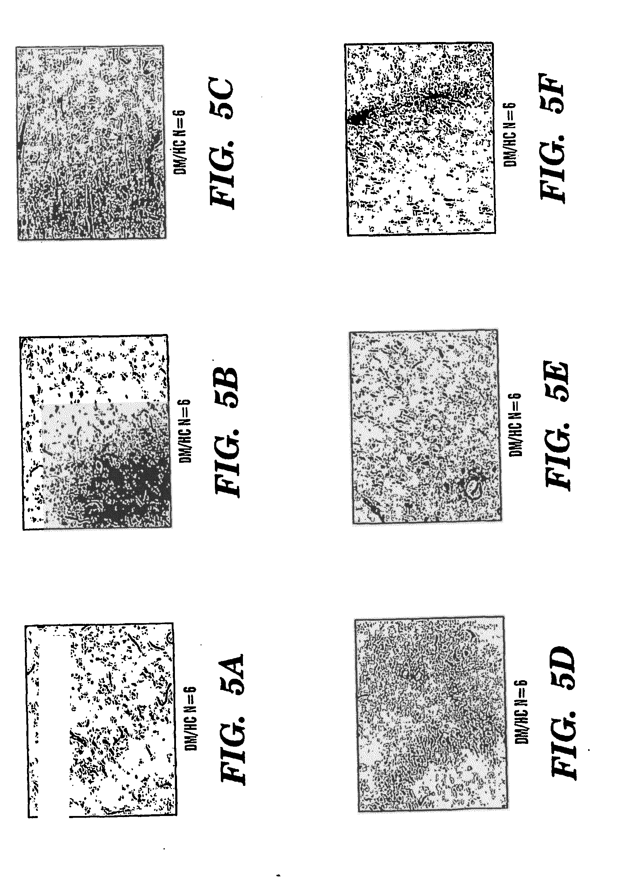

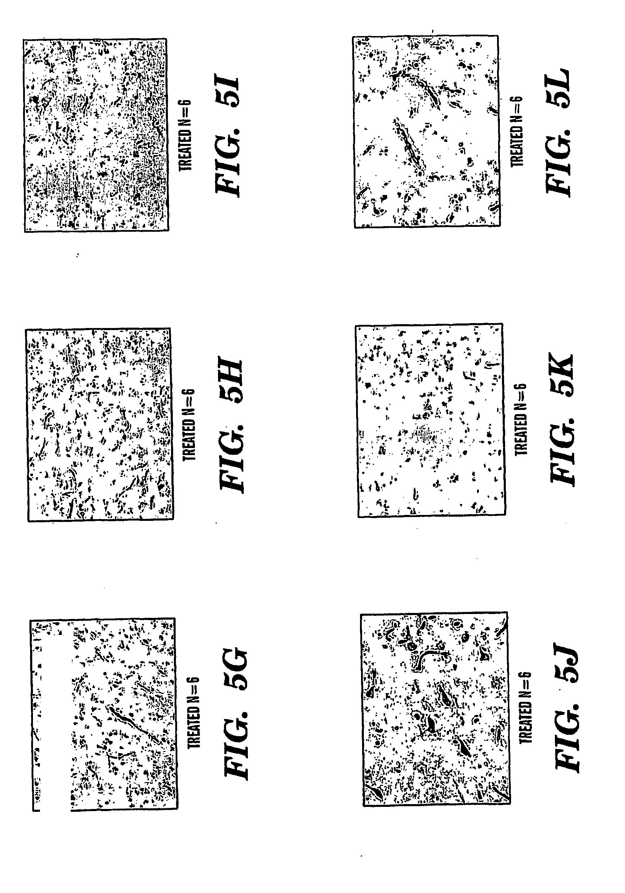

Figure 5 shows the Lp-PLA₂ inhibitor prevents BBB leakage and Ig binding to neurons. Panels A to F are representative images of BBB compromise (permeability) of the brains from DM/HC animals with no treatment (n=6) and panels G to L are representative images of intact BBB of comparable regions in the brain from DM/HC animals treated with an inhibitor of Lp-PLA₂ (n=6). Panels A-F show abundant Ig leaking from the local brain microvasculature into the brain tissue and Ig binding to neurons compared to panels G-L.

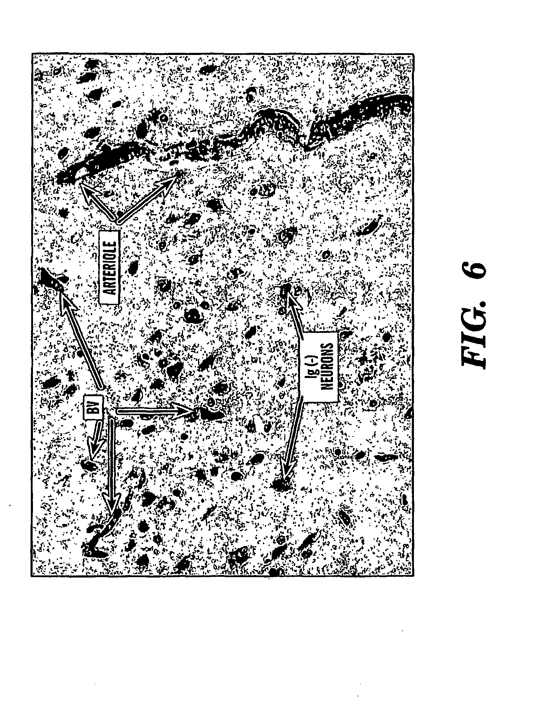

Figure 6 shows the Lp-PLA₂ inhibitor preserves the health of neurons in DM/HC animals by blocking the efflux of plasma components (including Ig) from blood vessels. As a result, Ig is confined to the lumen of the blood vessels in DM/HC animals treated with an inhibitor of Lp-PLA₂, rendering both arteriole walls and neurons immunonegative for Ig.

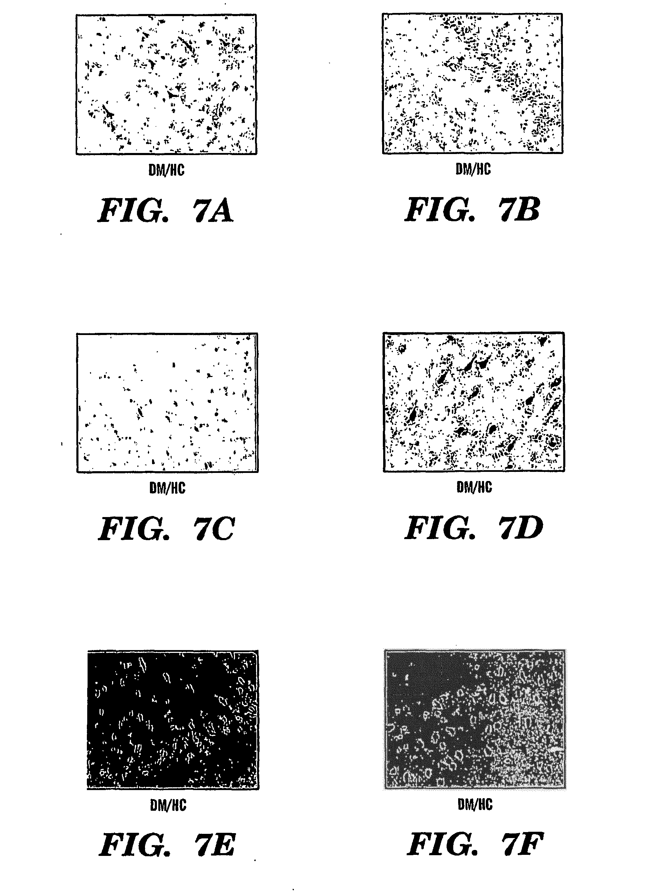

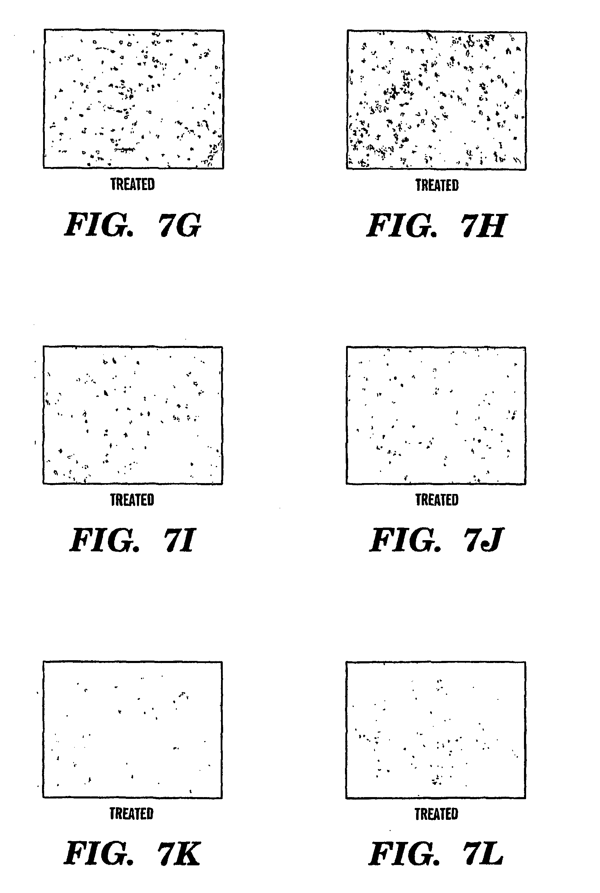

Figure 7 shows Lp-PLA₂ inhibitor prevents Abeta-42 accumulation in neurons. Panels A to F are representative images of Abeta-42 immunostaining of the brains from DM/HC animals with no treatment (*n*=6) and panels G to L are representative images of Abeta-42 immunostaining in comparative regions in the brain from DM/HC animals treated with an inhibitor of Lp-PLA₂ (n=6). As shown in panels A-F, increased immunoreactivity of Abeta-42 shows Abeta-42 binding to and accumulation within neurons as compared to immunonegative neurons for Abeta-42 in panels G-L.

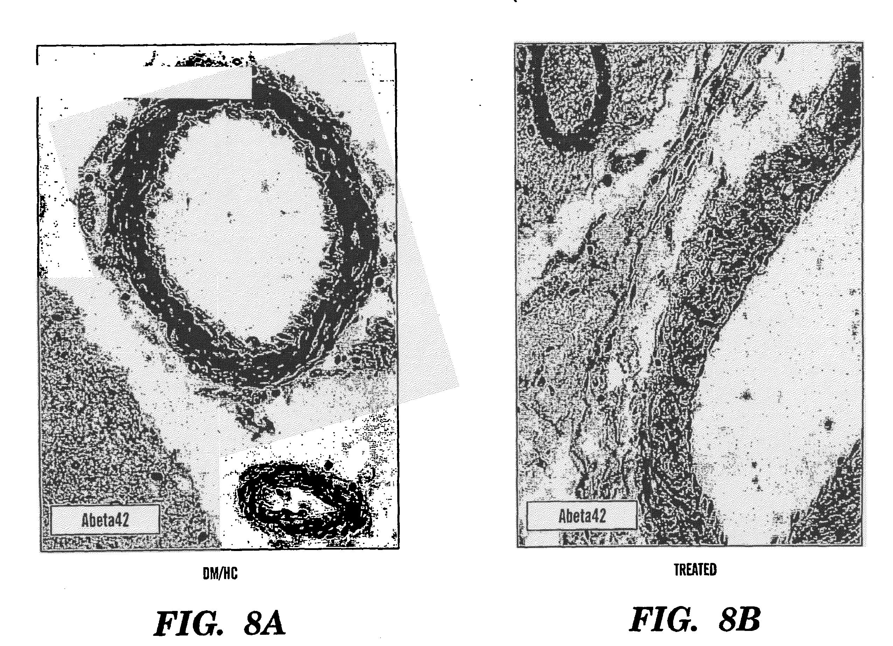

Figure 8 shows the Lp-PLA₂ inhibitor reduced cerebrovascular amyloidosis. Panel A shows Abeta-42 immunostaining in the smooth muscle cell layer of arterioles (i.e., cerebrovascular amyloidosis) in brains from DM/HC animals with no treatment, as compared to Panel B, which shows little or no arterial Abeta-42 immunostaining in comparable regions of the brain from DM/HC animals treated with an inhibitor of Lp-PLA₂.

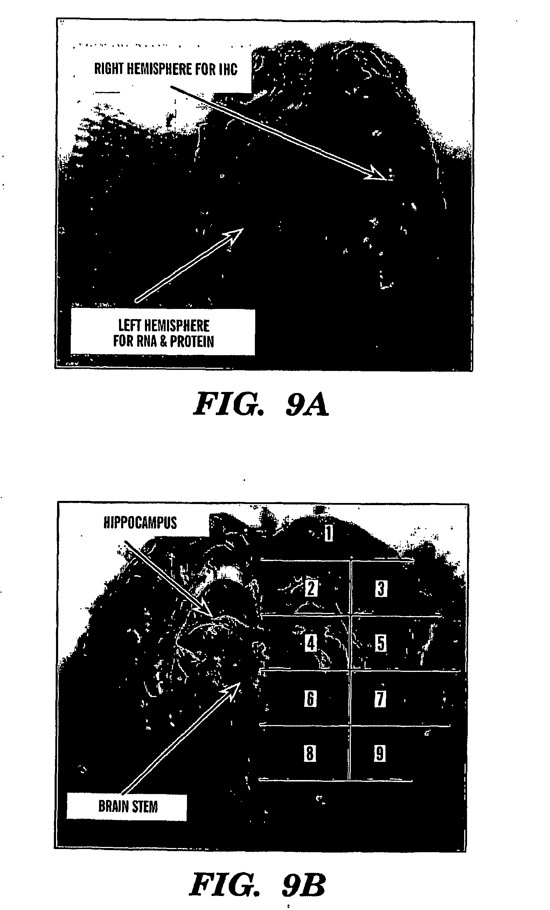

Figure 9 shows a representative image of the porcine brain, showing the full intact brain in panel A and hemisectioned along the midline in panel B with arrows indicating the location of the brain stem and hippocampus, as well as a grid of the brain regions used for immunohistochemical analyses.

### DETAILED DESCRIPTION

As disclosed herein, the inventors have discovered that animals prone to pathological features of vascular dementia, including BBB leakage and permeability and hemorrhage, when treated with an Lp-PLA₂ inhibitor, have been found to exhibit reduced signs and symptoms of BBB permeability and reduced abnormal BBB. For example, animals treated with an Lp-PLA₂ inhibitor showed reduced BBB permeability, less activation of glial cells and less neuronal damage. In particular, the animals treated with an Lp-PLA₂ inhibitor also had less accumulation of amyloid, for example beta-amyloid (or Abeta-42) accumulation in the brain and brain vasculature. Therefore, the inventors have discovered that Lp-PLA₂ inhibitors can be used in the treatment and/or prevention of neurodegenerative diseases and disorders, in particular neurodegenerative diseases and disorders associated with abnormal blood brain barrier function, for example neurodegenerative diseases with BBB permeability or increased BBB permeability. Such diseases include, for example, but are not limited to vascular dementia, Alzheimer's Disease, Huntington's Disease, Parkinson's Disease and the like.

### Definitions

For convenience, certain terms employed in the entire application (including the specification, examples, and appended claims) are collected here. Unless defined otherwise, all technical and scientific terms used herein have the same meaning as commonly understood by one of ordinary skill in the art to which this invention belongs.

The term "neurodegenerative disease" as used herein refers to a varied assortment of central nervous system disorders characterised by gradual and progressive loss of neural tissue and/or neural tissue function. A neurodegenerative disease is a class of neurological disorder or disease, and where the neurological disease is characterized by a gradual and progressive loss of neural tissue, and/or altered neurological function, typically reduced neurological function as a result of a gradual and progressive loss of neural tissue. The neurodegenerative diseases amenable to prevention and/or treatment using the methods as described herein are neurodegenerative diseases whereby there is an abnormal blood brain barrier, for example a permeable blood brain barrier. Examples of neurodegenerative diseases where there is a defective blood brain barrier include for example, but are not limited to Alzheimer's Disease, Huntington's Disease, Parkinson's Disease, vascular dementia and the like.

The term "vascular dementia" is also referred to as "multi-infarct dementia" in the art refers to a group of syndromes caused by different mechanisms all resulting in vascular lesions in the brain. The main subtypes of vascular dementia are, for example vascular mild cognitive impairment, multi-infarct dementia, vascular dementia due to a strategic single infarct (affecting the thalamus, the anterior cerebral artery, the parietal lobes or the cingulate gyrus), vascular dementia due to hemorrhagic lesions, small vessel disease (including, e.g. vascular dementia due to lacunar lesions and Binswanger disease), and mixed Alzheimer's Disease with vascular dementia.

The term "disease" or "disorder" is used interchangeably herein, and refers to any alteration in state of the body or of some of the organs, interrupting or disturbing the performance of the functions and/or causing symptoms such as discomfort, dysfunction, distress, or even death to the person afflicted or those in contact with a person. A disease or disorder can also relate to a distemper, ailing, ailment, malady, disorder, sickness, illness, complaint, inderdisposion or affectation.

The terms "blood-brain barrier" or "BBB" are used interchangeably herein, and are used to refer to the permeability barrier that exists in blood vessels as they travel through the brain tissue that severely restricts and closely regulates what is exchanged between the blood and the brain tissue. The blood brain barrier components include the endothelial cells that form the innermost lining of all blood vessels, the tight junctions between adjacent endothelial cells that are the structural correlate of the BBB, the basement membrane of endothelial cells and the expanded foot processes of nearby astrocytes which cover nearly all of the exposed outer surface of the blood vessel. The BBB prevents most substances in the blood from entering brain tissue, including most large molecules such as Ig, antibodies, complement, albumin and drugs and small molecules.

The term "abnormal BBB" is used to refer to a dysfunctional BBB, for example, where the BBB does not allow transit of molecules that normally transit a functional BBB, for example nutrients and sugars such as glucose. An abnormal BBB can also refer to when the BBB is permeable to molecules that a normally functioning BBB would typically exclude, which is typically referred to "BBB permeability" herein.

The terms "BBB permeability" or "permeable BBB" are commonly referred to by persons in the art as "leaky BBB". The terms are used interchangeably herein to refer to impaired BBB integrity and increased vascular permeability. For example, a permeable BBB allows transit of molecules through the BBB that an intact BBB would normally exclude from the brain tissue, for example, Ig molecules, complement proteins, serum albumin and numerous other proteins. An assay to determine the presence of a permeable BBB can be, for example, to assess the presence of extravascular Ig in the brain tissue which is normally be restricted to the lumen of blood vessels when the BBB is functioning normally (i.e. when the BBB is not permeable).

The term "agent" refers to any entity which is normally not present or not present at the levels being administered in the cell. Agent can be selected from a group comprising: chemicals; small molecules; nucleic acid sequences; nucleic acid analogues; proteins; peptides; aptamers; antibodies; or fragments thereof. A nucleic acid sequence can be RNA or DNA, and can be single or double stranded, and can be selected from a group comprising; nucleic acid encoding a protein of interest, oligonucleotides, nucleic acid analogues, for example peptide-nucleic acid (PNA), pseudo-complementary PNA (pc-PNA), locked nucleic acid (LNA) etc. Such nucleic acid sequences include, for example, but are not limited to, nucleic acid sequence encoding proteins, for example that act as transcriptional repressors, antisense molecules, ribozymes, small inhibitory nucleic acid sequences, for example but are not limited to RNAi, shRNAi, siRNA, micro RNAi (mRNAi), antisense oligonucleotides etc. A protein and/or peptide or fragment thereof can be any protein of interest, for example, but are not limited to: mutated proteins; therapeutic proteins and truncated proteins, wherein the protein is normally absent or expressed at lower levels in the cell. Proteins can also be selected from a group comprising; mutated proteins, genetically engineered proteins, peptides, synthetic peptides, recombinant proteins, chimeric proteins, antibodies, midibodies, minibodies, triabodies, humanized proteins, humanized antibodies, chimeric antibodies, modified proteins and fragments thereof. Alternatively, the agent can be intracellular within the cell as a result of introduction of a nucleic acid sequence into the cell and its transcription resulting in the production of the nucleic acid and/or protein inhibitor of Lp-PLA₂ within the cell. The agent may be any chemical, entity or moiety, including without limitation synthetic and naturally-occurring non-proteinaceous entities. The agent may be a small molecule having a chemical moiety. For example, chemical moieties included unsubstituted or substituted alkyl, aromatic, or heterocyclyl moieties including macrolides, leptomycins and related natural products or analogues thereof. Agents can be known to have a desired activity and/or property, or can be selected from a library of diverse compounds.

The term "inhibiting" as used herein means that the expression or activity of Lp-PLA₂ protein or variants or homologues thereof is reduced to an extent, and/or for a time, sufficient to produce the desired effect. The reduction in activity can be due to affecting one or more characteristics of Lp-PLA₂ including decreasing its catalytic activity or by inhibiting a cofactor of Lp-PLA₂ or by binding to Lp-PLA2 with a degree of avidity that is such that the outcome is that of treating or preventing a neurodegenerative disorder or abnormal BBB such as a permeable BBB. In particular, inhibition of Lp-PLA₂ can be determined using an assay for Lp-PLA₂ inhibition, for example but are not limited to by using the bioassay for Lp-PLA₂ protein as described herein.

As used herein, the term "Lp-PLA₂" refers to the protein target to be inhibited by the methods described herein. Lp-PLA₂ is used interchangeably with Lp-PLA₂ and lipoprotein associated phospholipase A₂, also previously known in the art as Platelet Activating Factor Acetyl Hydrolase (PAF acetyl hydrolase). Human Lp-PLA₂ is encoded by nucleic acid corresponding to accession No: U20157 (SEQ ID NO:1) or Ref Seq ID: NM_005084 (SEQ ID NO:2) or and the human Lp-PLA₂ corresponds to protein sequence corresponding to accession No: NP_005075 (SEQ ID NO:3), which are disclosed in U.S. Patent Application 5,981,252.

As used herein, "gene silencing" or "gene silenced" in reference to an activity of an RNAi molecule, for example a siRNA or miRNA refers to a decrease in the mRNA level in a cell for a target gene by at least about 5%, about 10%, about 20%, about 30%, about 40%, about 50%, about 60%, about 70%, about 80%, about 90%, about 95%, about 99%, about 100% of the mRNA level found in the cell without the presence of the miRNA or RNA interference molecule. The mRNA levels may be decreased by at least about 70%, about 80%, about 90%, about 95%, about 99%, about 100%.

As used herein, the term "RNAi" refers to any type of interfering RNA, including but are not limited to, siRNAi, shRNAi, endogenous microRNA and artificial microRNA. For instance, it includes sequences previously identified as siRNA, regardless of the mechanism of down-stream processing of the RNA (i.e. although siRNAs are believed to have a specific method of in vivo processing resulting in the cleavage of mRNA, such sequences can be incorporated into the vectors in the context of the flanking sequences described herein).

As used herein an "siRNA" refers to a nucleic acid that forms a double stranded RNA, which double stranded RNA has the ability to reduce or inhibit expression of a gene or target gene when the siRNA is present or expressed in the same cell as the target gene, for example Lp-PLA₂. The double stranded RNA siRNA can be formed by the complementary strands. A siRNA may refer to a nucleic acid that can form a double stranded siRNA. The sequence of the siRNA can correspond to the full length target gene, or a subsequence thereof. Typically, the siRNA is at least about 15-50 nucleotides in length (e.g., each complementary sequence of the double stranded siRNA is about 15-50 nucleotides in length, and the double stranded siRNA is about 15-50 base pairs in length, preferably about 19-30 base nucleotides, preferably about 20-25 nucleotides in length, e.g., 20, 21, 22, 23, 24, 25, 26, 27, 28, 29, or 30 nucleotides in length).

As used herein "shRNA" or "small hairpin RNA" (also called stem loop) is a type of siRNA. These shRNAs may be composed of a short, e.g. about 19 to about 25 nucleotide, antisense strand, followed by a nucleotide loop of about 5 to about 9 nucleotides, and the analogous sense strand. Alternatively, the sense strand can precede the nucleotide loop structure and the antisense strand can follow.

The terms "microRNA" or "miRNA" are used interchangeably herein are endogenous RNAs, some of which are known to regulate the expression of protein-coding genes at the posttranscriptional level. Endogenous microRNA are small RNAs naturally present in the genome which are capable of modulating the productive utilization of mRNA. The term artificial microRNA includes any type of RNA sequence, other than endogenous microRNA, which is capable of modulating the productive utilization of mRNA. MicroRNA sequences have been described in publications such as Lim, et al., Genes & Development, 17, p. 991-1008 (2003), Lim et al Science 299, 1540 (2003), Lee and Ambros Science, 294, 862 (2001), Lau et al., Science 294, 858-861 (2001), Lagos-Quintana et al, Current Biology, 12, 735-739 (2002), Lagos Quintana et al, Science 294, 853-857 (2001), and Lagos-Quintana et al, RNA, 9, 175-179 (2003). Multiple microRNAs can also be incorporated into a precursor molecule. Furthermore, miRNA-like stem-loops can be expressed in cells as a vehicle to deliver artificial miRNAs and short interfering RNAs (siRNAs) for the purpose of modulating the expression of endogenous genes through the miRNA and or RNAi pathways.

As used herein, "double stranded RNA" or "dsRNA" refers to RNA molecules that are comprised of two strands. Double-stranded molecules include those comprised of a single RNA molecule that doubles back on itself to form a two-stranded structure. For example, the stem loop structure of the progenitor molecules from which the single-stranded miRNA is derived, called the pre-miRNA (Bartel et al. 2004. Cell 116:281-297), comprises a dsRNA molecule.

The terms "patient", "subject" and "individual" are used interchangeably herein, and refer to an animal, particularly a human, to whom treatment including prophylaxic treatment is provided. The term "subject" as used herein refers to human and non-human animals. The term "non-human animals" and "non-human mammals" are used interchangeably herein includes all vertebrates, e.g., mammals, such as non-human primates, (particularly higher primates), sheep, dog, rodent (e.g. mouse or rat), guinea pig, goat, pig, cat, rabbits, cows, and non-mammals such as chickens, amphibians, reptiles etc. The subject may be human. The subject may be an experimental animal or animal substitute as a disease model.

The term "gene" used herein can be a genomic gene comprising transcriptional and/or translational regulatory sequences and/or a coding region and/or non-translated sequences (e.g., introns, 5'- and 3'- untranslated sequences and regulatory sequences). The coding region of a gene can be a nucleotide sequence coding for an amino acid sequence or a functional RNA, such as tRNA, rRNA, catalytic RNA, siRNA, miRNA and antisense RNA. A gene can also be an mRNA or cDNA corresponding to the coding regions (e.g. exons and miRNA) optionally comprising 5'- or 3' untranslated sequences linked thereto. A gene can also be an amplified nucleic acid molecule produced in vitro comprising all or a part of the coding region and/or 5'- or 3'- untranslated sequences linked thereto.

The term "nucleic acid" or "oligonucleotide" or "polynucleotide" used herein can mean at least two nucleotides covalently linked together. As will be appreciated by those in the art, the depiction of a single strand also defines the sequence of the complementary strand. Thus, a nucleic acid also encompasses the complementary strand of a depicted single strand. As will also be appreciated by those in the art, many variants of a nucleic acid can be used for the same purpose as a given nucleic acid. Thus, a nucleic acid also encompasses substantially identical nucleic acids and complements thereof. As will also be appreciated by those in the art, a single strand provides a probe for a probe that can hybridize to the target sequence under stringent hybridization conditions. Thus, a nucleic acid also encompasses a probe that hybridizes under stringent hybridization conditions.

Nucleic acids can be single stranded or double stranded, or can contain portions of both double stranded and single stranded sequence. The nucleic acid can be DNA, both genomic and cDNA, RNA, or a hybrid, where the nucleic acid can contain combinations of deoxyribo- and ribo- nucleotides, and combinations of bases including uracil, adenine, thymine, cytosine, guanine, inosine, xanthine hypoxanthine, isocytosine and isoguanine. Nucleic acids can be obtained by chemical synthesis methods or by recombinant methods.

A nucleic acid will generally contain phosphodiester bonds, although nucleic acid analogs can be included that can have at least one different linkage, e.g., phosphoramidate, phosphorothioate, phosphorodithioate, or O-methylphosphoroamidite linkages and peptide nucleic acid backbones and linkages. Other analog nucleic acids include those with positive backbones; non-ionic backbones, and non-ribose backbones, including those described in U.S. Pat. Nos. 5, 235,033 and 5, 034,506. Nucleic acids containing one or more non-naturally occurring or modified nucleotides are also included within one definition of nucleic acids. The modified nucleotide analog can be located for example at the 5'-end and/or the 3'-end of the nucleic acid molecule. Representative examples of nucleotide analogs can be selected from sugar- or backbone-modified ribonucleotides. It should be noted, however, that also nucleobase- modified ribonucleotides, i.e. ribonucleotides, containing a non naturally occurring nucleobase instead of a naturally occurring nucleobase such as uridines or cytidines modified at the 5-position, e.g. 5-(2-amino)propyl uridine, 5-bromo uridine; adenosines and guanosines modified at the 8-position, e.g. 8- bromo guanosine; deaza nucleotides, e. g. 7 deaza-adenosine; O- and N- alkylated nucleotides, e.g. N6-methyl adenosine are suitable. The 2' OH- group can be replaced by a group selected from H. OR, R. halo, SH, SR, NH₂, NHR, NR₂ or CN, wherein R is C- C6 alkyl, alkenyl or alkynyl and halo is F. Cl, Br or I. Modifications of the ribose-phosphate backbone can be done for a variety of reasons, e.g., to increase the stability and half- life of such molecules in physiological environments or as probes on a biochip. Mixtures of naturally occurring nucleic acids and analogs can be made; alternatively, mixtures of different nucleic acid analogs, and mixtures of naturally occurring nucleic acids and analogs can be made.

The term "vector" used herein refers to a nucleic acid sequence containing an origin of replication. A vector can be a plasmid, bacteriophage, bacterial artificial chromosome or yeast artificial chromosome. A vector can be a DNA or RNA vector. A vector can be either a self replicating extrachromosomal vector or a vector which integrate into a host genome.

As used herein, the term "treating" includes reducing or alleviating at least one adverse effect or symptom of a condition, disease or disorder associated with permeable BBB and/or vascular dementia and/or Alzheimer's disease. As used herein, the term treating is used to refer to the reduction of a symptom and/or a biochemical marker of Alzheimer's disease by at least 10%. For example but are not limited to, a reduction in a biochemical marker of Alzheimer's disease, for example a reduction in amyloid plaque deposition by 10%, or a reduction in the activation of glial cells, for example a reduction in cells expressing GFAP by 10%, would be considered effective treatments by the methods as described herein. As alternative examples, a reduction in a symptom, for example, a slowing of the rate of memory loss by 10% or a cessation of the rate memory decline, or a reduction in memory loss by 10% or an improvement in memory by 10% would also be considered as effective treatments by the methods as described herein.

The term "effective amount" as used herein refers to the amount of therapeutic agent of pharmaceutical composition to reduce or stop at least one symptom of the disease or disorder, for example a symptom or disorder of a defective BBB or vascular dementia. For example, an effective amount using the methods as described herein would be considered as the amount sufficient to reduce a symptom of the disease or disorder, for example vascular dementia or BBB permeability by at least 10%. An effective amount as used herein would also include an amount sufficient to prevent or delay the development of a symptom of the disease, alter the course of a symptom disease (for example but not limited to, slow the progression of a symptom of the disease), or reverse a symptom of the disease.

As used herein, the terms "administering," and "introducing" are used interchangeably and refer to the placement of the agents that inhibit Lp-PLA₂ as described herein into a subject by a method or route which results in at least partial localization of the agents at a desired site. The compounds can be administered by any appropriate route which results in an effective treatment in the subject.

The term "vectors" is used interchangeably with "plasmid" to refer to a nucleic acid molecule capable of transporting another nucleic acid to which it has been linked. Vectors capable of directing the expression of genes and/or nucleic acid sequence to which they are operatively linked are referred to herein as "expression vectors". In general, expression vectors of utility in recombinant DNA techniques are often in the form of "plasmids" which refer to circular double stranded DNA loops which, in their vector form are not bound to the chromosome. Other expression vectors can be used, for example, but are not limited to, plasmids, episomes, bacteriophages or viral vectors, and such vectors can integrate into the host's genome or replicate autonomously in the particular cell. Other forms of expression vectors known by those skilled in the art which serve the equivalent functions can also be used. Expression vectors comprise expression vectors for stable or transient expression encoding the DNA.

The articles "a" and "an" are used herein to refer to one or to more than one (i.e., to at least one) of the grammatical object of the article. By way of example, "an element" means one element or more than one element.

### Lp-PLA₂: General Information

Lp-PLA₂ is also referred to in the art as aliases Lp-PLA₂, LDL-PLA₂, lipoprotein associated phospholipase A₂, PLA2G7, phospholipase A2 (group VII), or Platelet Activating Factor Acetyl Hydrolase (PAF acetyl hydrolase or PAFAH). Human Lp-PLA₂ is encoded by nucleic acid corresponding to GenBank Accession No: U20157 (SEQ ID NO:1) or Ref Seq ID: NM_005084 (SEQ ID NO:2) and the human Lp-PLA₂ corresponds to protein sequence corresponding to GenBank Accession No: NP_005075 (SEQ ID NO:3), which are disclosed in U.S. Patent 5,981,252.

Phospholipase A₂ enzyme Lipoprotein Associated Phospholipase A₂ (Lp-PLA₂), the sequence, isolation and purification thereof, isolated nucleic acids encoding the enzyme, and recombinant host cells transformed with DNA encoding the enzyme are disclosed in WO 95/00649 (SmithKline Beecham plc). A subsequent publication from the same group further describes this enzyme (Tew D et al, Arterioscler Thromb Vas Biol 1996:16; 591-9) wherein it is referred to as LDL-PLA₂ and later patent application (WO 95/09921, Icos Corporation) and a related publication in Nature (Tjoelker et al, vol 374, 6 April 1995, 549) describe the enzyme PAF-AH which has essentially the same sequence as Lp-PLA₂.

It has been shown that Lp-PLA₂ is responsible for the conversion of phosphatidylcholine to lysophosphatidylcholine, during the conversion of low density lipoprotein (LDL) to its oxidised form. The enzyme is known to hydrolyse the sn-2 ester of the oxidised phosphatidylcholine to give lysophosphatidylcholine and an oxidatively modified fatty acid. Both products of Lp-PLA₂ action are biologically active with lysophosphatidylcholine, in particular having several pro-atherogenic activities ascribed to it including monocyte chemotaxis and induction of endothelial dysfunction, both of which facilitate monocyte-derived macrophage accumulation within the artery wall.

The inventors have discovered that animals prone to disorders characterized by BBB leakage have been found to exhibit an intact BBB and reduced signs of BBB permeability when treated with an Lp-PLA₂ inhibitor. Such animals treated with an inhibitor to Lp-PLA₂ also showed reduced signs of vascular dementia, and reduced accumulation of beta-amyloid in the brain tissue and in the blood vessels coursing through the brain as compared to animals not treated with the inhibitor. Therefore, Lp-PLA₂ inhibitors can be used to treat and/or prevent diseases and disorders with BBB permeability, for example but are not limited to neurodegenerative diseases. Examples of neurodegenerative diseases amenable to the treatment and/or prevention using the methods as described herein include, but are not limited to vascular dementia, Alzheimer's disease, and other neurodegenerative diseases, for example Huntington's Disease and Parkinson's disease.

### Agents that inhibit Lp-PLA₂

The methods described herein relate to the inhibition of Lp-PLA₂. Inhibition may be inhibition of nucleic acid transcripts encoding Lp-PLA₂, for example inhibition of messenger RNA (mRNA). Alternatively, inhibition of Lp-PLA₂ may be inhibition of the expression and/or inhibition of activity of the gene product of Lp-PLA₂, for example the polypeptide or protein of Lp-PLA₂, or isoforms thereof. As used herein, the term "gene product" refers to RNA transcribed from a gene, or a polypeptide encoded by a gene or translated from RNA.

Inhibition of Lp-PLA₂ may be by an agent. One can use any agent, for example but are not limited to nucleic acids, nucleic acid analogues, peptides, phage, phagemids, polypeptides, peptidomimetics, ribosomes, aptamers, antibodies, small or large organic or inorganic molecules, or any combination thereof. Agents useful in methods described herein include agents that function as inhibitors of Lp-PLA expression, for example inhibitors of mRNA encoding Lp-PLA.

Agents useful in the methods as described herein can also inhibit gene expression (i.e. suppress and/or repress the expression of the gene). Such agents are referred to in the art as "gene silencers" and are commonly known to those of ordinary skill in the art. Examples include, but are not limited to a nucleic acid sequence, for an RNA, DNA or nucleic acid analogue, and can be single or double stranded, and can be selected from a group comprising nucleic acid encoding a protein of interest, oligonucleotides, nucleic acids, nucleic acid analogues, for example but are not limited to peptide nucleic acid (PNA), pseudo-complementary PNA (pc-PNA), locked nucleic acids (LNA) and derivatives thereof etc. Nucleic acid agents also include, for example, but are not limited to nucleic acid sequences encoding proteins that act as transcriptional repressors, antisense molecules, ribozymes, small inhibitory nucleic acid sequences, for example but are not limited to RNAi, shRNAi, siRNA, micro RNAi (miRNA), antisense oligonucleotides,etc.

As used herein, agents useful in the described methods as inhibitors of Lp-PLA₂ expression and/or inhibition of Lp-PLA₂ protein function can be any type of entity, for example but are not limited to chemicals, nucleic acid sequences, nucleic acid analogues, proteins, peptides or fragments thereof. The agent may be any chemical, entity or moiety, including without limitation, synthetic and naturally-occurring non-proteinaceous entities. The agent may be a small molecule having a chemical moiety. For example, the chemical moiety may be a pyrimidione-based compound as described herein.

Agents useful in the methods as described herein may be proteins and/or peptides or fragment thereof, which inhibit the gene expression of Lp-PLA₂ or the function of the Lp-PLA₂ protein. Such agents include, for example but are not limited to protein variants, mutated proteins, therapeutic proteins, truncated proteins and protein fragments. Protein agents can also be selected from a group comprising mutated proteins, genetically engineered proteins, peptides, synthetic peptides, recombinant proteins, chimeric proteins, antibodies, midibodies, minibodies, triabodies, humanized proteins, humanized antibodies, chimeric antibodies, modified proteins and fragments thereof.

Alternatively, agents useful in the methods as described herein as inhibitors of Lp-PLA₂ can be a chemical, small molecule, large molecule or entity or moiety, including without limitation synthetic and naturally-occurring non-proteinaceous entities. The agent may be a small molecule having the chemical moieties as described herein.

### Small molecules

Agents that inhibit Lp-PLA₂ may be small molecules. Irreversible or reversible inhibitors of Lp-PLA₂ can be used in the methods described herein.

Irreversible inhibitors of Lp-PLA₂ are disclosed in patent applications WO 96/13484, WO96/19451, WO 97/02242, WO97/217675, WO97/217676, WO 97/41098, and WO97/41099 (SmithKline Beecham plc) and disclose *inter alia* various series of 4-thionyl/sulfinyl/sulfonyl azetidinone compounds which are inhibitors of the enzyme Lp-PLA₂. These are irreversible, acylating inhibitors (Tew et al, Biochemistry, 37, 10087, 1998).

Lp-PLA₂ inhibitors effective in humans are commonly known by persons of ordinary skill and include those undergoing evaluation, for example undergoing pre-clinical and clinical assessment including Phase II clinical trials. A number of applications have been filed and published by SmithKline Beecham and its successor GlaxoSmithKline. A list of relevant published applications assigned to same is: WO01/60805, WO02/30904, WO03/016287, WO00/66567, WO03/042218, WO03/042206, WO03/042179, WO03/041712, WO03/086400, WO03/087088, WO02/30911, WO99/24420, WO00/66566, WO00/68208, WO00/10980, and WO2005/021002. In addition, reference is made to U.S. provisional applications 60/829,328 and 60/829,327, both having been filed 13 October 2006.

Other Lp-PLA₂ inhibitors useful in the methods as described herein are described in published patent applications, for example WO2006063791-A1, WO2006063811-A1, WO2006063812-A1, WO2006063813-A1, all in the name of Bayer Healthcare; and US2006106017-A1 assigned to Korea Res. Inst. Bioscience & Biotechnology. Lp-PLA₂ inhibitors also include known agents, for example but not limited to include the use of statins with Niacin (see www.genengnews.com/news/bnitem.aspx?name=6724568) and fenofibrate (see www.genengnews.com/news/bnitem.aspx?name=14817756&taxid=19).

It is believed that any or all of the compounds disclosed in these documents are useful for prophylaxis or treatment of neurodegenerative diseases and disorders, including, for example, but are not limited to vascular dementia and Alzheimer's disease, and other neurodegenerative diseases as disclosed herein where abnormal BBB occurs. The porcine model of BBB permeability and neurodegenerative disease described herein as exemplified in the Examples can be used by one of ordinary skill in the art to determine which of the described compounds or other inhibitors of Lp-PLA₂, for example antibodies, or RNAi are effective for the treatment or prevention of neurodegenerative diseases or disorders as claimed herein. Agents inhibiting Lp-PLA₂ can be assessed in animal models for effect on alleviating BBB permeability. For example, one can use the porcine model of hyperglycemia and hypercholesterolemia as disclosed in the Examples herein, where the BBB permeability is abnormal, for example the BBB is permeable, in which the BBB permeability can be assessed in the present and absence of inhibitors for Lp-PLA₂ by methods commonly known by persons in the art. Markers for BBB function can be used, for example, as disclosed in U.S. Patent 6,884,591.

Lp-PLA₂ inhibitors as disclosed in U.S. patent 6,649,619 and 7,153,861 (and International Application WO 01/60805) and U.S. patent 7,169,924 (and International Patent Application WO 02/30911), are useful in the methods described herein for the prophylaxis or for the treatment of vascular dementia, for example Alzheimer's disease and/or disorders of BBB permeability. The Lp-PLA₂ inhibitors as disclosed in U.S. publication No. 2005/0033052A1 and International Patent Applications WO 02/30904, WO 03/042218, WO 03/042206, WO03/042179, WO 03/041712, WO 03/086400, and WO 03/87088 are reversible Lp-PLA₂ inhibitors.

**Formula (I)** One can use a group of reversible Lp-PLA₂ inhibitors that are disclosed in international application WO 01/60805, from which arose U.S. patents 6,649,619 and 7,153,861. A narrower group of compounds of interest are those of formula (I) described in WO 01/60805 and claimed in US patents 6,649,619 and 7,153,861, namely: 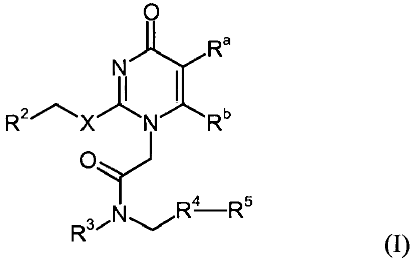 wherein:
R^{a} and R^{b} together with the pyrimidine ring carbon atoms to which they are attached form a fused 5-membered carbocyclic ring;
R² is phenyl, substituted by one to three fluorine atoms;
R³ is methyl or C₍₁₋₃₎alkyl substituted by NR⁸R⁹; or
R³ is Het-C₍₀₋₂₎alkyl in which Het is a 5- to 7- membered heterocyclic ring having N and in which N is unsubstituted or substituted by C₍₁₋₆₎alkyl;
R⁴ and R⁵ together form a 4-(4-trifluoromethylphenyl)phenyl moiety;
R⁸ and R⁹ which can be the same or different are selected from the group consisting of hydrogen, or C₍₁₋₆₎alkyl);
X is S, or a pharmaceutically acceptable salt thereof.

Of even more interest are the following compounds, all within the scope of formula (I) and disclosed in the application and patents noted above:
1-(N-(2-(diethylamino)ethyl)-N-(4-(4-trifluoromethylphenyl)benzyl)-aminocarbonylmethyl)-2-(4-fluorobenzyl)thio-5,6-trimethylenepyrimidin-4-one, used in the pig study described herein;
1-(N-(2-(diethylamino)ethyl)-N-(4-(4-trifluoromethylphenyl)benzyl)-aminocarbonylmethyl)-2-(2,3-difluorobenzyl)thio-5,6-trimethylenepyrimidin-4-one;
1-(N-(2-(diethylamino)ethyl)-N-(4-(4-trifluoromethylphenyl)benzyl)amino-carbonylmethyl)-2-(3,4-difluorobenzyl)thio-5,6-trimethylenepyrimidin-4-one;
1-(N-(2-(diethylamino)ethyl)-N-(4-(4-trifluoromethylphenyl)benzyl)amino-carbonylmethyl)-2-(2,3,4-trifluorobenzyl)thio-5,6-trimethylenepyrimidin-4-one;
1-(N-(2-(diethylamino)ethyl)-N-(4-(4-trifluoromethylphenyl)benzyl)amino-carbonylmethyl)-2-(2-fluorobenzyl)thio-5,6-trimethylenepyrimidin-4-one;
1-(N-methyl-N-(4-(4-trifluoromethylphenyl)benzyl)aminocarbonylmethyl)-2-(4-fluorobenzyl)thio-5,6-trimethylenepyrimidin-4-one;
1-(N-(2-(1-piperidino)ethyl)-N-(4-(4-trifluoromethylphenyl)benzyl)amino-carbonylmethyl)-2-(4-fluorobenzyl)thio-5,6-trimethylenepyrimidin-4-one;
1-(N-(1-ethylpiperidin-4-yl)-N-(4-(4-trifluoromethylphenyl)benzyl)amino-carbonylmethyl)-2-(4-fluorobenzyl)thio-5,6-trimethylenepyrimidin-4-one;
1-(N-(2-ethylamino-2-methylpropyl)-N-(4-(4-trifluoromethylphenyl)benzyl)-aminocarbonylmethyl)-2-(4-fluorobenzyl)thio-5,6-trimethylenepyrimidin-4-one;
N-(2-tert-butylaminoethyl)-N-(4-(4-trifluoromethylphenyl)benzyl)amino-carbonylmethyl)-2-(4-fluorobenzyl)thio-5,6-trimethylenepyrimidin-4-one;
1-(N-(1-methylpiperidin-4-yl)-N-(4-(4-trifluoromethylphenyl)benzyl)-aminocarbonylmethyl)-2-(4-fluorobenzyl)thio-5,6-trimethylenepyrimidin-4-one;
1-(N-(1-isopropylpiperidin-4-yl)-N-(4-(4-trifluoromethylphenyl)benzyl)-aminocarbonylmethyl)-2-(4-fluorobenzyl)thio-5,6-trimethylenepyrimidin-4-one;
1-(N-(1-(2-methoxyethyl)piperidin-4-yl)-N-(4-(4-trifluoromethylphenyl)benzyl)-aminocarbonylmethyl)-2-(4-fluorobenzyl)thio-5,6-trimethylenepyrimidin-4-one;
1-(N-(2-(ethylamino)ethyl)-N-(4-(4-trifluoromethylphenyl)benzyl)-aminocarbonylmethyl)-2-(4-fluorobenzyl)thio-5,6-trimethylenepyrimidin-4-one; or a pharmaceutically acceptable salt of these compounds.

### Methods for preparing these compounds are disclosed in the noted documents.

A second process for making 1-(N-(2-(diethylamino)ethyl)-N-(4-(4-trifluoromethylphenyl)benzyl)-aminocarbonylmethyl)-2-(4-fluorobenzyl)thio-5,6-trimethylenepyrimidin-4-one can be found in application WO 03/016287 (U.S. publication No 20050014793A1).

### Formula (II)

A further group of compounds which can be useful in practicing the described methods are disclosed in WO 02/30911; US patent 7,169,924 corresponds to this international application. The generic formula in that case, represented here as formula (II), is as follows: 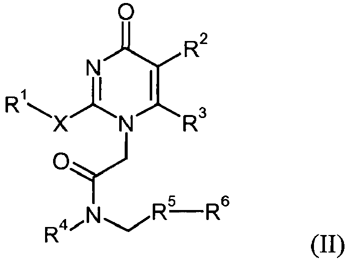 in which:
R¹ is an aryl group, optionally substituted by 1, 2, 3 or 4 substituents which can be the same or different selected from C₍₁₋₆₎alkyl, C₍₁₋₆₎alkoxy, C₍₁₋₆₎alkylthio, hydroxy, halogen, CN, and mono to perfluoro-C₍₁₋₄₎alkyl;
R² is halogen, C₍₁₋₃₎alkyl, C₍₁₋₃₎alkoxy, hydroxyC₍₁₋₃₎alkyl, C₍₁₋₃₎alkylthio, C₍₁₋₃₎alkylsulphinyl, aminoC₍₁₋₃₎alkyl, mono- or di-C₍₁₋₃₎alkylaminoC₍₁₋₃₎alkyl, C₍₁₋₃₎alkylcarbonylaminoC₍₁₋₃₎alkyl, C₍₁₋₃₎alkoxyC₍₁₋₃₎alkylcarbonylaminoC₍₁₋₃₎alkyl, C₍₁₋₃₎alkylsulphonylaminoC₍₁₋₃₎alkyl, C₍₁₋₃₎alkylcarboxy, C₍₁₋₃₎alkylcarboxyC₍₁₋₃₎alkyl, and
R³ is hydrogen, halogen, C₍₁₋₃₎alkyl, or hydroxyC₍₁₋₃₎alkyl; or
R² and R³ together with the pyrimidone ring carbon atoms to which they are attached form a fused 5-or 6-membered carbocyclic ring; or
R² and R³ together with the pyrimidone ring carbon atoms to which they are attached form a fused benzo or heteroaryl ring ring optionally substituted by 1, 2, 3 or 4 substituents which can be the same or different selected from halogen, C₍₁₋₄₎alkyl, cyano, C₍₁₋₆₎alkoxy, C₍₁₋₆₎alkylthio or mono to perfluoro-C₍₁₋₄₎alkyl;
R⁴ is hydrogen, C₍₁₋₆₎alkyl which can be unsubstituted or substituted by 1, 2 or 3 substituents selected from hydroxy, halogen, OR⁷, COR⁷, carboxy, COOR⁷, CONR⁹R¹⁰,
NR⁹R¹⁰, NR⁷COR⁸, mono- or di-(hydroxyC₍₁₋₆₎alkyl)amino and N-hydroxyC₍₁₋₆₎alkyl-N-C₍₁₋₆₎alkylamino; or
R⁴ is Het-C₍₀₋₄₎alkyl in which Het is a 5- to 7- membered heterocyclyl ring comprising N and optionally O or S, and in which N can be substituted by COR⁷, COOR⁷,
CONR⁹R¹⁰, or C₍₁₋₆₎alkyl optionally substituted by 1, 2 or 3 substituents selected from hydroxy, halogen, OR⁷, COR⁷, carboxy, COOR⁷, CONR⁹R¹⁰ or NR⁹R¹⁰, for instance, piperidin-4-yl, pyrrolidin-3-yl;
R⁵ is an aryl or a heteroaryl ring optionally substituted by 1, 2, 3 or 4 substituents which can be the same or different selected from C₍₁₋₆₎alkyl, C₍₁₋₆₎alkoxy, C₍₁₋₆₎alkylthio, arylC₍₁₋₆₎alkoxy, hydroxy, halogen, CN, COR⁷, carboxy, COOR⁷, NR⁷COR⁸, CONR⁹R¹⁰, SO₂NR⁹R¹⁰, NR⁷SO₂R⁸, NR⁹R¹⁰, mono to perfluoro-C₍₁₋₄₎alkyl and mono to perfluoro-C₍₁₋₄₎alkoxy;
R⁶ is an aryl or a heteroaryl ring which is further optionally substituted by 1, 2, 3 or 4 substituents which can be the same or different selected from C₍₁₋₁₈₎alkyl, C₍₁₋₁₈₎alkoxy, C₍₁₋₆₎alkylthio, C₍₁₋₆₎alkylsulfonyl, arylC₍₁₋₆₎alkoxy, hydroxy, halogen, CN, COR⁷, carboxy, COOR⁷, CONR⁹R¹⁰, NR⁷COR⁸, SO₂NR⁹R¹⁰, NR⁷SO₂R⁸, NR⁹R¹⁰, mono to perfluoro-C₍₁₋₄₎alkyl and mono to perfluoro-C₍₁₋₄₎alkoxy, or C₍₅₋₁₀₎alkyl;
R⁷ is hydrogen or C₍₁₋₁₂₎alkyl, for instance C₍₁₋₄₎alkyl (e.g. methyl or ethyl);
R⁸ is hydrogen, OC₍₁₋₆₎alkyl, or C₍₁₋₁₂₎alkyl, for instance C₍₁₋₄₎alkyl (e.g. methyl or ethyl);
R⁹ and R¹⁰ which can be the same or different is each selected from hydrogen, or C₍₁₋₁₂₎alkyl, or R⁹ and R¹⁰ together with the nitrogen to which they are attached form a 5-to 7 membered ring optionally containing one or more further heteroatoms selected from oxygen, nitrogen and sulphur, and optionally substituted by one or two substituents selected from hydroxy, oxo, C₍₁₋₄₎alkyl, C₍₁₋₄₎alkylcarboxy, aryl, e.g. phenyl, or aralkyl, e.g benzyl, for instance morpholine or piperazine; and
X is C₍₂₋₄₎alkylene, optionally substituted by 1,2 or 3 substituents selected from methyl and ethyl, or CH=CH.

All salts of formula (II), as well, can be used in the method of treatment described herein.

Of particular interest are the compounds of formula (II) here, where, as noted in WO 02/30911 for formula (I) there, R¹ can be a phenyl group optionally substituted by 1, 2, 3 or 4 substituents which can be the same or different selected from halo, C₁₋C₆ alkyl, trifluoromethyl or C₁₋C₆ alkoxy. More specifically, phenyl is unsubstituted or substituted by 1, 2, 3 or 4 halogen substituents, particularly, from 1 to 3 fluoro groups, and most particularly, 2,3-difluoro, 2,4-difluoro or 4-fluoro.

A further example of formula (II) here, is where Y is -CH₂CH₂-.

In addition, of interest are compounds of formula (II) where R² is hydrogen, by default, or is halo, C₁₋C₆ alkyl, mono to perfluoro- C₁₋C₄ alkyl, mono to perfluoro C₁₋C4₆ alkoxy, or C₁₋C₆ alkoxy; particularly mono to perfluoro- C₁₋C₄ alkyl, mono to perfluoro-C₁₋C₄ alkoxy, or C₁₋C₆ alkoxy. Of particular interest are the compounds of formula (II) where R² is other than hydrogen, n in (R²)ₙ is 1,2, or 3, and the substitution pattern is meta and/or para, particularly para, i.e. a 4-position substituent. See also those compounds where R² is 4-trifluoromethyl or 4-trifluoromethoxy.

R³ and R⁴ can be the same or different and are methyl, ethyl, *n*-propyl, or *n*-butyl. Of particular interest are those compounds of formula (II) herein where R³ and R⁴ are the same and are methyl, or ethyl; methyl is of particular interest.

R⁵ can be hydrogen, C₍₁₋₆₎ alkyl which is a straight chain, or branched. Of particular interest is methyl, ethyl, propyl, isopropyl, *n*-butyl, *sec*-butyl, *iso*-butyl, *t*-butyl, *n*-pentyl or *n-*hexyl.

It will be appreciated that within the compounds of formula (II) herein there is a further sub-group of compounds in which:
R¹ is phenyl substituted by 2,3 difluoro;
R² and R³, together with the pyrimidine ring carbon atoms to which they are attached, form a fused 5-membered cyclopentenyl ring;
R⁴ is 2-(diethylamino)ethyl;
R⁵ is phenyl;
R⁶ is phenyl substituted by trifluoromethyl at the 4-position, or thien-2-yl substituted by trifluoromethyl in the 5-position; and
X is (CH₂)₂.

Particular compounds of formula (II) herein of interest are:
*N*-(2-diethylaminoethyl)-2-[2-(2-(2,3-difluorophenyl)ethyl)-4-oxo-4,5,6,7-tetrahydro-cyclopentapyrimidin-1-yl]-*N*-(4'-trifluoromethyl-biphenyl-4-ylmethyl)acetamide;
*N*-(2-diethylaminoethyl)-2-[2-(2-(2,3-difluorophenyl)ethyl)-4-oxo-4,5,6,7-tetrahydro-cyclopentapyrimidin-1-yl]-*N-*(4'-trifluoromethyl-biphenyl-4-ylmethyl)acetamide bitartrate;
*N*-(2-ethylamino-2-methyl-propyl)-2-(2-(2-(2,3-difluorophenyl)-ethyl)-4-oxo-4,5,6,7-tetrahydro-cyclopentapyrimidin-1-yl)-*N*-(4'-trifluoromethyl-biphenyl-4-ylmethyl)-acetamide bitartrate;
*N*-(2-*t*-butylaminoethyl)-2-(2-(2-(2,3-difluorophenyl)-ethyl)-4-oxo-4,5,6,7-tetrahydro-cyclopentapyrimidin-1-yl)-*N-*(4'-trifluoromethyl-biphenyl-4-ylmethyl)acetamide bitartrate;
*N*-(1-ethyl-piperidin-4-yl)-2-(2-(2-(2,3-difluorophenyl)-ethyl)-4-oxo-4,5,6,7-tetrahydro-cyclopentapyrimidin-1-yl)-*N*-(4'-trifluoromethyl-biphenyl-4-ylmethyl)acetamide bitartrate;
*N*-(2-diethylaminoethyl)-2-(2-(2-(4-fluoro-2-(trifluoromethyl)phenyt)-ethyl)-4-oxo-4,5,6,7-tetrahydro-cyclopentapyrimidin-1-yl)-*N*-(4'-trifluoromethyl-biphenyl-4-ylmethyl)-acetamide bitartrate;
*N*-(2-diethylaminoethyl)-2-(2-(2-(4-fluoro-3-(trifluoromethyl)phenyl)-ethyl)-4-oxo-4,5,6,7-tetrahydro-cyclopentapyrimidin-1-yl)-*N*-(4'-trifluoromethyl-biphenyl-4-ylmethyl)-acetamide bitartrate;
*N*-(2-diethylaminoethyl)-2-(2-(2-(3-chloro-4-fluorophenyl)-ethyl)-4-oxo-4,5,6,7-tetrahydro-cyclopentapyrimidin-1-yl)-*N*-(4'-trifluoromethyl-biphenyl-4-ylmethyl)acetamide bitartrate;
(+/-)-*N*-(2-diethylaminoethyl)-2-(2phenyl-propyl)-4-oxo-4,5,6,7-tetrahydro-cyclopentapyrimidin-1-yl)-*N*-(4'-trifluoromethyl-biphenyl-4-ylmethyl)acetamide bitartrate;
*N*-(2-diethylaminoethyl)-2-(2-(2-(2,4-difluorophenyl)-ethyl)-4-oxo-4,5,6,7-tetrahydro-cyclopentapyrimidin-1-yl)-*N*-(4'-trifluoromethyl-biphenyl-4-ylmethyl)acetamide bitartrate;
*N*-(2-diethylaminoethyl)-2-(2-(2-(2,5-difluorophenyl)-ethyl)-4-oxo-4,5,6,7-tetrahydro-cyclopentapyrimidin-1-yl)-*N*-(4'-trifluoromethyl-biphenyl-4-ylmethyl)acetamide bitartrate;
*N*-(2-diethylaminoethyl)-2-(2-(2-(3,4-difluorophenyl)-ethyl)-4-oxo-4,5,6,7-tetrahydro-cyclopentapyrimidin-1-yl)-*N-*(4'-trifluoromethyl-biphenyl-4-ylmethyl)acetamide;
*N*-(2-diethylaminoethyl)-2-(2-(2-(2-fluorophenyl)-ethyl)-4-oxo-4,5,6,7-tetrahydro-cyclopentapyrimidin-1-yl)-*N*-(4'-trifluoromethyl-biphenyl-4-ylmethyl)acetamide;
*N*-(2-diethylaminoethyl)-2-(2-(2-(3-fluorophenyl)-ethyl)-4-oxo-4,5,6,7-tetrahydro-cyclopentapyrimidin-1-yl)-*N*-(4'-trifluoromethyl-biphenyl-4-ylmethyl)acetamide bitartrate;
*N*-(2-diethylaminoethyl)-2-(2-(2-(3-chlorophenyl)-ethyl)-4-oxo-4,5,6,7-tetrahydro-cyclopentapyrimidin-1-yl)-*N-*(4'-trifluoromethyl-biphenyl-4-ylmethyl)acetamide;
*N*-(2-diethylaminoethyl)-2-(2-(2-(4-chlorophenyl)-ethyl)-4-oxo-4,5,6,7-tetrahydro-cyclopentapyrimidin-1-yl)-*N*-(4'-trifluoromethyl-biphenyl-4-ylmethyl)acetamide;
*N*-(2-diethylaminoethyl)-2-(2-(2-(4-methylphenyl)-ethyl)-4-oxo-4,5,6,7-tetrahydro-cyclopentapyrimidin-1-yl)-*N*-(4'-trifluoromethyl-biphenyl-4-ylmethyl)acetamide;
*N*-(2-diethylaminoethyl)-2-(2-(2-(4-(trifluoromethyl)phenyl)-ethyl)-4-oxo-4,5,6,7-tetrahydro-cyclopentapyrimidin-1-yl)-*N*-(4'-trifluoromethyl-biphenyl-4-ylmethyl)acetamide;
*N*-(2-diethylaminoethyl)-2-(2-(2-(4-methoxyphenyl)-ethyl)-4-oxo-4,5,6,7-tetrahydro-cyclopentapyrimidin-1-yl)-*N*-(4'-trifluoromethyl-biphenyl-4-ylmethyl)acetamide bitartrate;
*N*-(2-diethylaminoethyl)-2-(2-(2-(4-(trifluoromethoxy)phenyl)-ethyl)-4-oxo-4,5,6,7-tetrahydro-cyclopentapyrimidin-1-yl)-*N*-(4'-trifluoromethyl-biphenyl-4-ylmethyl)acetamide bitartrate;
or the free base of any of the bitartrate salts , or another pharmaceutically acceptable salt.

Further, of interest are compounds of formula (III), disclosed in WO 02/30904: 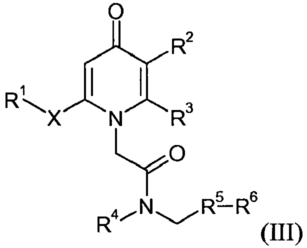 in which:
R¹ is an aryl group, optionally substituted by 1, 2, 3 or 4 substituents which can be the same or different selected from C₍₁₋₆₎alkyl, C₍₁₋₆₎alkoxy, C₍₁₋₆₎alkylthio, hydroxy, halogen, CN, mono to perfluoro-C₍₁₋₄₎alkyl, mono to perfluoro-C₍₁₋₄₎alkoxyaryl, and arylC₍₁₋₄₎alkyl;
R² is halogen, C₍₁₋₃₎alkyl, C₍₁₋₃₎alkoxy, hydroxyC₍₁₋₃₎alkyl, C₍₁₋₃₎alkylthio, C₍₁₋₃₎alkylsulphinyl, aminoC₍₁₋₃₎alkyl, mono- or di-C₍₁₋₃₎alkylaminoC₍₁₋₃₎alkyl, C₍₁₋₃₎alkylcarbonylaminoC₍₁₋₃₎alkyl, C₍₁₋₃₎alkoxyC₍₁₋₃₎alkylcarbonylaminoC₍₁₋₃₎alkyl, C₍₁₋₃₎alkylsulphonylaminoC₍₁₋₃₎alkyl, C₍₁₋₃₎alkylcarboxy, C₍₁₋₃₎alkylcarboxyC₍₁₋₃₎alkyl, and
R³ is hydrogen, halogen, C₍₁₋₃₎alkyl, or hydroxyC₍₁₋₃₎alkyl; or
R² and R³ together with the pyridone ring carbon atoms to which they are attached form a fused 5-or 6-membered carbocyclic ring; or
R² and R³ together with the pyridone ring carbon atoms to which they are attached form a fused benzo or heteroaryl ring optionally substituted by 1, 2, 3 or 4 substituents which can be the same or different selected from halogen, C₍₁₋₄₎alkyl, cyano, C₍₁₋₃₎alkoxyC₍₁₋₃₎alkyl, C₍₁₋₄₎alkoxy or C₍₁₋₄₎alkylthio, or mono to perfluoro-C₍₁₋₄₎alkyl;
R⁴ is hydrogen, C₍₁₋₆₎alkyl which can be unsubstituted or substituted by 1, 2 or 3 substituents selected from hydroxy, halogen, OR⁷, COR⁷, carboxy, COOR⁷, CONR⁹R¹⁰, NR⁹R¹⁰, NR⁷COR⁸, mono- or di-(hydroxyC₍₁₋₆₎alkyl)amino and N-hydroxyC₍₁₋₆₎alkyl-N-C₍₁₋₆₎alkylamino; or
R⁴ is Het-C₍₀₋₄₎alkyl in which Het is a 5- to 7- membered heterocyclyl ring comprising N and optionally O or S, and in which N can be substituted by COR⁷, COOR⁷, CONR⁹R¹⁰, or C₍₁₋₆₎alkyl optionally substituted by 1, 2 or 3 substituents selected from hydroxy, halogen, OR⁷, COR⁷, carboxy, COOR⁷, CONR⁹R¹⁰ or NR⁹R¹⁰, for instance, piperidin-4-yl, pyrrolidin-3-yl;
R⁵ is an aryl or a heteroaryl ring optionally substituted by 1, 2, 3 or 4 substituents which can be the same or different selected from C₍₁₋₆₎alkyl, C₍₁₋₆₎alkoxy, C₍₁₋₆₎alkylthio, arylC₍₁₋₆₎alkoxy, hydroxy, halogen, CN, COR⁷, carboxy, COOR⁷, NR⁷COR⁸, CONR⁹R¹⁰, SO₂NR⁹R¹⁰, NR⁷SO₂R⁸, NR⁹R¹⁰, mono to perfluoro-C₍₁₋₄₎alkyl and mono to perfluoro-C₍₁₋₄₎alkoxy;
R⁶ is an aryl or a heteroaryl ring which is further optionally substituted by 1, 2, 3 or 4 substituents which can be the same or different selected from C_{(1_6)}alkyl, C₍₁₋₆₎alkoxy, C₍₁₋₆₎alkylthio, C₍₁₋₆₎alkylsulfonyl, arylC₍₁₋₆₎alkoxy, hydroxy, halogen, CN, COR⁷, carboxy, COOR⁷, CONR⁹R¹⁰, NR⁷COR⁸, S0₂NR⁹R¹⁰, NR⁷ SO₂R⁸, NR⁹R¹⁰, mono to perfluoro-C₍₁₋₄₎alkyl and mono to perfluoro-C₍₁₋₄₎alkoxy, or C₍₅₋₁₀₎alkyl;
R⁷ and R⁸ are independently hydrogen or C₍₁₋₁₂₎alkyl, for instance C₍₁₋₄₎alkyl (e.g. methyl or ethyl);
R⁹ and R¹⁰ which can be the same or different is each selected from hydrogen, or C₍₁₋₁₂₎alkyl, or R⁹ and R¹⁰ together with the nitrogen to which they are attached form a 5-to 7 membered ring optionally containing one or more further heteroatoms selected from oxygen, nitrogen and sulphur, and optionally substituted by one or two substituents selected from hydroxy, oxo, C₍₁₋₄₎alkyl, C₍₁₋₄₎alkylcarboxy, aryl, e.g. phenyl, or aralkyl, e.g benzyl, for instance morpholine or piperazine; and
X is a C₍₂₋₄₎alkylene group (optionally substituted by 1, 2 or 3 substituents selected from methyl and ethyl), CH=CH, (CH₂)ₙS or (CH₂)ₙO where n is 1, 2 or 3;
or a pharmaceutically acceptable salt thereof.

Of particular interest are those compounds of formula (III) where R² and R³ together with the pyridone ring carbon atoms to which they are attached form a fused benzo or heteroaryl ring optionally substituted by 1, 2, 3 or 4 substituents which can be the same or different selected from halogen, C₍₁₋₄₎alkyl, cyano, C₍₁₋₄₎alkoxy or C₍₁₋₄₎alkylthio, or mono to perfluoro-C₍₁₋₄₎alkyl. Preferably, R¹ is phenyl optionally substituted by halogen, C₍₁₋₆₎alkyl, trifluoromethyl, C₍₁₋₆₎alkoxy, preferably, from 1 to 3 fluoro, more preferably, 2,3-difluoro. Representative examples of R⁴ include piperidin-4-yl substituted at the 1-position by methyl, isopropyl, 1-(2-methoxyethyl), 1-(2-hydroxyethyl), t-butoxycarbonyl or ethoxycarbonylmethyl; ethyl substituted at the 2-position by aminoethyl; 1-ethylpiperidinylmethyl; piperidin-4-yl; 3-diethylaminopropyl; 4-pyrrolidin-1-ylbutyl and 1-ethylpyrrolidin-3-yl. Preferably R⁴ is 1-(2-methoxyethyl)piperidin-4-yl, 1-methylpiperidin-4-yl or 1-ethylpyrrolidin-3-yl. Representative examples of R⁵ include phenyl and pyridyl. Preferably, R⁵ is phenyl. Representative examples of R⁶ include phenyl optionally substituted by halogen, or trifluoromethyl, preferably at the 4-position and hexyl. Preferably, R⁶ is phenyl substituted by trifluoromethyl at the 4-position. Further representative examples of R⁶ include phenyl substituted by 1 or more C₍₁₋₃₎alkyl. Preferably, R⁶ is phenyl substituted by ethyl in the 4-position. Preferably, R⁵ and R⁶ together form a 4-(phenyl)phenyl or a 2-(phenyl)pyridinyl substituent in which the remote phenyl ring can be optionally substituted by halogen or trifluoromethyl, preferably at the 4-position. Preferably X is C₍₂₋₄₎alkylene, more preferably C₍₂₋₃₎alkylene, most preferably, (CH₂)₂, or CH₂S.

It will be appreciated that within the group of compounds comprising formula (III) there is sub-group of compounds in which:
R¹ is phenyl substituted by 2,3-difluoro;
R² and R³, together with the pyridone ring carbon atoms to which they are attached, form a fused benzo or pyrido ring;
R⁴ is 1-(2-methoxyethyl)piperidin-4-yl;
R⁵ and R⁶ together form a 4-(phenyl)phenyl substituent in which the remote phenyl ring is substituted by trifluoromethyl, preferably at the 4-position; and
X is CH₂S or (CH₂)₂.

The following compounds of formula (III) are of interest:
*N*-(2-diethylaminoethyl)-2-[2-(2,3-difluorobenzylthio)-4-oxo-4*H*-quinolin-1-yl]-*N-*(4'-trifluoromethylbiphenyl-4-ylmethyl)acetamide bitartrate;
*N*-(2-diethylaminoethyl)-2-[2-(2-(2,3-difluorophenyl)ethyl)-4-oxo-4*H*-quinolin-1-yl]-*N*-(4'-trifluoromethylbiphenyl-4-ymethyl)acetamide;
*N-*(2-diethylaminoethyl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(2-diethylaminoethyl)-2-[2-(2,3-difluorobenzylthio)-4-oxo-5,6-trimethylene-pyridin-1-yl]-*N*-(4'-trifluoromethylbiphenyl-4-ylmethyl)acetamide bitartrate;
*N*-(1-(2-methoxyethyl)piperidin-4-yl)-2-[2-(2,3-difluorobenzylthio)-4-oxo-4*H-*quinolin-1-yl]-*N*-(4'-trifluoromethylbiphenyl-4-ylmethyl)acetamide;
*N*-(1-methylpiperidin-4-yl)-2-[2-(2,3-difluorobenzylthio)-4-oxo-4*H*-quinolin-1-yl]-*N*-(4'-trifluoromethylbiphenyl-4-ylmethyl)acetamide bitartrate;
*N*-(1-methylpiperidin-4-yl)-2-[2-(2-(2,3-difluorophenyl)ethyl)-4-oxo-4*H-*[1,8]naphthyridin-1-yl]-*N*-(4'-trifluoromethylbiphenyl-4-ylmethyl)acetamide bitartrate;
*N*-(1-(2-methoxyethyl)piperidin-4-yl)-2-[2-(2-(2,3-difluorophenyl)ethyl)-4-oxo-4*H-*[1,8]naphthyridin -1-yl]-*N*-(4'-trifluoromethylbiphenyl-4-ylmethyl)acetamide;
*N*-(1-(2-methoxyethyl)piperidin-4-yl)-2-[2-(2,3-difluorobenzylthio)-4-oxo-4*H-*[1,8]naphthyridin -1-yl]-*N*-(4'-trifluoromethylbiphenyl-4-ylmethyl)acetamide bitartrate;
*N*-(1-ethylpiperidin-4-yl)-2-[2-(2,3-difluorobenzylthio)-4-oxo-4*H*-quinolin-1-yl]-*N-*(4'-ethylbiphenyl-4-ylmethyl)acetamide bitartrate;
*N*-(1-ethylpiperidin-4-yl)-2-[5-(2-(2,3-difluorophenyl)ethyl)-2-methyl-7-oxo-7*H-*thiazolo[4,5-b]pyridin-4-yl]-*N*-(4'-trifluoromethylbiphenyl-4-ylmethyl)acetamide bitartrate;
(±)*N*-(1-ethylpyrrolidin-3-yl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
(±)*N*-(1-ethylpyrrolidin-3-yl)-2-[2-(2,3-difluorobenzylthio)-4-oxo-4*H*-quinolin-1-yl]-*N*-(4'-trifluoromethylbiphenyl-4-ylmethyl)acetamide bitartrate;
*N*-(1-(2-methoxyethyl)piperidin-4-yl)-2-[2-(2,3-difluorobenzylthio)-4-oxo-4*H-*quinolin-1-yl]-*N*-(4'-trifluoromethylbiphenyl-4-ylmethyl)acetamide bitartrate;
*N*-(1-(2-methoxyethyl)piperidin-4-yl)-2-[2-(2,3-difluorobenzylthio)-4-oxo-4*H-*quinolin-1-yl]-*N*-(4'-trifluoromethylbiphenyl-4-ylmethyl)acetamide dihydrochloride;
N-(1-(2-methoxyethyl)piperidin-4-yl)-2-[2-(2,3-difluorobenzylthio)-4-oxo-4*H-*quinolin-1-yl]-*N*-(4'-trifluoromethylbiphenyl-4-ylmethyl)acetamide mono paratoluenesulphonate;
*N*-(1-(2-methoxyethyl)piperidin-4-yl)-2-[2-(2-(2,3-difluorophenyl)ethyl)-4-oxo-4*H-*[1,8]naphthyridin -1-yl]-*N*-(4'-trifluoromethylbiphenyl-4-ylmethyl)acetamide bitartrate;
*N*-(1-(2-methoxyethyl)piperidin-4-yl)-2-[2-(2-(2,3-difluorophenyl)ethyl)-4-oxo-4*H-*[1,8]naphthyridin -1-yl]-*N*-(4'-trifluoromethylbiphenyl-4-ylmethyl)acetamide monohydrochloride;
*N*-(1-(2-methoxyethyl)piperidin-4-yl)-2-[2-(2-(2,3-difluorophenyl)ethyl)-4-oxo-4*H-*[1,8]naphthyridin -1-yl]-*N*-(4'-trifluoromethylbiphenyl-4-ylmethyl)acetamide dihydrochloride;
*N*-(2-diethylaminoethyl)-2-[2-(4-fluorobenzylthio)-4-oxo-4*H*-quinotin-1-yl]-*N*-(4'-trifluoromethylbiphenyl-4-ylmethyl)acetamide bitartrate;
*N*-(2-diethylaminoethyl)-2-[2-(4-fluorobenzylthio)-4-oxo-5,6-trimethylenepyridin-1-yl]-*N*-(4'-trifluoromethyl-biphenyl-4-ylmethyl)acetamide bitartrate;
*N*-(2-diethylaminoethyl)-2-[2-(2,3-difluorobenzylthio)-4-oxo-5,6-trimethylene-pyridin-1-yl]-*N*-(4'-trifluoromethyl-biphenyl-4-ylmethyl)acetamide bitartrate;
*N*-(2-diethylaminoethyl)-2-[2-(4-fluorophenyl)ethyl)-4-oxo-4*H*-quinolin-1-yl]-N-(4'-trifluoromethylbiphenyl-4-ylmethyl)acetamide;
*N*-(2-diethylaminoethyl)-2-[2-(2-(3,4-difluorophenyl)ethyl)-4-oxo-4*H*-quinolin-1-yl]-*N*-(4'-trifluoromethylbiphenyl-4-ylmethyl)acetamide bitartrate;
*N*-(2-diethylaminoethyl)-2-[2-(2-(2-fluorophenyl)ethyl)-4-oxo-4*H*-quinolin-1-yl]-*N-*(4'-trifluoromethylbiphenyl-4-ylmethyl)acetamide bitartrate;
*N*-(2-diethylaminoethyl)-2-[2-(2-(3-chlorophenyl)ethyl)-4-oxo-4*H*-quinolin-1-yl]-*N-*(4'-trifluoromethylbiphenyl-4-ylmethyl)acetamide bitartrate;
*N*-(2-diethylaminoethyl)-2-[2-(2-(2,3-difluorophenyl)ethyl)-4-oxo-4*H-*[1,8]naphthyridin-1-yl)]-*N*-(4'-trifluoromethylbiphenyl-4-ylmethyl)acetamide bitartrate;
*N-*(1-ethylpiperidin-4-yl)-2-[2-(2-(2,3-difluorophenyl)ethyl)-4-oxo-4*H-*[1,8]naphthyridin-1-yl)]-*N*-(4'-trifluoromethylbiphenyl-4-ylmethyl)acetamide;
*N*-(1-ethylpiperidin-4-yl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(2-pyrrolidin-1-ylethyl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(1-isopropylpiperidin-4-yl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(2-piperidin-1-ylethyl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(2-diethylaminoethyl)-2-[2-(2,3-difluorobenzylthio)7-fluoro-4-oxo-4*H*-quinolin-1-yl]-*N*-(4'-trifluoromethylbiphenyl-4-ylmethyl)acetamide bitartrate;
*N*-(2-diethylaminoethyl)-5-[2-(2-(2,3-difluorophenyl)ethyl)-2-methyl-7-oxo-7*H-*thieno[3,2-b]pyridin-4-yl]-*N*-(4'-trifluoromethylbiphenyl-4-ylmethyl)acetamide bitartrate;
*N*-(2-diethylaminoethyl)-2-[2-(2-(2,3-difluorophenyl)ethyl)-5,6-dimethyl-4-oxo-4*H-*pyridin-1-yl]-*N*-(4'-trifluoromethylbiphenyl-4-ylmethyl)acetamide bitartrate;
*N*-(2-diethylaminoethyl)-2-[2-(2-(2,3-difluorophenyl)ethyl)-5-ethyl-4-oxo-4*H-*pyridin-1-yl]-*N*-(4'-trifluoromethylbiphenyl-4-ylmethyl)acetamide bitartrate;
*N*-(1-(2-methoxyethyl)piperidin-4-yl)-2-[2-(2-(2,3-difluorophenyl)ethyl)-4-oxo-4*H-*quinolin-1-yl]-*N*-(4'-trifluoromethylbiphenyl-4-ylmethyl)acetamide bitartrate;
*N-*(1-methylpiperidin-4-yl)-2-[2-(2-(2,3-difluorophenyl)ethyl)-4-oxo-4*H*-quinolin-1-yl]-*N*-(4'-trifluoromethylbiphenyl-4-ymethyl)acetamide bitartrate;
*N*-(2-diethylaminoethyl)-2-[2-(2-(2,3-difluorophenyl)ethyl)-4-oxo-4*H*-thieno[3,4-b]pyridin-1-yl]-*N*-(4'-trifluoromethylbiphenyl-4-ylmethyl)acetamide bitartrate;
*N*-(1-ethylpiperidin-4-yl)-2-[2-(2,3-difluorobenzylthio)-4-oxo-4*H*-quinolin-1-yl]-*N-*(4'-trifluoromethylbiphenyl-4-ylmethyl)acetamide bitartrate;
*N*-(2-pyrrolidin-1-ylethyl)-2-[2-(2,3-difluorobenzylthio)-4-oxo-4*H*-quinolin-1-yl]-*N-*(4'-trifluoromethylbiphenyl-4-ylmethyl)acetamide bitartrate;
*N*-(1-ethylpiperidin-4-yl)-2-[6-(2-(2,3-difluorophenyl)ethyl)-2-methyl-4-oxo-4*H-*pyrazolo[3,4-b]pyridin-7-yl]-*N*-(4'-trifluoromethylbiphenyl-4-ylmethyl)acetamide bitartrate;
*N*-(1-isopropylpiperidin-4-yl)-2-[2-(2,3-difluorobenzylthio)-4-oxo-4*H*-quinolin-1-yl]-*N*-(4'-trifluoromethylbiphenyl-4-ylmethyl)acetamide bitartrate;
*N*-(1-ethylpiperidin-4-ylmethyl)-2-[2-(2-(2,3-difluorophenyl)ethyl)-4-oxo-4*H-*quinolin-1-yl]-*N*-(4'-trifluoromethylbiphenyl-4-ylmethyl)acetamide bitartrate;
*N*-(3-diethylaminopropyl)-2-[2-(2,3-difluorobenzylthio)-4-oxo-4*H*-quinolin-1-yl]-*N-*(4'-trifluoromethylbiphenyl-4-ylmethyl)acetamide bitartrate;
*N*-(4-pyrro-lidin-1-ylbutyl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(3-diethylaminopropyl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N*-(4-pyrrolidin-1-ylbutyl)-2-[2-(2,3-difluorobenzylthio)-4-oxo-4*H*-quinolin-1-yl]-*N-*(4'-trifluoromethylbiphenyl-4-ylmethyl)acetamide bitartrate;
*N*-(1-ethylpiperidin-4-yl)-2-[5-(2,3-difluorobenzylthio)-7-oxo-7*H*-thieno[3,2-b]pyridin-4-yl]-*N*-(4'-trifluoromethylbiphenyl-4-ylmethyl)acetamide bitartrate;
*N*-(2-diethylaminoethyl)-2-[5-(2-(2,3-difluorophenyl)ethyl)-2-methyl-7-oxo-7*H-*thiazolo[4,5-b]pyridin-4-yl]-*N*-(4'-trifluoromethylbiphenyl-4-ylmethyl)acetamide bitartrate;
*N*-(2-diethylaminoethyl)-2-[2-(2,3-difluorobenzylthio)-4-oxo-4*H*-quinolin-1-yl]-*N-*(4'-ethylbiphenyl-4-ylmethyl)acetamide bitartrate;
*N*-(2-diethylaminoethyl)-2-[2-(2-(2,3-difluorophenyl)ethyl)-4-oxo-4*H*-quinolin-1-yl]-*N*-(4'-ethylbiphenyl-4-ylmethyl)acetamide bitartrate;
*N*-(2-diethylaminoethyl)-2-[2-(2,3-difluorobenzylthio)-4-oxo-4*H*-quinolin-1-yl]-*N-*(4'-isopropylbiphenyl-4-ylmethyl)acetamide bitartrate;
*N*-(2-diethylaminoethyl)-2-[2-(2-(2,3-difluorophenyl)ethyl)-4-oxo-4*H*-quinolin-1-yl]-*N*-(4'-isopropylbiphenyl-4-ylmethyl)acetamide bitartrate;
*N*-(2-diethylaminoethyl)-2-[2-(2,3-difluorobenzylthio)-4-oxo-4*H*-[1,8]naphthyridin-1-yl]-*N*-(4'-trifluoromethylbiphenyl-4-ylmethyl)acetamide bitartrate;
*N*-(1-ethylpiperidin-4-yl)-2-[2-(2,3-difluorobenzylthio)-4-oxo-4*H*-[1,8]naphthyridin-1-yl]-*N*-(4'-trifluoromethylbiphenyl-4-ylmethyl)acetamide bitartrate;
*N*-(2-diethylaminoethyl)-2-[2-(2-(2,3-difluorophenyl)ethyl)-4-oxo-4*H*-quinolin-1-yl]-*N*-(4'-methylbiphenyl-4-ylmethyl)acetamide bitartrate;
*N*-(2-diethylaminoethyl)-2-[2-(2,3-difluorobenzylthio)-4-oxo-4*H*-quinolin-1-yl]-*N-*(4'-methylbiphenyl-4-ylmethyl)acetamide bitartrate;
*N*-(1-ethoxycarbonylmethylpiperidin-4-yl)-2-[2-(2-(2,3-difluorophenyl)ethyl)-4-oxo-4*H*-quinolin-1-yl]-*N*-(4'-trifluoromethylbiphenyl-4-ylmethyl)acetamide bitartrate;
*N-*(1-isopropylpiperidin-4-yl)-2-[2-(2-(2,3-difluorophenyl)ethyl)-4-oxo-4*H-*[1,8]naphthyridin-1-yl]-*N*-(4'-trifluoromethylbiphenyl-4-ylmethyl)acetamide bitartrate;
*N*-(2-diethylaminoethyl)-2-[2-(2,3-difluorobenzylthio)-4-oxo-4*H*-quinolin-1-yl]-*N-*(3',4'-dimethylbiphenyl-4-ylmethyl)acetamide bitartrate;
*N*-(1-(t-butoxycarbonyl)piperidin-4-yl)-2-[2-(2-(2,3-difluorophenyl)ethyl)-4-oxo-4*H*-[1,8]naphthyridin-1-yl]-*N*-(4'-trifluoromethylbiphenyl-4-ylmethyl)acetamide bitartrate;
*N*-(2-diethylaminoethyl)-2-[2-(2,3-difluorobenzylthio)-4-oxo-4*H*-quinolin-1-yl]-*N-*(3',4'-difluorobiphenyl-4-ylmethyl)acetamide bitartrate;
*N*-(2-diethylaminoethyl)-2-[6-(2,3-difluorobenzylthio)-4-oxo-4*H*-thieno-[2,3-b]pyridin-7-yl]-*N*-(4'-trifluoromethylbiphenyl-4-ylmethyl)acetamide bitartrate;
*N*-(1-methylpiperidin-4-yl)-2-[2-(2,3-difluorobenzylthio)-4-oxo-4*H-*[1,8]naphthyridin-1-yl]-*N*-(4'-trifluoromethylbiphenyl-4-ylmethyl)acetamide bitartrate;
*N-*(1-ethylpiperidin-4-yl)-2-[2-(2-(2,3,4-trifluorophenylethyl)-4-oxo-4H-quinolin-1-yl]-N-(4'-trifluoromethylbiphenyl-4-ylmethyl)acetamide bitartrate;
*N*-(2-diethylaminoethyl)-2-[6-(2,3-difluorobenzylthio)-2-methyl-4-oxo-2,4-dihydro-pyrazolo[3,4-b]pyridin-7-yl]-*N*-(4'-trifluoromethylbiphenyl-4-ylmethyl)acetamide bitartrate;
*N*-(1-ethylpiperidin-4-yl)-2-[6-(2-(2,3-difluorophenyl)ethyl)-2-ethyl-4-oxo-2,4-dihydropyrazolo[3,4-b]pyridin-7-yl]-*N*-(4'-trifluoromethylbiphenyl-4-ylmethyl)acetamide bitartrate;
*N-*(1-ethylpiperidin-4-yl)-2-[6-(2-(2,3-difluorophenyl)ethyl)-2-isopropyl-4-oxo-2,4-dihydropyrazolo[3,4-b]pyridin-7-yl]-*N*-(4'-trifluoromethylbiphenyl-4-ylmethyl)acetamide bitartrate;
*N*-(1-ethylpiperidin-4-yl)-2-[2-(2-(2,3-difluorophenyl)ethyl)-4-oxo-4*H*-quinolin-1-yl]-*N*-(4'-ethylbiphenyl-4-ylmethyl)acetamide bitartrate;
*N-*(1-isopropylpiperidin-4-yl)-2-[5-(2-(2,3-difluorophenyl)ethyl)-2-methyl-7-oxo-7*H*-thiazolo[4,5-b]pyridin-4-yl]-*N*-(4'-trifluoromethylbiphenyl-4-ylmethyl)acetamide bitartrate;
*N-*(1-(2-methoxyethyl)piperidin-4-yl)-2-[5-(2-(2,3-difluorophenyl)ethyl)-2-methyl-7-oxo-7*H*-thiazolo[4,5-b]pyridin-4-yl]-*N*-(4'-trifluoromethylbiphenyl-4-ylmethyl)acetamide bitartrate;
*N*-(1-ethylpiperidin-4-yl)-2-[2-(2-(2,3-difluorophenyl)ethyl)-4-oxo-5,6-trimethylene-pyridin-1-yl]-*N*-(4'-trifluoromethylbiphenyl-4-ylmethyl)acetamide bitartrate;
*N*-(1-methylpiperidin-4-yl)-2-[2-(2-(2,3-difluorophenyl)ethyl)-4-oxo-5,6-trimethylenepyridin-1-yl]-*N*-(4'-trifluoromethylbiphenyl-4-ylmethyl)acetamide bitartrate;
*N*-(1-(2-methoxyethyl)piperidin-4-yl)-2-[2-(2-(2,3-difluorophenyl)ethyl)-4-oxo-5,6-trimethylenepyridin-1-yl]-*N*-(4'-trifluoromethylbiphenyl-4-ylmethyl)acetamide bitartrate;
*N*-(1-isopropylpiperidin-4-yl)-2-[2-(2-(2,3-difluorophenyl)ethyl)-4-oxo-5,6-trimethylenepyridin-1-yl]-*N*-(4'-trifluoromethylbiphenyl-4-ylmethyl)acetamide bitartrate;
*N*-(1-ethylpiperidin-4-yl)-2-[5-(2-(2,3-difluorophenyl)ethyl)-2-methyl-7-oxo-2,7-dihydropyrazolo[4,3-b]pyridin-4-yl]-*N*-(4'-trifluoromethylbiphenyl-4-ylmethyl)acetamide bitartrate;
*N*-(1-ethylpiperidin-4-yl)-2-[5-(2-(2,3-difluorophenyl)ethyl)-1-methyl-7-oxo-1,7-dihydropyrazolo[4,3-b]pyridin-4-yl]-*N*-(4'-trifluoromethylbiphenyl-4-ylmethyl)acetamide bitartrate;
*N*-(1-ethylpiperidin-4-yl)-2-[2-(2-(2,3-difluorophenyl)ethyl)-4-oxo-5,6-trimethylene-pyridin-1-yl]-*N*-(4'-trifluoromethylbiphenyl-4-ylmethyl)acetamide bitartrate;
*N-*(1-ethylpiperidin-4-yl)-2-[2-(2-(2,3-difluorophenyl)ethyl)-7-methyl-4-oxo-4*H-*[1,8]naphthyridin-1-yl]-*N*-(4'-trifluoromethylbiphenyl-4-ylmethyl)acetamide bitartrate;
*N*-(2-diethylaminoethyl)-2-[2-(2-(2,3-difluorophenyl)ethyl)-7-methyl-4-oxo-4*H-*[1,8]naphthyridin-1-yl]-*N*-(4'-trifluoromethylbiphenyl-4-ylmethyl)acetamide bitartrate;
*N*-(1-methylpiperidin-4-yl)-2-[2-(2-(2,3-difluorophenyl)ethyl)-7-methyl-4-oxo-4*H-*[1,8]naphthyridin-1-yl]-*N*-(4'-trifluoromethylbiphenyl-4-ylmethyl)acetamide bitartrate;
*N-*(1-isopropylpiperidin-4-yl)-2-[2-(2-(2,3-difluorophenyl)ethyl)-7-methyl-4-oxo-4*H*-[1,8]naphthyridin-1-yl]-*N*-(4'-trifluoromethylbiphenyl-4-ylmethyl)acetamide bitartrate;
*N*-(1-(2-methoxyethyl)piperidin-4-yl)-2-[2-(2-(2,3-difluorophenyl)ethyl)-7-methyl-4-oxo-4*H*-[1,8]naphthyridin-1-yl]-*N*-(4'-trifluoromethylbiphenyl-4-ylmethyl)acetamide bitartrate;
*N*-(1-methylpiperidin-4-yl)-2-[2-(2,3-difluorobenzylthio)-4-oxo-5,6-trimethylene-pyridin-1-yl]-*N*-(4'-trifluoromethylbiphenyl-4-ylmethyl)acetamide bitartrate;
*N*-(1-ethylpiperidin-4-yl)-2-[2-(2,3-difluorobenzylthio)-4-oxo-5,6-trimethylene-pyridin-1-yl]-*N*-(4'-trifluoromethylbiphenyl-4-ylmethyl)acetamide bitartrate;
*N*-(1-isopropylpiperidin-4-yl)-2-[2-(2,3-difluorobenzylthio)-4-oxo-5,6-trimethylene-pyridin-1-yl]-*N*-(4'-trifluoromethylbiphenyl-4-ylmethyl)acetamide bitartrate;
*N*-(1-(2-methoxyethyl)piperidin-4-yl)-2-[2-(2,3-difluorobenzylthio)-4-oxo-5,6-trimethylenepyridin-1-yl]-*N*-(4'-trifluoromethylbiphenyl-4-ylmethyl)acetamide bitartrate;
*N*-(2-diethylaminoethyl)-2-[2-(2-(2,3-difluorophenyl)ethyl)-4-oxo-5,6-tetramethylenepyridin-1-yl]*-N*-(4'-trifluoromethylbiphenyl-4-ylmethyl)acetamide bitartrate;
*N*-(1-methylpiperidin-4-yl)-2-[2-(2,3-difluorobenzylthio)-4-oxo-4*H*-quinolin-1-yl]-*N*-(4'-chlorobiphenyl-4-ylmethyl)acetamide bitartrate;
*N*-(1-methylpiperidin-4-yl)-2-[2-(2-(2,3-difluorophenyl)ethyl)-4-oxo-4*H*-quinolin-1-yl]-*N*-(4'-chlorobiphenyl-4-ylmethyl)acetamide bitartrate;
N-(1-ethylpiperidin-4-yl)-2-[2-(2-(2,3-difluorophenyl)ethyl)-4-oxo-4*H*-quinolin-1-yl]-*N*-(4'-chlorobiphenyl-4-ylmethyl)acetamide bitartrate;
*N-*(1-(2-methoxyethyl)piperidin-4-yl)-2-[2-(2-(2,3-difluorophenyl)ethyl)-4-oxo-4*H-*quinolin-1-yl]-*N*-(4'-chlorobiphenyl-4-ylmethyl)acetamide bitartrate;
*N*-(1-isopropylpiperidin-4-yl)-2-[2-(2-(2,3-difluorophenyl)ethyl)-4-oxo-4*H*-quinolin-1-yl]-*N*-(4'-chlorobiphenyl-4-ylmethyl)acetamide bitartrate;
*N*-(2-diethylaminoethyl)-2-[6-(2-(2,3-difluorophenyl)ethyl)-2-methyl-4-oxo-4*H-*pyrazolo[3,4-b]pyridin-7-yl]-*N*-(4'-trifluoromethylbiphenyl-4-ylmethyl)acetamide bitartrate;
*N-*(1-(t-butoxycarbonyl)piperidin-4-yl)-2-[2-(2,3-difluorobenzylthio)-4-oxo-4*H-*[1,8]naphthyridin-1-yl]-*N*-(4'-trifluoromethylbiphenyl-4-ylmethyl)acetamide;
*N-*(1-ethylpiperidin-4-yl)-2-[6-(2-(2,3-difluorophenyl)ethyl)-2-(2-methoxyethyl)-4-oxo-4*H*-pyrazolo[3,4-b]pyridin-7-yl]-*N*-(4'-trifluoromethylbiphenyl-4-ylmethyl)acetamide bitartrate;
*N*-(2-diethylaminoethyl)-2-[4-oxo-2-(2-(2,3,4-trifluorophenyl)ethyl)-4H-quinolin-1-yl]-*N*-(4'-trifluoromethyl-biphenyl-4-ylmethyl)acetamide bitartrate;
*N*-(2-diethylaminoethyl)-2-[2-(2-(2,4-difluorophenyl)ethyl)-4-oxo-4*H*-quinolin-1-yl]-*N*-(4'-trifluoromethylbiphenyl-4-ylmethyl)acetamide bitartrate;
*N*-(2-diethylaminoethyl)-2-[2-(2-(3-fluorophenyl)ethyl)-4-oxo-4*H*-quinolin-1-yl]-*N-*(4'-trifluoromethylbiphenyl-4-ylmethyl)acetamide bitartrate;
*N*-(piperidin-4-yl)-2-[2-(2,3-difluorobenzylthio)-4-oxo-4*H*-quinolin-1-yl]-*N*-(4'-trifluoromethylbiphenyl-4-ylmethyl)acetamide bitartrate;
*N*-(piperidin-4-yl)-2-[2-(2-(2,3-difluorophenyl)ethyl)-4-oxo-4*H*-quinolin-1-yl]-*N-*(4'-trifluoromethylbiphenyl-4-ylmethyl)acetamide bitartrate;
*N*-(piperidin-4-yl)-2-[2-(2-(2,3-difluorophenyl)ethyl)-4-oxo-5,6-trimethylenepyridin-1-yl]-*N*-(4'-trifluoromethylbiphenyl-4-ylmethyl)acetamide bitartrate;
*N*-(piperidin-4-yl)-2-[2-(2-(2,3-difluorophenyl)ethyl)-4-oxo-4*H*-[1,8]naphthyridin-1-yl]-*N*-(4'-trifluoromethylbiphenyl-4-ylmethyl)acetamide bitartrate;
*N-*(piperidin-4-yl)-2-[2-(2,3-difluorobenzylthio)-4-oxo-4*H*-[1,8]naphthyridin-1-yl]-*N*-(4'-trifluoromethylbiphenyl-4-ylmethyl)acetamide trifluoroacetate;
*N*-(2-ethylaminoethyl)-2-[2-(2,3-difluorobenzylthio)-4-oxo-4*H*-quinolin-1-yl]-*N*-(4'-trifluoromethylbiphenyl-4-ylmethyl)acetamide;
*N*-(2-ethylaminoethyl)-2-[2-(2-(2,3-difluorophenyl)ethyl)-4-oxo-4*H*-quinolin-1-yl]-*N*-(4'-trifluoromethylbiphenyl-4-ylmethyl)acetamide;
*N-*(1-(2-hydroxyethyl)piperidin-4-yl)-2-[2-(2-(2,3-difluorophenyl)ethyl)-4-oxo-4*H-*quinolin-1-yl]-*N*-(4'-trifluoromethylbiphenyl-4-ylmethyl)acetamide bitartrate; or the free base thereof, or another pharmaceutically acceptable salt.

### Formula (IV)

Also of interest are compounds of formula (IV) 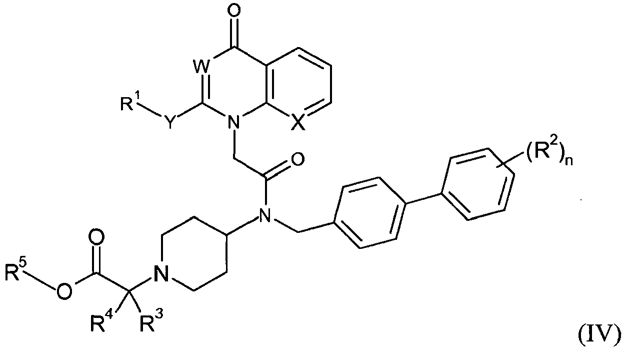 wherein:
R¹ is an aryl group, unsubstituted or substituted by 1, 2, 3 or 4 substituents which can be the same or different selected from the group consisting of C₁₋C₆ alkyl, C₁₋C₆ alkoxy, C₁₋C₆ alkylthio, aryl C₁₋C₆ alkoxy, hydroxy, halo, CN, COR⁶, COOR⁶, NR⁶COR⁷, CONR⁸R⁹, SO₂NR⁸R⁹, NR⁶SO₂R⁷, NR⁸R⁹, halo C₁₋C₄ alkyl, and halo C₁₋C₄ alkoxy;
W is CH and X is N, or W is N and X is CH, W and X are both CH, or W and X are N;
Y is C₂-C₄alkyl,
R² is hydrogen, C₁₋C₆ alkyl, C₁₋C₆ alkoxy, C₁₋C₆ alkylthio, aryl C₁₋C₆ alkoxy, hydroxy, halo, CN, COR⁶, carboxy, COOR⁶, NR⁶COR⁷, CONR⁸R⁹, SO₂NR⁸R⁹, NR⁶SO₂R⁷, NR⁸R⁹, mono to perfluoro- C₁₋C₆ alkyl, or mono to perfluoro- C₁₋C₆ alkoxy;
n is 0-5;
R³ is C₁₋C₄ alkyl;
R⁴ is C₁₋C₄ alkyl;
R⁵ is hydrogen, C₁₋C₁₀ alkyl, C₂₋C₁₀ alkenyl, C₂₋C₁₀ alkynyl, halo C₁₋C₄ alkyl, C₃-C₈ cycloalkyl, C₃-C₈ cycloalkyl, C₃-C₈ cycloalkyl C₁₋C₄ alkyl, C₅-C₅cycloalkenyl, C₅-C₈cycloalkenyl C₁₋C₄ alkyl, 3-8-membered heterocycloalkyl, 3-8-membered heterocycloalkyl C₁₋C₄ alkyl, C₆-C₁₄ aryl, C₆-C₁₄ aryl C₁₋C₁₀ alkyl, heteroaryl, or heteroaryl C₁₋C₁₀alkyl; wherein each group is optionally one or more times by the same and/or a different group which is C₁₋C₆ alkoxy, C₁₋C₆ alkylthio, aryl C₁₋C₆ alkoxy, hydroxy, halo, CN, NR⁸R⁹, or halo C₁₋C₄ alkoxy
R⁶ and R⁷ are independently hydrogen or C₁₋C₁₀ alkyl;
R^{g} and R⁹ are the same or different and are hydrogen or C₁₋C₁₀ alkyl, or R⁹ and R¹⁰ together with the nitrogen to which they are attached form a 5- to 7 membered ring optionally containing one or more further heteroatoms selected from oxygen, nitrogen and sulphur, and optionally substituted by one or two substituents selected from the group consisting of hydroxy, oxo, C₁₋C₄ alkyl, C₁₋C₄ alkylcarboxy, aryl, and aryl C₁₋C₄ alkyl;
or a pharmaceutically acceptable salt thereof.

Without intending to exclude any defined substituents and/or their recited radicals from the scope of formula (IV), the following R groups and the associated radicals are of particular interest:

As regards R¹, it can be an phenyl group optionally substituted by 1, 2, 3 or 4 substituents which can be the same or different selected from halo, C₁₋C₆ alkyl, trifluoromethyl or C₁₋C₆ alkoxy. More specifically, phenyl is unsubstituted or substituted by 1, 2, 3 or 4 halogen substituents, particularly, from 1 to 3 fluoro groups, and most particularly, 2,3-difluoro, 2,4-difluoro or 4-fluoro.

A further example of formula (I) is where Y is -CH₂CH₂-.

Also described is a compound of formula (I) in which R² is hydrogen, by default, or is halo, C₁₋C₆ alkyl, mono to perfluoro- C₁₋C₄ alkyl, mono to perfluoro C₁₋C4₆ alkoxy, or C₁₋C₆ alkoxy; particularly mono to perfluoro- C₁₋C₄ alkyl, mono to perfluoro- C₁₋C₄ alkoxy, or C₁₋C₆ alkoxy. Of particular interest are the compounds where R² is other than hydrogen, n in (R²)ₙ is 1, 2, or 3, and the substitution pattern is meta and/or para, particularly para, i.e. a 4-position substituent. Exemplified compounds include those where R² is 4-trifluoromethyl or 4-trifluoromethoxy.

R³ and R⁴ can be the same or different and are methyl, ethyl, *n*-propyl, or *n*-butyl. Of particular interest are those compounds of formula (I) where R³ and R⁴ are the same and are methyl, or ethyl; methyl is of particular interest.

R⁵ can be hydrogen, C₍₁₋₆₎ alkyl which is a straight chain, or branched. Of particular interest is methyl, ethyl, propyl, isopropyl, *n*-butyl, *sec*-butyl, *iso*-butyl, *t*-butyl, n-pentyl or *n-*hexyl.

Any of the compounds described herein above can be prepared in crystalline or non-crystalline form, and, if crystalline, can be solvated, e.g. as the hydrate. Included are stoichiometric solvates (e.g. hydrates).

Certain of the compounds described herein can contain one or more chiral atoms, or can otherwise be capable of existing as two enantiomers. The compounds useful in the methods as described herein include mixtures of enantiomers as well as purified enantiomers or enantiomerically enriched mixtures. Also included are the individual isomers of the compounds represented by formulas (I) - (IV), as well as any wholly or partially equilibrated mixtures thereof. Also included are the individual isomers of the compounds as mixtures with isomers thereof in which one or more chiral centers are inverted. Also, it is understood that any tautomers and mixtures of tautomers of the compounds are included within the scope of the compounds of formulas (I) - (IV). The different isomeric forms can be separated or resolved one from the other by conventional methods, or any given isomer can be obtained by conventional synthetic methods or by stereospecific or asymmetric syntheses.

### Syntheses of the Compounds of Formula (I), (II), (III) and (IV)

Methods for preparing compounds of formula (I), (II) and (III) have been published in the patent literature. For example, methods for making formula (I) can be found in WO 01/60805 and WO03/016287. Methods for making compounds of formula (II) have been set out in WO 02/30911. And methods for making compounds of formula (III) can be found in WO 02/30904. This document provides methods for making compounds of formula (IV), methods copied from US provisional applications 60/829,328 and 60/829,327.

Some examples of syntheses are provided below. To differentiate between the several generic groups of compounds in the examples herein, materials relating to formula (I) will be labeled as "Example of Synthesis Approach (I)-1" et seq., for formula (II) "Example of Synthesis Approach (II)-1" et seq., for formula (III), "Example of Synthesis Approach (III)-1 et seq., and for formula (I), "Example of Synthesis Approach (IV)-1, et seq.

### Synthesis of Formula (I)

Compounds of formulae (I) can be prepared by processes scheme I, as disclosed in WO 01/60805: 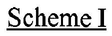 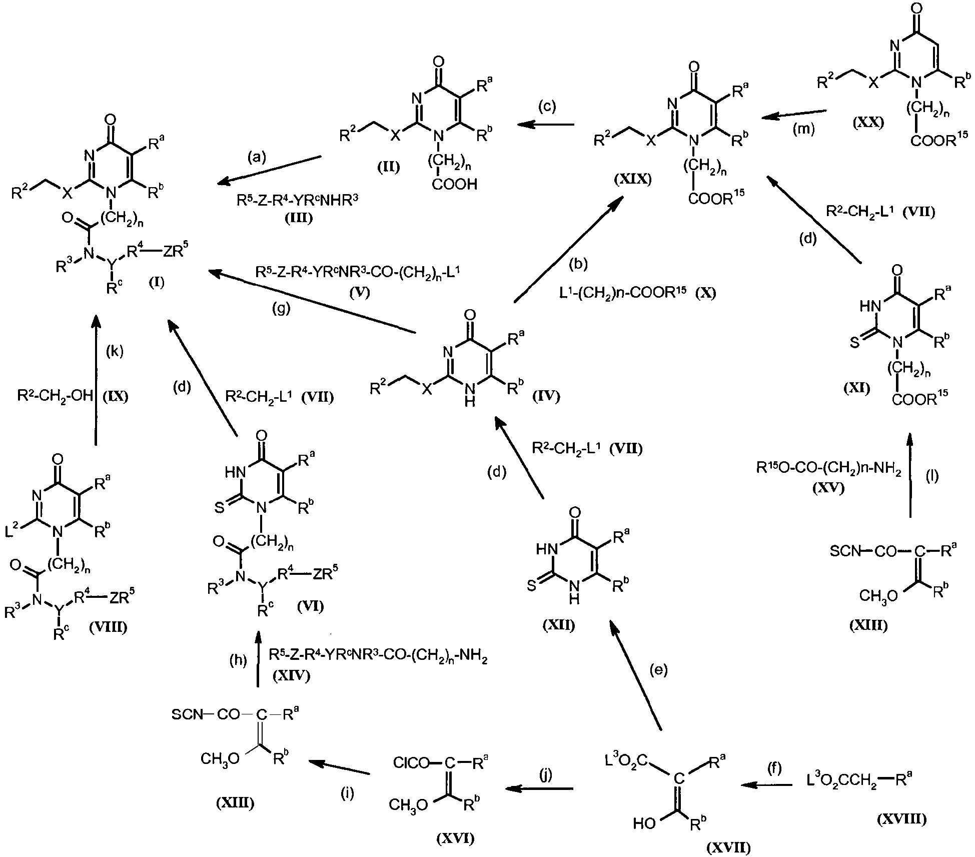 in which:
L³ is a C(1-6)alkyl group, for instance methyl;
R¹⁵ is a C₍₁₋₆₎alkyl group, for instance ethyl or t-butyl and
L¹, L², R^{a}, R^{b}, R^{c}, R², R³, R⁴, R⁵, n, X, Y and Z are as defined in WO 01/60805.

An exemplary reaction for making a compound of formula (I) of interest is as follows:

### Example of Synthesis Approach (I)-1(a)

1-(N-(2-(Diethylamino)ethyl)-N-(4-(4-trifluoromethylphenyl)benzyl)aminocarbonylmethyl)-2-(4-fluorobenzyl)thio-5,6-trimethylenepyrimidin-4-one

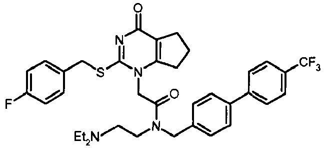

Intermediate B69 of WO 01/60805 (87.1g, 0.26 mol.) was suspended in dichloromethane (2.9 litre). 1-Hydroxybenzotriazole hydrate (35.2g, 0.26 mol.) and 1-(3-dimethylaminopropyl)-3-ethylcarbodiimide hydrochloride (99.7g, 0.52 mol.) were added and the suspension stirred for 45 minutes by which time complete solution had been obtained. Intermediate A30 of WO 01/60805 (91.2g, 0.26 mol.) was added as a solution in dichloromethane (100ml) over 5 minutes and the solution stirred for 4 hours. Saturated ammonium chloride solution:water mixture (1:1, 1 litre) was added and the solution stirred for 10 minutes. The organic phase was separated and extracted with saturated ammonium chloride:water mixture (1:1, 1 litre), extracts were pH 6. The organic phase was separated and extracted with water (1 litre) containing acetic acid (10ml), extract pH 5. The dichloromethane layer was separated and extracted with saturated sodium carbonate solution:water:saturated brine mixture (1:3:0.2, 1 litre), pH 10.5, then with saturated brine:water mixture (1:1, 1 litre). The brown solution was dried over anhydrous sodium sulfate in the presence of decolourising charcoal (35g), filtered and the solvent removed *in vacuo* to give a dark brown foam. The foam was dissolved in iso-propyl acetate (100ml) and the solvent removed *in vacuo.* The dark brown gummy residue was dissolved in boiling *iso*propyl acetate (500ml), cooled to room temperature, seeded and stirred overnight. The pale cream solid produced was filtered off and washed with iso-propyl acetate (100ml). The solid was sucked dry in the sinter for 1 hour then recrystallized from iso-propyl acetate (400ml). After stirring overnight the solid formed was filtered off, washed with iso-propyl acetate (80ml) and dried *in vacuo* to give the title compound, 110g, 63.5% yield. 1H NMR (CDCl₃, ca 1.9:1 rotamer mixture) δ 0.99 (6H, t), 2.10 (2H, m), 2.50 (4H, q), 2.58/2.62 (2H, 2 x t), 2.70/2.82 (2H, 2 x t), 2.86 (2H, t), 3.28/3.58 (2H, 2 x t), 4.45/4.52 (2H, 2 x s), 4.68/4.70 (2H, 2 x s), 4.93 (2H, s), 6.95 (2H, m), 7.31 (2H, d), 7.31/7.37 (2H, 2 x m), 7.48/7.52 (2H, d), 7.65 (2H, m), 7.72 (2H, m); MS (APCI) (M+H)⁺ 667; mp 125°C (by DSC - assymetric endotherm).

### Example of Synthesis Approach (I)-1(b)

### 1-(N-(2-(Diethylamino)ethyl)-N-(4-(4-trifluoromethylphenyl)benzyl)aminocarbonylmethyl)-2-(4-fluorobenzyl)thio-5,6-trimethylenepyrimidin-4-one bitartrate

Prepared from intermediates A30 and B69 in WO 01/60805 by the method of Example 1 in WO 01/60805. ¹H-NMR (d₆-DMSO, ca 1:1 rotamer mixture) □ 0.92/0.99 (6H,2x t), 1.99 (2H,m), 2.54 (6H,m), 2.68/2.74 (4H,m), 3.36 (2H,m), 4.21 (2H,s), 4.37/4.44 (2H,2x s), 4,63/4.74 (2H,2x s), 4,89/5.13 (2H,2x s), 7.08/7.14 (2H,2x m), 7.36-7.50 (4H, m), 7.64/7.70 (2H,2x d), 7.83 (4H,m); MS (APCI+) found (M+1) = 667; C₃₆H₃₈F₄N₄O₂S requires 666.

### Example of Synthesis Approach (I)-1(c)

### 1-(N-(2-(Diethylamino)ethyl)-N-(4-(4-trifluoromethylphenyl)benzyl)aminocarbonylmethyl)-2-(4-fluorobenzyl)thio-5,6-trimethylenepyrimidin-4-one hydrochloride

The free base from Example (I)-1(a) (3.00g, 0.0045 mol) was suspended with stirring in isopropanol (30 ml) and warmed to 45°C to give a clear solution. The solution was then cooled to ambient temperature and conc. hydrochloric acid (0.40 ml, 0.045 mol) was added. The resultant slurry was then stirred at ambient temperature for 35 minutes, before being cooled to 0°C for 35 minutes. The slurry was then filtered and washed with isopropanol (10 ml), followed by heptane (30 ml), before being dried under vacuum to give the title compound as a white solid (3.00 g, 95%). ¹H NMR (CDCl₃) δ 1.38 (6H, t), 2.08 (2H, m), 2.82 (2H, t), 2.99 (2H, t), 3.19 (4H, m), 3.35 (2H, m), 3.97 (2H, s), 4.42 (2H, s), 4.81 (2H, s), 4.99 (2H, s), 6.87 (2H, t), 7.26 (2H, t), 7.33 (2H, d), 7.41 (2H, d), 7.53 (2H, d), 7.71 (2H, d), 11.91 (1H, s).

### Synthesis of Formula (II)

A description of how to make the compounds of formula (II) and examples of intermediates and final products for the compounds named above can be found in published international application WO 02/30911. A last-step method for making a compound useful in the described methods is Example (II)-1.

### Example of Synthesis Approach (II)-1

### N-(2-Diethylaminoethyl)-2-[2-(2-(2,3-difluorophenyl)ethyl)-4-oxo-4,5,6,7-tetrahydrocyclopentapyrimidin-1-yl]-N-(4'-trifluoromethyl-biphenyl-4-ylmethyl)acetamide bitartrate

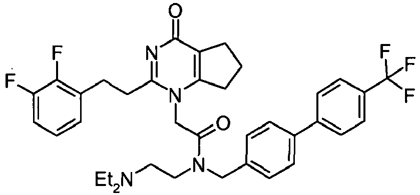

A solution of *N,N-*diethyl-N-(4'-trifluoromethyl-biphenyl-4-ylmethyl)-ethane-1,2-diamine (Int D4 in WO 02/30911) (0.50g, 1.44mmol), 1-(3-dimethylaminopropyl)-3-ethylcarbodiimide (0.56g, 1.45mmol), 1-hydroxybenzotriazole hydrate (0.12g) and 2-(2-[2-(2,3-difluorophenyl)-ethyl]-4-oxo-4,5,6,7-tetrahydro-cyclopentapyrimidin-1-yl)-acetic acid (Int C1 in WO 02/30911) (0.48g, 1.44mmol) in dichloromethane (10ml) was stirred at ambient temperature overnight then diluted with dichloromethane (30ml), washed with aqueous sodium bicarbonate and evaporated. The residue was purified by chromatography (10g silica cartridge, ethyl acetate-acetone) to give the title compound as a yellow foam (free base) (0.50g, 52%). ¹H-NMR (DMSO, rotamer mixture) δ 0.83-0.89 (6H, m), 1.98 (2H, m), 2.40 (4H, m), 2.45-2.82 (10H, m), 3.02 (2H, m), 4.64/4.75 (2H,2x s), 4.96/5.19 (2H,2x s), 7.11-7.40 (5H, m), 7.65 (2H, m), 7.84 (4H, m); MS (APCI+) found (M+1) = 667;

₃₇H₃₉F₅N₄O₂ requires 666.

*d*-Tartaric acid (0.09g, 0.60mmol) was added to a solution of the free base (0.40g, 0.60mmol) in methanol (10ml) with stirring. The resulting solution was evaporated to yield the salt (0.49g). ¹H-NMR (DMSO, rotamer mixture) δ 0.85-0.97 (6H, m), 1.91-2.00 (2H, m), 2.40-2.49 (4H, m), 2.54-2.82 (10H, m), 3.02-3.46 (2H, m), 4.20 (2H, s), 4.64/4.75 (2H, 2x s), 4.97/5.18 (2H, 2x s), 7.11-7.40 (5H, m), 7.65 (2H, m), 7.84 (4H, m); MS (APCI+) found (M+1) = 667; C₃₇H₃₉F₅N₄O₂ requires 666.

Following this process, or alternatively other processes described in WO 02/30911, one can prepare the other compounds named above that have the structure of formula (II).

### Synthesis of Formula (III)

The overall synthesis of compounds of formula (III) is illustrated in the following scheme III, as presented in WO02/30904:

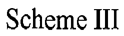

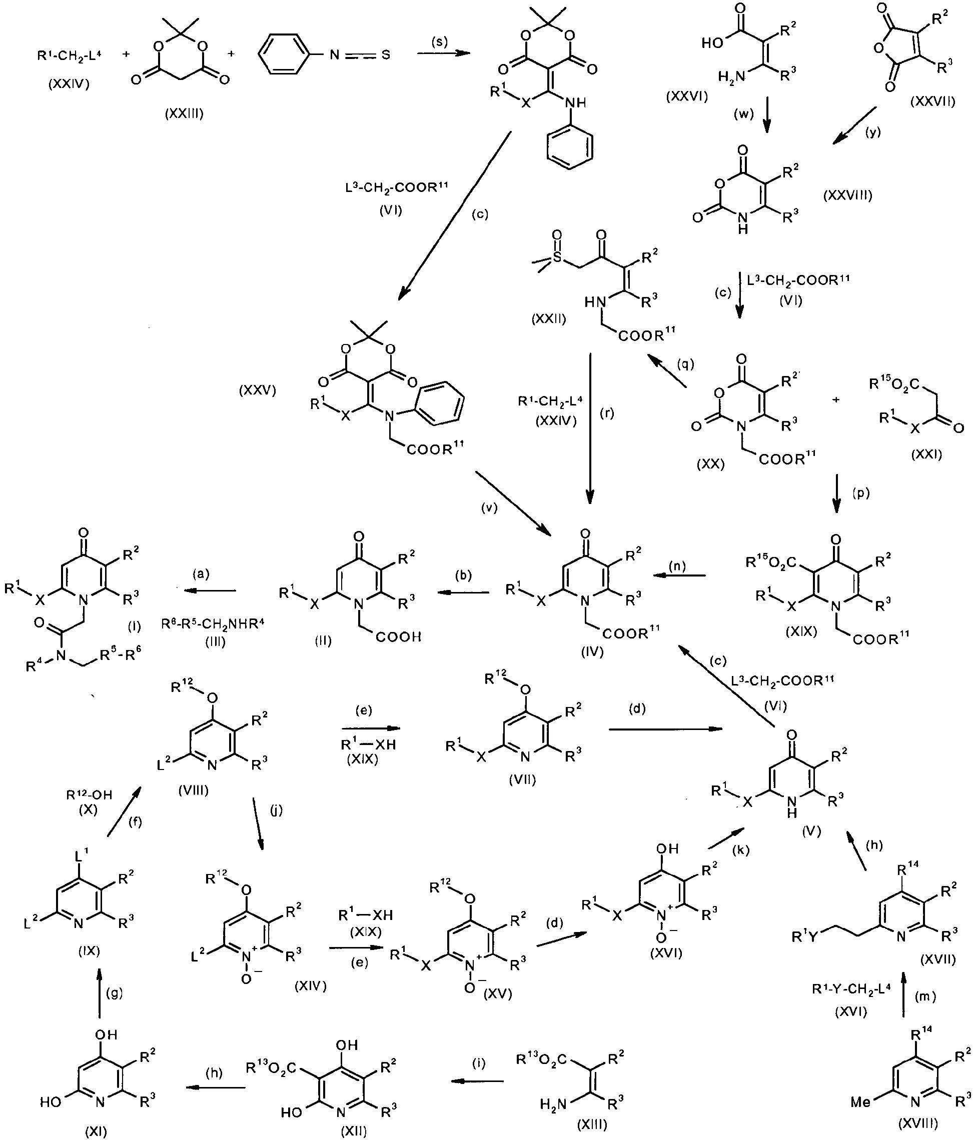

Referring to this scheme, the ester (IV) is usually prepared by N-1 alkylation of (V) using (VI), in which R¹¹ is as hereinbefore defined e.g. (VI) is t-butyl bromoacetate or ethyl bromoacetate, in the presence of a base e.g. BuLi in THF or sodium hydride in N-methyl pyrrolidinone (NMP) (step c).

When X is CH₂S, the key intermediate (IV) can be synthesised by reacting (XX) with dimethyloxosulfonium methylide, generated via the treatment of trimethylsulfoxonium iodide with sodium hydride at low temperature, to yield a sulfur ylid (XXII) (step q). Subsequent treatment of (XXII) with carbon disulfide in the presence of diisopropylamine, followed by R¹CH₂-L⁴, where L⁴ is a leaving group, yields intermediate (IV) (step r).

Alternatively, when X is CH₂S, the R¹X substituent can be introduced by displacement of a leaving group L² (e.g. Cl) (step e) either on a pyridine (VIII) or pyridine N-oxide (XIV), to give 2-substituted pyridines (VII) and (XV). Transformation of (VII) or (XV) to the 4-pyridone (V) is accomplished by deprotection of the 4-oxygen (e.g. using (Ph₃P)₃RhCl when in aq. ethanol when R¹² = allyl) (step d), followed, for (XVI), by removal of the N-oxide substituent, using hydrogen in the presence of Pd/C in acetic acid (step k). The pyridine (VIII) or pyridine N-oxide (XIV) can be prepared by steps (i), (h), (g), (f), and (j), in which:
(j) treatment of (VIII) with m-chloroperbenzoic acid in dichloromethane;
(f) treatment of (IX) with R¹²OH (X), in which R¹² is allyl, and sodium hydride in DMF;
(g) treatment of (XI) with phosphorus oxychloride;
(h) treatment of (XII) with aq HCl with heating;
(i) treatment of (XIII) with di-lower alkyl malonate and sodium alkoxide in alcohol (in which R¹³ is C₍₁₋₆₎alkyl, typically R¹³ = Et); and

R¹- CH₂SH (XIX) is typically prepared from the thioacetate, which is formed from the corresponding alkyl bromide R¹-CH₂Br.

Alternatively, when X is CH₂S and R² and R³, together with the pyridone ring carbon atoms to which they are attached, form a fused benzo ring, intermediate (IV) can be synthesised from known starting materials by steps (s), (c) and (v) in which:
(s) treatment of Meldrum's acid (XXIII) with sodium hydride at low temperature, followed by reaction with phenylisothiocyanate and subsequent treatment with R¹CH₂-L⁴;
(c) as hereinbefore discussed;
(v) treatment of (XXV) with trifluoroacetic acid.

When X is alkylene, it is preferable to use steps (m) and (h) (intermediates (XVII), (XVIII)) or steps (n) and (p) (intermediates (XIX), (XX), (XXI)) in which:
(h) transformation of a 4-substituted pyridine into a 4-pyridone e.g. by treatment of (XVII) R¹⁴ = Cl with aq HCl and dioxan, or deprotection of R¹⁴ = OR¹², e.g. using conditions of step (d).
(m) chain extension of a 2-alkyl pyridine, e.g. where X = YCH₂CH₂ by treatment of a 2-methylpyridine (XVIII) with R¹-Y-CH₂-L⁴ (XVI) in which L⁴ is a leaving group and a strong base, such as BuLi, in THF.

In the alternative route, the 3-ester group is removed from intermediate (XIX) R¹⁵ - C₍₁₋₆₎alkyl by heating in diphenyl ether where R¹⁵ = tBu (step n); Intermediate (XIX) is formed from the 2,6-dioxo-1,3-oxazine (XX) and ester (XXI) by treatment with a base such as NaH in DMF or 1,8-diazabicyclo[5.4.0]undec-7-ene in dichloromethane.

Synthesis of (XX) from known starting materials can be achieved via steps (w) and (c) or steps (y) and (c) in which:
(w) treatment of (XXVII) with azidotrimethylsilane in THF;
(y) treatment of (XXVI) with phosgene;
(c) as hereinbefore described.

See WO02/30904 for additional details and exposition of how to make compounds of formula (III).

### Example of Synthesis Approach (III)-1

### N-(1-(2-Methoxyethyl)piperidin-4-yl)-2-[2-(2,3-difluorobenzylthio)-4-oxo-4H-quinolin-1-yl]-N-(4'-trifluoromethylbiphenyl-4-ylmethyl)acetamide

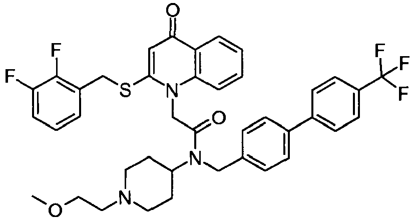

The free base was prepared from Int. E1 and Int. A42 by the method of Example 1 in WO 02/30904, except using DMF as solvent in place of dichloromethane. 1.97g of this material was crystallised from n.butyl acetate (10ml) to give the title compound (1.35g). ¹H-NMR (CD₃OD) δ 1.7-2.05 (4H, m), 2.05-2.3 (2H, 2xt), 2.5-2.65 (2H, m), 2.95-3.1 (2H, m), 3.3 (3H, s), 3.45-3.55 (2H, m), 3.9-4.05 + 4.4-4.5 (1H, 2xm), 4.37 + 4.48 (2H, 2xs), 4.71 + 4.87 (2H, 2xbr s), 5.31 + 5.68 (2H, 2xs), 6.44 + 6.52 (1H, 2xs), 6.95-7.3 (3H, m), 7.35-7.85 (11H, m), 8.2-8.35 (1H, m); MS (APCI+) found (M+1) 736; C₄₀H₃₈F₅N₃O₃S requires 735.

### Synthesis of Formula (IV)

The following flow chart illustrates a process for making exemplified compounds.

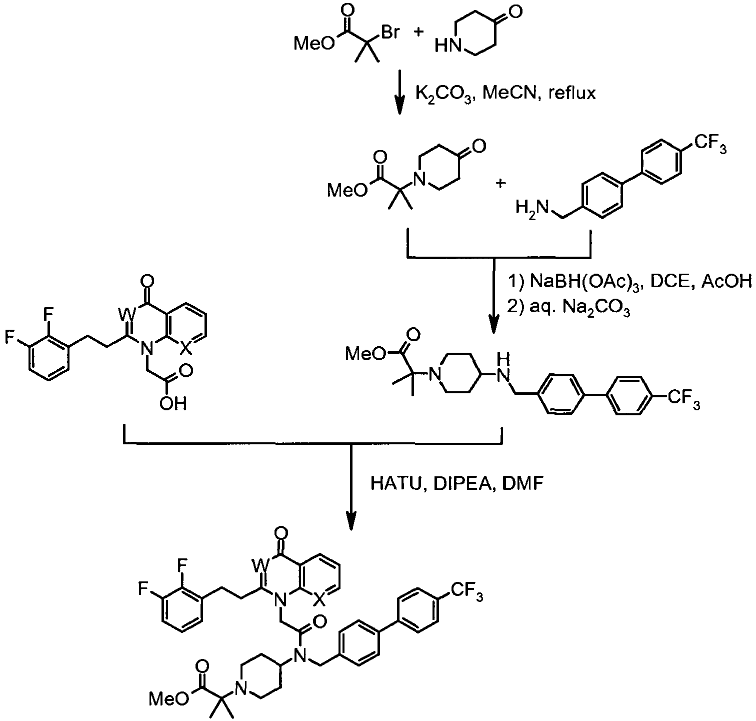

In addition, the reader is referred to published PCT application WO 03/016287 for chemistries that can be useful in preparing some of the intermediates set out in this flow chart. In addition, reference is made to the syntheses set out in published PCT applications WO 01/60805, WO 02/30911, WO 02/30904, WO 03/042218, WO 03/042206, WO 03/041712, WO 03/086400, and WO 03/87088, and co-pending US provisional applications 60/829,328 and 60/829,327 both filed 13 October 2006 noted above. To the extent the reader wishes to prepare the compounds of formula (IV) by using intermediates, reagents, solvents, times, temperatures, etc., other than those in the route on the foregoing page, these published PCT applications and co-pending US applications can provide useful guidance. *Intermediate (IV)-A1* {[4'-(Trifluoromethyl)-4-biphenylyl]methyl}amine

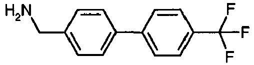

The preparation of this compound was described in WO 02/30911 as Intermediate D7.

### Intermediate (IV)-A2 ({4'-[(Trifluoromethyl)oxy]-4-biphenylyl}methyl)amine hydrochloride

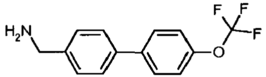

A solution of 4'-[(trifluoromethyl)oxy]-4-biphenylcarbonitrile (prepared from {4-[(trifluoromethyl)oxy]phenyl}boronic acid by a method analogous to that described for the 4'-trifluoromethyl analogue, Intermediate D6 of WO 02/30911) (66.6g) in ethanol (2000ml) and concentrated hydrochloric acid (100ml) was hydrogenated over Pearlman's catalyst (10g) at 25psi until reduction was complete. The catalyst was removed by filtration through celite, then the solvent was removed in vacuo to obtain the desired product.

LCMS Rt = 2.212 minutes; m/z [M+H]⁺ =251.0

### Intermediates for making formula (IV)

*Intermediate (IV)-A3*

### Methyl 2-methyl-2-(4-oxo-1-piperidinyl)propanoate

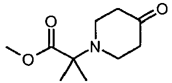

A mixture of methyl 2-bromo-2-methylpropanoate (80.87ml, 5 equiv), 4-piperidone hydrochloride monohydrate (19.6g, 1 equiv), acetonitrile (200ml) and potassium carbonate (69.1g, 4 equiv) was heated at reflux under nitrogen with mechanical stirring for 17.5h then cooled in an ice bath before adding diethyl ether (100ml). Filtration through celite followed by flash chromatography (silica, 10-50% ethyl acetate in hexane) and evaporation of the product fractions gave the desired product as a yellow oil (14.28g).

¹H NMR (CDCl₃) δ 1.41 (6H,s), 2.47 (4H,m), 2.88 (4H,m), 3.73 (3H,s).

### Intermediate (IV)-A4

### Ethyl 2-methyl-2-(4-oxo-1-piperidinyl)propanoate

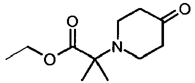

A mixture of ethyl 2-bromo-2-methylpropanoate (48.3ml, 5 equiv), 4-piperidone hydrochloride monohydrate (100g, 1 equiv), acetonitrile (1216ml) and potassium carbonate (353g, 4 equiv) was heated at reflux under nitrogen with mechanical stirring for 20h then cooled in an ice bath before adding diethyl ether (approx. 1400ml). The mixture was filtered through celite, evaporated in vacuo, then excess bromoester distilled off (50°C still head temperature/10 Torr). Flash chromatography (silica, 5-30% ethyl acetate in hexane) and evaporation of the product fractions gave the crude product as a yellow oil. To remove some remaining bromoester contaminant this was partitioned between ethyl acetate and 2M aqueous hydrochloric acid. The organic layer was discarded and the aqueous layer was basified with sodium carbonate, saturated with sodium chloride and extracted with ethyl acetate. Drying and evaporation of the organic extracts gave the desired product as a yellow oil (54.7g).

¹H NMR (CDCl₃) δ 1.27 (3H,t), 1.40 (6H,s), 2.47 (4H,m), 2.90 (4H,m), 4.20 (2H,q).

### Intermediate (IV)-A5

### 1,1-Dimethylethyl 2-methyl-2-(4-oxo-1-piperidinyl)propanoate

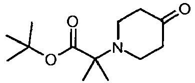

A mixture of 1,1-dimethylethyl 2-bromo-2-methylpropanoate (8.0g, 1.1 equiv), 4-piperidone hydrochloride (5.0g, 1 equiv), acetone (50ml) and potassium carbonate (13.0g, 3 equiv) was heated at reflux with stirring for 24h, then filtered and the filtrate evaporated. The crude residue was used in the next step without purification.

ES+MS m/z [M+H-tBu]⁺ =186.1

*Intermediate (IV)-B1*

### Methyl 2-methyl-2-[4-({[4'-(trifluoromethyl)-4-biphenylyl]methyl}amino)-1-piperidinyl]propanoate

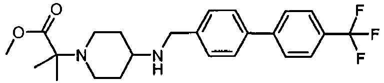

A mixture of methyl 2-methyl-2-(4-oxo-1-piperidinyl)propanoate (Int. A3) (14.28g, 1 equiv), {[4'-(trifluoromethyl)-4-biphenylyl]methyl}amine (Int. A1) (19.6g, 0.85 equiv), DCE (300ml), acetic acid (3.8ml, 0.90 equiv) and sodium triacetoxyborohydride (20.7g, 1.25 equiv) was stirred at room temperature under nitrogen for 17.5h. Aqueous sodium carbonate (2M solution, excess) was added and stirred for 4h, then the mixture was extracted with a mixture of diethyl ether and THF. The organic extracts were backwashed with water and brine, dried over sodium sulfate and filterered through a pad of silica gel which was rinsed with 2.5% methanol in DCM. After evaporation in vacuo, the crude product was crystallised from ether/hexane, finally at ice bath temperature, which after drying yielded a white solid (20.9g).

LCMS Rt = 2.070 minutes; m/z [M+H]⁺ = 435.2

¹H NMR (d₆-DMSO) δ 1.15-1.32 (8H, m), 1.75-187(2H,m), 1.97-2.12 (2H,m), 2.27-2.40 (1H, m), 2.77-2.90(2H,m), 3.60 (3H,s), 3.76 (2H,s), 7.46 (2H, d, J=8.03Hz), 7.67 (2H, d, J=8.28Hz), 7.80 (2H, d, J=8.53Hz), 7.88 (2H, d, 8.03Hz)

### Intermediate (IV)-B2

### Ethyl 2-methyl-2-[4-({[4'-(trifluoromethyl)-4-biphenylyl]methyl}amino)-1-piperidinyl]propanoate

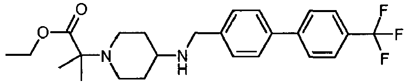

A mixture of ethyl 2-methyl-2-(4-oxo-1-piperidinyl)propanoate (Int. A4) (25.6g, 1.2 equiv), {[4'-(trifluoromethyl)-4-biphenylyl]methyl}amine (Int. A1) (31.1g, 1.0 equiv), DCE (400ml) and acetic acid (6.3ml, 1.1 equiv) was stirred at room temperature under nitrogen. Sodium triacetoxyborohydride (33.5g, 1.5 equiv) was added and stirring contined for 19 hours. Aqueous sodium carbonate (2M solution, excess) was added and stirred for 1.5h, then the mixture was extracted with a mixture of diethyl ether and THF. The organic extracts were backwashed with water and brine, filterered through a pad of silica gel, dried over sodium sulfate and evaporated in vacuo. The desired product was obtained as a white solid (44.2g) which was used without further purification.

LCMS Rt = 2.194 minutes; m/z [M+H]⁺ = 449.3

¹H NMR (d₆-DMSO) δ 1.06-1.32 (11H,m), 1.74-1.89 (2H,m), 1.99-2.14 (2H, m), 2.25-2.39 (1H. m), 2.69-2.89 (2H, m), 3.75 (2H, s), 4.01-4.12 (2H, m), 7.45 (2H, d, J=7.55 Hz), 7.67 (2H, d, J=7.81 Hz), 7.79 (2H, d, J=8.06 Hz), 7.88 (2H. d, J=8.06Hz)

### Intermediate (IV)-B3

### Ethyl 2-methyl-2-{4-[({4'-[(trifluoromethyl)oxy]-4-biphenylyl}methyl)amino]-1-piperidinyl} propanoate

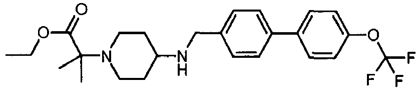

A mixture of ethyl 2-methyl-2-(4-oxo-1-piperidinyl)propanoate (Int. A4) (1.09g, 1.2 equiv), ({4'-[(trifluoromethyl)oxy]-4-biphenylyl}methyl)amine hydrochloride (Int. A2) (1.28g, 1.0 equiv), DCE (21ml) and acetic acid (0.27ml, 1.1 equiv) was stirred at room temperature under nitrogen. Sodium triacetoxyborohydride (1.42g, 1.5 equiv) was added and stirring continued for 3 hours. Aqueous sodium carbonate (2M solution, excess) was added and stirred for 45min, then the mixture was partitioned with a mixture of diethyl ether/THF and water. The organic extracts were backwashed with water and brine, and dried over sodium sulfate and evaporated in vacuo. The desired product was obtained as a light yellow solid (2.14g) which was used without further purification.

LCMS Rt = 2.244 minutes; m/z [M+H]⁺ = 465.3

### Intermediate (IV)-B4

### 1,1-Dimethylethyl 2-methyl-2-[4-({[4'-(trifluoromethyl)-4-biphenylyl]methyl}amino)-1-piperidinyl]propanoate

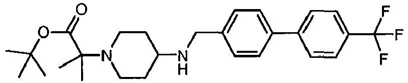

A mixture of 1,1-dimethylethyl 2-methyl-2-(4-oxo-1-piperidinyl)propanoate (Int. A6) (370mg, 1.2 equiv), {[4'-(trifluoromethyl)-4-biphenylyl]methyl}amine (Int. A1) (397mg, 1 equiv), sodium triacetoxyborohydride (400mg, 1.5 equiv), DCM (10ml) and acetic acid (0.076ml, 1 equiv) was combined and stirred at room temperature until LCMS confirmed disappearance of the amine starting material (approx. 18 hours). Aqueous sodium carbonate was added and then extracted with DCM. The organics were dried over sodium sulfate and concentrated to give a solid (420mg) that was used without further purification.

LCMS Rt = 2.24 minutes; m/z [M+H]⁺ = 477.3

### Intermediate (IV)-C1

### [2-[2-(2,3-Difluorophenyl)ethyl]-4-oxo-1(4H)-quinazolinyl]acetic acid

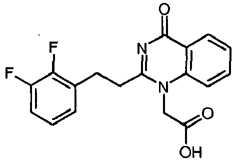

The preparation of this compound was described in WO 02/30911 as Intermediate C43.

### Intermediate (IV)-C2

[2-[2-(2,3-Difluorophenyl)ethyl]-4-oxo-1,8-naphthyridin-1(*4H*)-yl]acetic acid

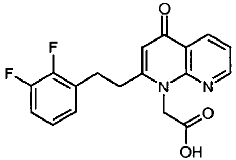

The preparation of this compound was described in WO 02/30904 as Intermediate E21.

### Intermediate (IV)-C3

### [2-[2-(2,4-Difluorophenyl)ethyl]-4-oxo-1(4H)-quinazolinyl]acetic acid

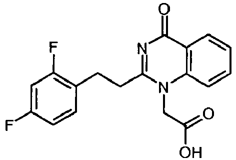

The preparation of this compound was described in WO 02/30911 as Intermediate C45.

### Intermediate (IV)-C4

### Ethyl [2-[2-(2,4-difluorophenyl)ethyl]-4-oxopyrido[2,3-d]pyrimidin-(4H)-yl]acetate

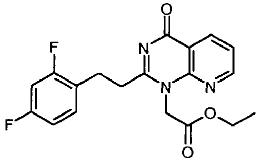

A mixture of ethyl (2,4-dioxo-3,4-dihydropyrido[2,3-*d*]pyrimidin-1(2*H*)-yl)acetate (WO 02/30911, Intermediate B52) (40.8g, 1.2 equiv) and 3-(2,4-difluorophenyl)-propanimidamide (made by methods analogous to those described for the 2,3-difluoro isomer, Intermediates A1 to A3 of WO 02/30911) (30.0g, 1 equiv) was fused in a 150°C oil bath for 25 min, then cooled quickly to room temperature in a water bath. Chromatography (silica, crude product loaded in DCM and eluted with 50-100% ethyl acetate in hexane) gave the desired product (43.56g).

LCMS Rt = 2.521 minutes; m/z [M+H]⁺ = 374.1

¹H NMR (CDCl₃) δ 1.31 (3H, t), 3.13 (2H, m), 3.26 (2H, m), 4.28 (2H, q), 5.27 (2H, s), 6.82 (2H, m), 7.34 (1H, m), 7.50 (1H, m), 8.65 (1H, m), 8.74 (1H, m).

### Intermediate (IV)-C5

### [2-[2-(2,3-Difluorophenyl)ethyl]-4-oxopyrido[2,3-d]pyrimidin-1(4H)-yl]acetic acid

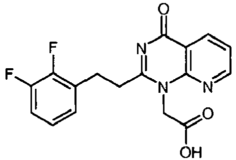

The preparation of this compound was described in WO 02/30911 as Intermediate C35.

### Intermediate (IV)-C5

### [2-[2-(2,4-Difluorophenyl)ethyl]-4-oxopyrido[2,3-d]pyrimidin-1(4H)-yl]acetic acid

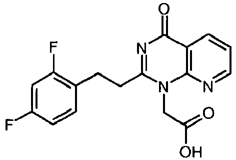

Ethyl [2-[2-(2,4-difluorophenyl)ethyl]-4-oxopyrido[2,3-*d*]pyrimidin-1(*4H*)-yl]acetate (Int. C1) (32.76g, 1 equiv) was dissolved in ethanol (350ml) and water (70ml), cooled in ice, then aqueous lithium hydroxide (2M solution, 43.42ml, 0.99 equiv) was added. Stirring was continued for 2h at room temperature. The solution was concentrated in vacuo and the residue was redissolved in water (700ml) and saturated aqueous sodium bicarbonate (50ml), then washed with ethyl acetate (200ml). The aqueous layer was acidified to pH 2 with 2M hydrochloric acid, and the precipitate was filtered off, washed with ice water (50ml) and dried in vacuo (50°C, 16h) to obtain the desired product (23.2g).

¹H NMR (d₆-DMSO) δ 2.4-2.6 (4H, m), 5.24 (2H, s), 7.04 (1H, m), 7.22 (1H, m), 7.48 (1H, m), 7.60 (1H, m), 8.47 (1H, m), 8.84 (1H, m).

### Example (IV)-1

Methyl 2-[4-({[2-[2-(2,3-difluorophenyl)ethyl]-4-oxo-1(*4H*)-quinazolinyl]acetyl} {[4'-(trifluoromethyl)-4-biphenylyl]methyl}amino)-1-piperidinyl]-2-methylpropanoate 2,3-dihydroxybutanedioate (salt)

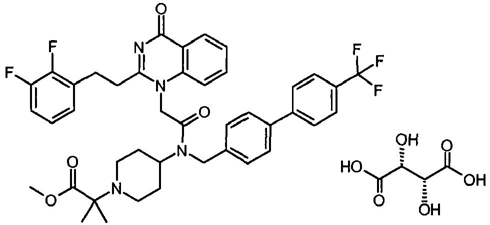

A mixture of [2-[2-(2,3-difluorophenyl)ethyl]-4-oxo-1(*4H*)-quinazolinyl]acetic acid (Int. C1) (100mg, 1 equiv), methyl 2-methyl-2-[4-({[4'-(trifluoromethyl)-4-biphenylyl]methyl}amino)-1-piperidinyl]propanoate (Int. B1) (130mg, 1.03 equiv), DIPEA (0.1ml, 3.6 equiv), acetonitrile (2ml) and HATU (130mg, 1.4 equiv) was stirred at room temperature for 1h, then evaporated and redissolved in acetonitrile. Purification by reverse phase HPLC (Preparative Method A) gave methyl 2-[4-({[2-[2-(2,3-difluorophenyl)ethyl]-4-oxo-1(*4H*)-quinazolinyl]acetyl} {[4'-(trifluoromethyl)-4-biphenylyl]methyl]amino)-1-piperidinyl]-2-methylpropanoate (128mg).

LCMS Rt = 2.686 minutes; m/z [M+H]⁺ = 761.3

¹H NMR (CDCl₃) δ 1.33 (3H, s), 1.36 (3H, s), 1.83-2.02 (4H, m), 2.36-2.48 (2H, m), 2.87-2.91 (1H, m), 3.06-3.09 (2H, m), 3.16-3.20 (2H, m), 3.26-3.29 (1H, m), 3.71-3.73 (3H, m), 4.02/4.51 (1H, 2x br m), 4.74 (1H, s), 4.92 (1H, s), 5.12 (1H, s), 5.56 (1H, s), 7.00-7.19 (3H, m), 7.32-7.37 (1H, m), 7.48-7.62 (5H, m), 7.72-7.81 (5H, m), 8.22-8.28 (1H, m).

The free base was converted to the bitartrate salt by adding L-tartaric acid (1.675g, 1.0 equiv) in one portion and stirred for 30 minutes at room temperature. The solution was concentrated in vacuo to an off-white powder that was dried in a vacuum oven at room temperature.

### Example of Synthesis Approach (IV)-2

### Methyl 2-[4-({[2-[2-(2,3-difluorophenyl)ethyl]-4-oxo-1,8-naphthyridin-1(4H)-yl]acetyl} {[4'-(trifluoromethyl)-4-biphenylyl]methyl}amino)-1-piperidinyl]-2-methylpropanoate 2,3-dihydroxybutanedioate (salt)

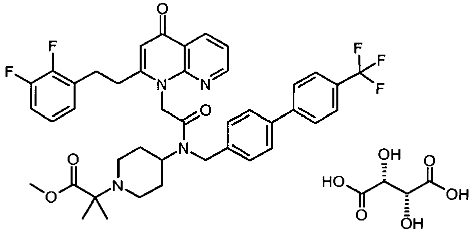

A mixture of [2-[2-(2,3-difluorophenyl)ethyl]-4-oxo-1,8-naphthyridin-1(*4H*)-yl]acetic acid (Int. C2) (100mg, 1 equiv), carbonyldiimidazole (50mg, 1.05 equiv) and dimethylacetamide (4ml) was stirred at 60°C for 30 min then methyl 2-methyl-2-[4-({[4'-(trifluoromethyl)-4-biphenylyl]methyl}amino)-1-piperidinyl]propanoate (Int. B1) (132mg, 1.05 equiv) was added and the temperature raised to 80°C for 2h. A further portion of carbonyldiimidazole (0.5 equiv) was added and stirring continued at 80°C for 15h. After cooling the crude mixture was applied to reverse phase HPLC (Preparative Method A) to obtain methyl 2-[4-({[2-[2-(2,3-difluorophenyl)ethyl]-4-oxo-1,8-naphthyridin-1(*4H*)-yl]-acetyl} {[4'-(trifluoromethyl)-4-biphenylyl]methyl}amino)-1-piperidinyl]-2-methylpropanoate (99mg).

LCMS Rt = 2.845 minutes; m/z [M+H]+ = 761.3

1H NMR (CDCl3) δ 1.28 (3H, s), 1.31 (3H, s), 1.73-2.05 (4H, m), 2.25 (1H, t), 2.39-2.46 (1H, m), 2.96-2.99 (1H, m), 3.00-3.12 (4H, m), 3.19 (1H, s), 3.68-3.73 (3H, m), 4.11/4.41 (1H, 2x br m), 4.73 (1H, s), 4.97 (1H, s), 5.51 (1H, s), 6.29-6.34 (1H, m), 7.06-7.20 (2H, m), 7.35-7.41 (1H, m), 7.48-7.58 (2H, m), 7.68-7.84 (6H, m), 8.60-8.68 (1H, m), 8.87-8.91 (1H, m).

This was converted to the bitartrate salt by a method analogous to that described for Example of Synthesis Approach (I).

### Example of Synthesis Approach (IV)-3

Ethyl 2-[4-({[2-[2-(2,3-difluorophenyl)ethyl]-4-oxo-1(*4H*)-quinazolinyl]acetyl}{[4'-(trifluoromethyl)-4-biphenylyl]methyl}amino)-1-piperidinyl]-2-methylpropanoate 2,3-dihydroxybutanedioate (salt)

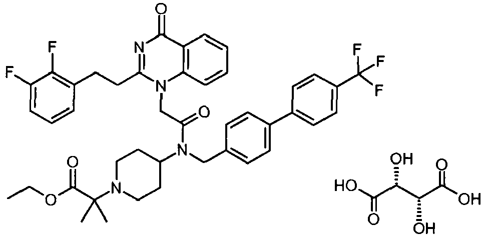

A mixture of [2-[2-(2,3-difluorophenyl)ethyl]-4-oxo-1(*4H*)-quinazolinyl]acetic acid (Int. C1) (115mg, 1 equiv), ethyl 2-methyl-2-[4-({[4'-(trifluoromethyl)-4-biphenylyl]methyl}amino)-1-piperidinyl]propanoate (Int. B2) (150mg, 1 equiv), HATU (151mg, 1.2 equiv), DMF (2.7ml) and DIPEA (0.17ml, 3 equiv) was shaken at room temperature for 5h. The reaction mixture was partitioned between ethyl acetate/methanol and aqueous sodium bicarbonate, then the organic layer was brine-washed and dried. Flash chromatography (silica, 3-4% methanol in DCM) gave ethyl 2-[4-({[2-[2-(2,3-difluorophenyl)ethyl]-4-oxopyrido[2,3-d]pyrimidin-1(*4H*)-yl]acetyl} {[4'-(trifluoromethyl)-4-biphenylyl]methyl}amino)-1-piperidinyl]-2-methylpropanoate as a white solid (190mg).

LCMS Rt = 2.55 minutes; m/z [M+H]⁺ = 775.3

¹H NMR (CDCl₃) δ 1.18-1.40 (9H, m), 1.61-2.09 (4H, m), 2.22-2.45 (2H, m), 2.75-2.85 (1H, m), 2.90-3.34 (5H, m), 3.71/4.66 (1H, 2x m), 4.12-4.26 (2H, m), 4.70-4.85 (3H, m), 5.08 (1H, s), 6.80-6.88 (1H,m), 6.95-7.13 (3H, m), 7.27-7.33 (1H, m), 7.34-7.52 (3H,m), 7.56-7.62 (1H, m), 7.63-7.77 (4H, m), 8.29-8.44 (2H, m).

This was converted to the bitartrate salt by a method analogous to that described in Example of Synthesis Approach (I).

### Example of Synthesis Approach (IV)-4

Ethyl 2-{4-[{[2-[2-(2,3-difluorophenyl)ethyl]-4-oxo-1(4*H*)-quinazolinyl]acetyl}({4'-[(trifluoromethyl)oxy]-4-biphenylyl}methyl)amino]-1-piperidinyl}-2-methylpropanoate 2,3-dihydroxybutanedioate (salt)

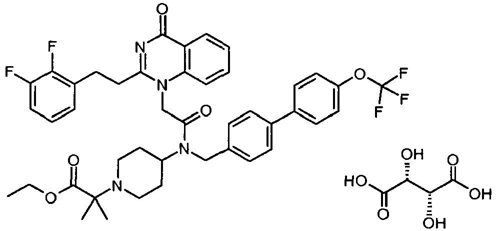

A mixture of [2-[2-(2,3-difluorophenyl)ethyl]-4-oxo-l(4//)-quinazolinyl]acetic acid (Int. C1) (124mg, 1.2 equiv), ethyl 2-methyl-2-{4-[({4'-[(trifluoromethyl)oxy]-4-biphenylyl}methyl)amino]-1-piperidinyl}propanoate (Int. B3) (139mg, 1 equiv), DMF (1.2ml) and DIPEA (0.16ml, 3 equiv) was shaken at room temperature for 30 min, then HATU (176mg, 1.5 equiv) was added and shaking continued for 4h. Reverse phase HPLC (Preparative Method B) gave ethyl 2-{4-[{[2-[2-(2,3-difluorophenyl)ethyl]-4-oxo-1(*4H*)-quinazolinyl]acetyl} ({4'-[(trifluoromethyl)oxy]-4-biphenylyl} methyl)amino]-1-piperidinyl}-2-methylpropanoate as a white solid (174mg).

LCMS Rt = 2.77 minutes; m/z [M+H]⁺ = 791.3

¹H NMR (CDCl₃) Characteristic peaks: δ 1.21-1.42 (9H, m), 1.58-2.08 (4H, m), 2.20-2.48 (2H, m), 2.71-5.1 (13H, br m), 6.79-6.87 (1H, d), 6.92-7.11 (3H, m), 7.30-7.46 (5H, m), 7.48-7.63 (5H, m), 8.26-8.40 (1H, m)

This was converted to the bitartrate salt by a method analogous to that described in Example of Synthesis Approach (I).

### Example of Synthesis Approach (IV)-5

### Methyl 2-[4-({[2-[2-(2,4-difluorophenyl)ethyl]-4-oxo-1(4H)-quinazolinyl]acetyl} {[4'-(trifluoromethyl)-4-biphenylyl]methyl}amino)-1-piperidinyl]-2-methylpropanoate 2,3-dihydroxybutanedioate (salt)

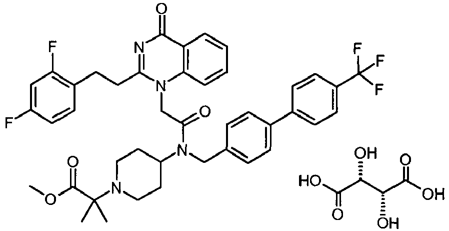

mixture of [2-[2-(2,4-difluorophenyl)ethyl]-4-oxo-1(*4H*)-quinazolinyl]acetic acid (Int. C3) (100mg, 1 equiv), methyl 2-methyl-2-[4-({[4'-(trifluoromethyl)-4-biphenylyl]methyl}amino)-1-piperidinyl]propanoate (Int. B1) (130mg, 1.03 equiv), DIPEA (0.1ml, 2 equiv), acetonitrile (2ml) and HATU (130mg, 1.4 equiv) was stirred at room temperature for 1h, then evaporated and redissolved in acetonitrile. Purification by reverse phase HPLC (Preparative Method B) gave methyl 2-[4-({[2-[2-(2,4-difluorophenyl)ethyl]-4-oxo-1(*4H*)-quinazolinyl]acetyl} {[4'-(trifluoromethyl)-4-biphenylyl]methyl}amino)-1-piperidinyl]-2-methylpropanoate (126mg).

LCMS Rt = 2.698 minutes; m/z [M+H]⁺ = 761.3

¹H NMR (CDCl₃) δ 1.30 (3H, s), 1.34 (3H s), 1.81-2.03 (4H, m), 2.29-2.35 (1H, m), 2.39-2.45 (1H, m), 2.82-2.87 (1H, m), 3.00-3.14 (4H, m), 3.19-3.24 (1H, m), 3.70-3.73 (3H, m), 4.00/4.51 (1H, 2x br m), 4.74 (1H, s), 4.91 (1H, s), 5.10 (1H, s), 5.54 (1H, s), 6.77-6.84 (1H, m), 6.87-6.98 (1H, m), 7.28-7.43 (2H, m), 7.48-7.61 (5H, m), 7.73-7.81 (5H, m), 8.23-8.29 (1H, m).

This was converted to the bitartrate salt by a method analogous to that described in Example of Synthesis Approach (I).

### Example Synthesis Approach (IV)-6

### Ethyl 2-[4-({[2-[2-(2,4-difluorophenyl)ethyl]-4-oxo-1(4H)-quinazolinyl]acetyl} {[4'-(trifluoromethyl)-4-biphenylyl]methyl}amino)-1-piperidinyl]-2-methylpropanoate 2,3-dihydroxybutanedioate (salt)

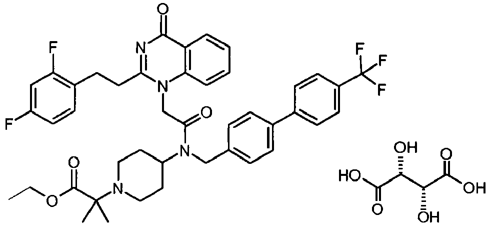

A mixture of [2-[2-(2,4-difluorophenyl)ethyl]-4-oxo-1(*4H*)-quinazolinyl]acetic acid (Int. C3) (120mg, 1 equiv), ethyl 2-methyl-2-[4-({[4'-(trifluoromethyl)-4-biphenylyl]methyl}-amino)-1-piperidinyl]propanoate (Int. B2) (204mg, 1.3 equiv), DMF (1.4ml) and DIPEA (0.183ml, 3 equiv) was shaken at room temperature, then HATU (206mg, 1.5 equiv) was added with vigorous agitation and shaking continued for 1.5h. A further portion of Intermediate D5 (12mg, 0.1 equiv) was added then shaking was continued for 2 days. Reverse phase HPLC (Preparative Method B) gave ethyl 2-[4-({[2-[2-(2,4-difluorophenyl)ethyl]-4-oxo-1(*4H*)-quinazolinyl]acetyl} {[4'-(trifluoromethyl)-4-biphenylyl]-methyl}amino)-1-piperidinyl]-2-methylpropanoate as a white solid (173mg).

LCMS Rt = 2.751 minutes; m/z [M+H]⁺ = 775.3

¹H NMR (CDCl₃) δ (mixture of rotomers) Characteristic peaks: 1.22-1.47 (9H, m), 1.63-2.10 (4H, m), 2.16-5.11 (15H, br m), 6.75-6.88 (2H, m), 7.14-7.80 (12H, m), 8.26-8.40 (1H, m).

This was converted to the bitartrate salt by a method analogous to that described in Example of Synthesis Approach (I).

### Example Synthesis Approach (IV)-7

### Ethyl 2-{4-[{[2-[2-(2,4-difluorophenyl)ethyl]-4-oxo-1(4H)-quinazolinyl]acetyl}({4'-[(trifluoromethyl)oxy]-4-biphenylyl}methyl)amino]-1-piperidinyl}-2-methylpropanoate 2,3-dihydroxybutanedioate (salt)

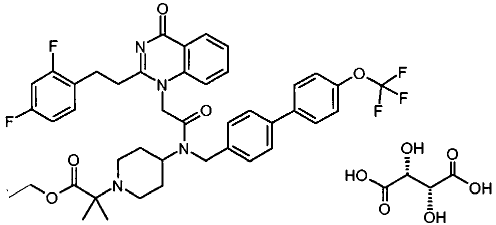

A mixture of [2-[2-(2,4-difluorophenyl)ethyl]-4-oxo-1(*4H*)-quinazolinyl]acetic acid (Int. C3) (114mg, 1.1 equiv), ethyl 2-methyl-2-{4-[({4'-[(trifluoromethyl)oxy]-4-biphenylyl}methyl)amino]-1-piperidiny1}propanoate (Int. B3) (139mg, 1 equiv), DMF (1.2ml) and DIPEA (0.16ml, 3 equiv) was shaken at room temperature, then HATU (176mg, 1.5 equiv) was added with vigorous agitation and shaking continued for 30 min. A further portion of Intermediate D5 (21mg, 0.2 equiv) was added, followed 1h later by further HATU (23mg, 0.2 equiv), then shaking was continued for 18h. Reverse phase HPLC (Preparative Method B) gave ethyl 2-{4-[{[2-[2-(2,4-difluorophenyl)ethyl]-4-oxo-1(*4H*)-quinazolinyl]-acetyl}({4'-[(trifluoromethyl)oxy]-4-biphenylyl}methyl)amino]-1-piperidinyl}-2-methylpropanoate as a white solid (149mg).

LCMS Rt = 2.793 minutes; m/z [M+H]⁺ = 791.3

¹H NMR (CDCl₃) Characteristic peaks: δ 1.20-1.45 (9H, m), 1.58-2.12 (4H, m), 2.14-2.48 (2H,m), 2.620-5.11 (11H, m), 6.59-6.72 (1H, m), 6.73-6.90 (2H, m), 7.16-7.64 (11H. m), 8.25-8.40 (1H, m).

This was converted to the bitartrate salt by a method analogous to that described in Example of Synthesis Approach (II).

### Example Synthesis Approach (IV)-8

### 2-[4-({[2-[2-(2,3-Difluorophenyl)ethyl]-4-oxo-1,8-naphthyridin-1(4H)-yl]acetyl} {[4'-(trifluoromethyl)-4-biphenylyl]methyl}amino)-1-piperidinyl]-2-methylpropanoic acid trifluoroacetate

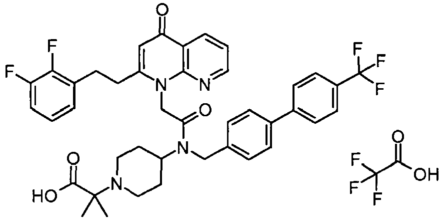

A mixture of 1,1-dimethylethyl 2-methyl-2-[4-({[4'-(trifluoromethyl)-4-biphenylyl]methyl}amino)-1-piperidinyl]propanoate (Int. B4) (1 equiv), [2-[2-(2,3-difluorophenyl)ethyl]-4-oxo-1,8-naphthyridin-1(*4H*)-yl]acetic acid (Int. C2) (1.2 equiv), DIPEA (3 equiv) and DMF (1.0ml) is stirred at room temperature for 5min. HATU (1.5 equiv) is added in 1 portion and stirred an additional 5 min. The crude reaction mixture is concentrated, filtered through a plug of silica eluted with acetone and evaporated to obtain crude 1,1-dimethylethyl 2-[4-({[2-[2-(2,3-difluorophenyl)ethyl]-4-oxo-1,8-naphthyridin-1(*4H*)-yl]acetyl} {[4'-(trifluoromethyl)-4-biphenylyl]methyl}amino)-1-piperidinyl]-2-methyl propanoate.

The proponate, without isolation, is dissolved in a 1:1 mixture of TFA and DCM and stirred at RT for 4h. Evaporation and prepative HPLC (Method A) gives the captioned compound.

Other salts can be prepared by conventional means. The free base can also be prepared by conventional means.

### Example Synthesis Approach (IV)-9

### 2-[4-({[2-[2-(2,3-Difluorophenyl)ethyl]-4-oxo-1(4H)-quinazolinyl]acetyl}{[4'-(trifluoromethyl)-4-biphenylyl]methyl}amino)-1-piperidinyl]-2-methylpropanoic acid trifluoroacetate

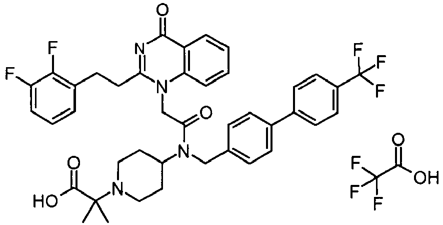

A mixture of 1,1-dimethylethyl 2-methyl-2-[4-({[4'-(trifluoromethyl)-4-biphenylyl]methyl}amino)-1-piperidinyl]propanoate (Int. B4) (1 equiv), [2-[2-(2,3-difluorophenyl)ethyl]-4-oxo-1(*4H*)-quinazolinyl] acetic acid (Int. C1) (1.2 equiv), DIPEA (3 equiv) and DMF (1.0ml) is stirred at room temperature for 5min. HATU (1.5 equiv) is added in 1 portion and stirred an additional 5 min. The crude reaction mixture is concentrated, filtered through a plug of silica eluted with acetone and evaporated to obtain crude 1,1-dimethylethyl 2-[4-({[2-[2-(2,3-difluorophenyl)ethyl]-4-oxo-1(4*H*)-quinazolinyl]acetyl} {[4'-(trifluoromethyl)-4-biphenylyl]methyl}amino)-1-piperidinyl]-2-methylpropanoate.

The proponate, without isolation, is dissolved in a 1:1 mixture of TFA and DCM and stirred at RT for 4h. Evaporation and prepative HPLC (Method A) gives the captioned compound.

### Example Synthesis Approach (IV)-10

### Methyl 2-[4-({[2-[2-(2,3-difluorophenyl)ethyl]-4-oxopyrido[2,3-d]pyrimidin-1(4H)-yl]acetyl} {[4'-(trifluoromethyl)-4-biphenylyl]methyl}amino)-1-piperidinyl]-2-methylpropanoate

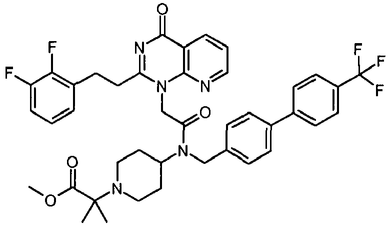

A mixture of [2-[2-(2,3-difluorophenyl)ethyl]-4-oxopyrido[2,3-d]pyrimidin-1(*4H*)-yl]acetic acid (Int. D1) (20.7g, 1.3 equiv), methyl 2-methyl-2-[4-({[4'-(trifluoromethyl)-4-biphenylyl]methyl}amino)-1-piperidinyl]propanoate (Int. B1) (20.0g, 1.3 equiv), DIPEA (24.0ml, 3 equiv) and DMF (184ml) was mechanically stirred, then HATU (27.1g, 1.5 equiv) was added in one portion and stirring continued for 2h. The reaction mixture was partioned between diethyl ether/THF (1:1) and sodium carbonate (1M, excess). The organic layer was washed with water and brine, dried and evaporated. Chromatography was run sequentially on three silica columns (firstly 3:1 EtOAc/hexanes; secondly 2% MeOH in DCM; thirdly 1:1 EtOAc/hexanes to 100% EtOAc). Product fractions were evaporated to obtain the desired product as an amorphous pink solid (27.5g).

LCMS Rt = 2.702 minutes; m/z [M+H]⁺ = 762.3

Crystallisation: A mixture of methyl 2-[4-({[2-[2-(2,3-difluorophenyl)ethyl]-4-oxopyrido[2,3-*d*]pyrimidin-1(*4H*)-yl]acetyl} {[4'-(trifluoromethyl)-4-biphenylyl]methyl}-amino)-1-piperidinyl]-2-methylpropanoate (8.0g) and ethanol (200ml) was warmed until fully dissolved. The solution was stirred magnetically for 24h at room temperature, then filtered and 7.5g of solid collected. These solvated crystals were placed into a 60°C vacuum oven with a nitrogen bleed to hold the vacuum at approximately 630 Torr for 24h to provide the unsolvated, crystalline title compound (7.15g), m.p. 150°C.

¹H NMR (CD₃OD) δ 1.25 (3H, s), 1.30 (3H, s), 1.63-1.99 (4H, m), 2.16-2.28 (1H, m), 2.3-2.43 (1H, m), 2.89-2.98 (1H, m), 2.98-3.08 (2H, m), 3.16-3.30 (3H, m), 3.66-3.69 (3H, m), 4.02/4.38 (1H, 2x br m), 4.69 (1H, s), 4.87 (1H, s), 5.4/5.73 (2H, 2x s), 6.99-7.19 (3H, m), 7.29-7.35 (1H, m), 7.50-7.61 (3H, m), 7.64-7.82 (5H, m), 8.48-8.57 (1H, m), 8.80-8.89 (1H, m) See Figure 1 below.

### Example Synthesis Approach (IV)-11

### Methyl 2-[4-({[2-[2-(2,3-difluorophenyl)ethyl]-4-oxopyrido[2,3-d]pyrimidin-1(4H)-yl]acetyl} {[4'-(trifluoromethyl)-4-biphenylyl]methyl}amino)-1-piperidinyl]-2-methylpropanoate 2,3-dihydroxybutanedioate (salt)

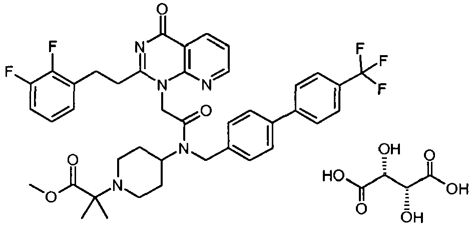

Methyl 2-[4-({[2-[2-(2,3-difluorophenyl)ethyl]-4-oxopyrido[2,3-*d*]pyrimidin-1(*4H*)-yl]acetyl} {[4'-(trifluoromethyl)-4-biphenylyl]methyl} amino)-1-piperidinyl]-2-methylpropanoate (8.5g, 1 equiv) was suspended in methanol (100ml) and warmed to 50°C until the solid dissolved. L-Tartaric acid (1.675g, 1.0 equiv) was added in one portion and stirred for 30 minutes at room temperature. The solution was concentrated in vacuo to an off-white powder that was dried in a vacuum oven at room temperature.

LCMS Rt = 2.697 minutes; m/z [M+H]⁺ = 762.3

¹H NMR (d₆-DMSO) □ 1.17 (3H, s), 1.23 (3H, s), 1.47-1.91 (4H, m), 1.98-2.41 (1H, m), 2.16-2.33 (1H, m), 2.80-3.26 (6H, m), 3.50-3.67 (3H, m), 3.95/4.17 (1H, 2x br m), 4.61 (1H, s), 4.85 (1H, s), 5.39/5.69 (2H, 2x s), 7.08-7.39 (4H, m), 7.53-7.70 (3H, m), 7.72-7.97 (5H, m), 8.42-8.54 (1H, m), 8.85-8.95 (1H, m)

### Example Synthesis Approach (IV)-12

### Ethyl 2-[4-({[2-[2-(2,3-difluorophenyl)ethyl]-4-oxopyrido[2,3-d]pyrimidin-1(4H)-yl]-acetyl} {[4'-(trifluoromethyl)-4-biphenylyl]methyl}amino)-1-piperidinyl]-2-methylpropanoate 2,3-dihydroxybutanedioate (salt)

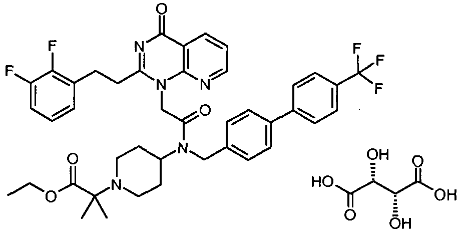

A mixture of [2-[2-(2,3-difluorophenyl)ethyl]-4-oxopyrido[2,3-*d*]pyrimidin-1(*4H*)-yl]acetic acid (Int. D1) (116mg, 1 equiv), ethyl 2-methyl-2-[4-({[4'-(trifluoromethyl)-4-biphenylyl]methyl}amino)-1-piperidinyl]propanoate (Int. B2) (150mg, 1 equiv), HATU (151mg, 1.2 equiv), DMF (2.72ml) and DIPEA (0.17ml, 3 equiv) was shaken at room temperature for 3.25h. The reaction mixture was partitioned between ethyl acetate/ methanol and aqueous sodium bicarbonate, the organic layer was brine-washed, dried and treated with activated charcoal (250mg). Flash chromatography (silica, 3-4% methanol in DCM) gave ethyl 2-[4-({[2-[2-(2,3-difluorophenyl)ethyl]-4-oxopyrido[2,3-*d*]pyrimidin-1(*4H*)-yl]-acetyl} {[4'-(trifluoromethyl)-4-biphenylyl]methyl} amino)-1-piperidinyl]-2-methylpropanoate as a white solid (178mg).

LCMS Rt = 2.58 minutes; m/z [M+H]⁺ = 776.3

¹H NMR (CDCl₃) δ 1.20-1.40 (9H, m), 1.56-2.02 (4H, m), 2.19-2.44 (2H, m), 2.88-3.20, (4H, m), 3.22-3.40 (2H, m), 3.81/4.58 (1H, 2x m), 4.11-4.27 (2H, m), 4.69/4.84 (2H, 2x s), 5.17/5.49 (2H, 2x s), 6.95-7.14 (3H, m), 7.25-7.31 (1H, m), 7.38-7.54 (3H, m), 7.54, 7.61 (1H, m), 7.62-7.79 (4H, m), 8.57-8.75 (2H, m)

This was converted to the bitartrate salt by a method analogous to that described for Example of Synthesis Approach (II).

### Examples Synthesis Approach (IV)-13

### Ethyl 2-{4-[{[2-[2-(2,3-difluorophenyl)ethyl]-4-oxopyrido[2,3-d]pyrimidin-1(4H)-yl]acetyl} ({4'-[(trifluoromethyl)oxy]-4-biphenylyl}methyl)amino]-1-piperidinyl}-2-methylpropanoate 2,3-dihydroxybutanedioate (salt)

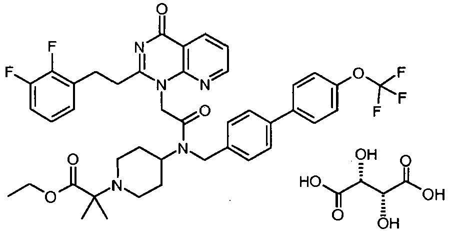

A mixture of [2-[2-(2,3-difluorophenyl)ethyl]-4-oxopyrido[2,3-*d*]pyrimidin-1(*4H*)-yl]acetic acid (Int. D1) (114mg, 1.1 equiv), ethyl 2-methyl-2-{4-[({4'-[(trifluoromethyl)oxy]-4-biphenylyl}methyl)amino]-1-piperidinyl}propanoate (Int. B4) (139mg, 1 equiv), DMF (1.2ml) and DIPEA (0.16ml, 3 equiv) was shaken at room temperature for 30 min, then HATU (176mg, 1.5 equiv) was added and shaking continued for 3h. Reverse phase HPLC (Preparative Method B) gave ethyl 2-{4-[{[2-[2-(2,3-difluorophenyl)ethyl]-4-oxo-1(*4H*)-quinazolinyl] acetyl} ({4'-[(trifluoromethyl)oxy]-4-biphenylyl}methyl)amino]-1-piperidinyl}-2-methylpropanoate as a white solid (166mg).

LCMS Rt = 2.87 minutes; m/z [M+H]⁺ = 792.3

¹H NMR (CDCl₃) δ 1.18-1.42 (9H, m), 1.54-2.04 (4H, m), 2.12-2.46 (2H, m), 2.86-3.21 (4H, m), 3.21-3.41 (2H, m), 3.79/4.57 (1H, 2x m), 4.10-4.27 (2H, m), 4.68 (1H, s), 4.82 (1H, s), 5.17 (1H, s), 5.47 (1H, s), 6.94-7.16 (3H, m), 7.20-7.36 (3H, m), 7.37-7.48 (3H, m), 7.48-7.61 (3H, m), 8.56-8.76 (2H, m).

This was converted to the bitartrate salt by a method analogous to that described for Example of Synthesis Approach (II).

### Example Synthesis Approach (IV)-14

1-Methylethyl 2-[4-({[2-[2-(2,3-difluorophenyl)ethyl]-4-oxopyrido[2,3-*d*]pyrimidin-1(*4H*)-yl]acetyl} {[4'-(trifluoromethyl)-4-biphenylyl]methyl}amino)-1-piperidinyl]-2-methylpropanoate 2,3-dihydroxybutanedioate (salt)

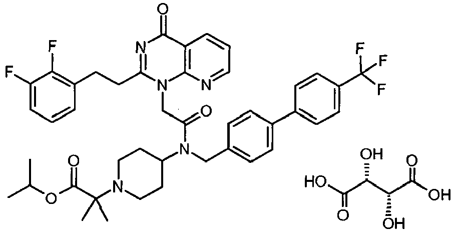

A mixture of 1-methylethyl 2-methyl-2-[4-({[4'-(trifluoromethyl)-4-biphenylyl]-methyl}amino)-1-piperidinyl]propanoate (Int. B3) (420 mg, 1 equiv), [2-[2-(2,3-difluorophenyl)ethyl]-4-oxopyrido[2,3-*d*]pyrimidin-1(*4H*)-yl]acetic acid (Int. D1) (300mg, 1 equiv), HATU (396mg, 1.2 equiv), DIPEA (0.22ml, 1.5 equiv) and DMF (3.0ml) was stirred at room temperature for 30 min. The crude reaction mixture was applied directly to reverse-phase HPLC (Preparative Method A) to obtain 1-methylethyl 2-[4-({[2-[2-(2,3-difluorophenyl)ethyl]-4-oxopyrido[2,3-*d*]pyrimidin-1(*4H*)-yl]acetyl}{[4'-(trifluoromethyl)-4-biphenylyl]methyl}amino)-1-piperidinyl]-2-methylpropanoate (171mag).

LCMS Rt = 2.837 minutes; m/z [M+H]⁺ = 790.3

¹H NMR (CD₃OD) δ 1.16-1.37 (12H, m), 1.62-2.01 (4H, m), 2.27-2.55 (2H, m), 2.95-3.12 (3H, m), 3.12-3.29 (3H, m), 4.06/4.40 (1H, 2x br m), 4.71 (1H, s), 4.89 (1H, s), 4.92-5.07 (1H, m), 5.43/5.76 (2H, 2x s), 7.00-7.21 (3H, m), 7.29-7.38 (1H, m), 7.49-7.65 (3H, m), 7.65-7.87 (5H, m), 8.48-8.58 (1H, m), 8.81-8.90 (1H, m).

This was converted to the bitartrate salt by a method analogous to that described for Example of Synthesis Approach (II).

### Example Synthesis Approach (IV)-15

### 1-Methylethyl 2-{4-[{[2-[2-(2,3-difluorophenyl)ethyl]-4-oxopyrido[2,3-d]pyrimidin-1(4H)-yl]acetyl}({4'-[(trifluoromethyl)oxy]-4-biphenylyl}methyl)amino]-1-piperidinyl}-2-methylpropanoate 2,3-dihydroxybutanedioate (salt)

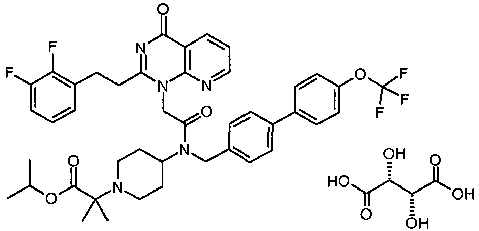

A mixture of 1-methylethyl 2-methyl-2-{4-[({4'-[(trifluoromethyl)oxy]-4-biphenylyl}methyl)amino]-1-piperidinyl}propanoate (Int. B5) (80 mg, 1 equiv), [2-[2-(2,3-difluorophenyl)ethyl]-4-oxopyrido[2,3-*d*]pyrimidin-1(*4H*)-yl]acetic acid (Int. D1) (67mg, 1 equiv), HATU (400mg, 5 equiv), DIPEA (0.22ml, 1.5 equiv) and DMF (2.0ml) was stirred at room temperature for 30 min. The crude reaction mixture was applied directly to reverse-phase HPLC (Preparative Method A) to obtain 1-methylethyl 2-{4-[{[2-[2-(2,3-difluorophenyl)ethyl]-4-oxopyrido[2,3-d]pyrimidin-1(*4H*)-yl]acetyl}({4'-[(trifluoromethyl)oxy]-4-biphenylyl}methyl)amino]-1-piperidinyl}-2-methylpropanoate (25mg).

LCMS Rt = 2.952 minutes; m/z [M+H]⁺ = 806.4

¹H NMR (DMSO-d6) δ 1.09-1.25 (12H, m), 1.47-1.91 (4H, m), 2.05-2.20 (1H, m), 2.21-2.38 (1H, m), 2.87-3.07 (3H, m), 3.08-3.22 (3H, m), 3.95/4.17 (1H, 2x br m), 4.59 (1H, s), 4.75-4.97 (2H, m), 5.38/5.68 (2H, 2x s), 7.90-7.21 (1H, m), 7.21-7.36 (3H, m), 7.42-7.55 (3H, m), 7.55-7-64 (2H, m), 7.66-7.77 (2H, m), 7.77-7.85 (1H, m), 8.43-8.52 (1H, m), 8.86-8.95 (1H, m)

This was converted to the bitartrate salt by a method analogous to that described for Example of Synthesis Approach (II).

### Example Synthesis Approach (IV)-16

### Methyl 2-[4-({[2-[2-(2,4-difluorophenyl)ethyl]-4-oxopyrido[2,3-d]pyrimidin-1(4H)-yl]acetyl}{[4'-(trifluoromethyl)-4-biphenylyl]methyl}amino)-1-piperidinyl]-2-methylpropanoate 2,3-dihydroxybutanedioate (salt)

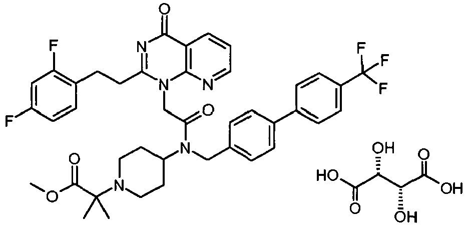

A mixture of [2-[2-(2,4-difluorophenyl)ethyl]-4-oxopyrido[2,4-d]pyrimidin-1(*4H*)-yl]acetic acid (Int. D2) (100mg, 1 equiv), methyl 2-methyl-2-[4-({[4'-(trifluoromethyl)-4-biphenylyl]methyl}amino)-1-piperidinyl]propanoate (Int. B1) (130mg, 1.03 equiv), DIPEA (0.16ml, 3 equiv), acetonitrile (2ml) and HATU (130mg, 1.2 equiv) was stirred at room temperature for 1h, then evaporated and redissolved in acetonitrile. Purification by reverse phase HPLC (Preparative Method B) gave methyl 2-[4-({[2-[2-(2,4-difluorophenyl)ethyl]-4-oxopyrido[2,3-*d*]pyrimidin-1(*4H*)-yl]acetyl}{[4'-(trifluoromethyl)-4-biphenylyl]methyl}amino)-1-piperidinyl]-2-methylpropanoate (145mg).

LCMS Rt = 2.716 minutes; m/z [M+H]⁺ = 762.3

¹H NMR (CDCl₃) δ 1.27 (3H, s), 1.33 (3H, s), 1.69-1.98 (4H, m), 2.22-2.29 (1H, m), 2.36-2.43 (1H, m), 2.96-3.08 (3H, m), 3.13-3.24 (3H, m), 3.69-3.72 (3H, m), 4.04/4.41 (1H, 2x br m), 4.72 (1H, s), 4.91 (1H, s), 5.41/5.73 (2H, 2x s), 6.84-6.97 (2H, m), 7.34-7.44 (2H, m), 7.54-7.63 (3H, m), 7.69-7.83 (5H, m), 8.55-8.60 (1H, m), 8.86-8.91 (1H, m).

This was converted to the bitartrate salt by a method analogous to that described for Example of Synthesis Approach (II).

### Example Synthesis Approach (IV)-17

### Ethyl 2-[4-({[2-[2-(2,4-difluorophenyl)ethyl]-4-oxopyrido[2,3-d]pyrimidin-1(4H)-yl]acetyl} {[4'-(trifluoromethyl)-4-biphenylyl]methyl}amino)-1-piperidinyl]-2-methylpropanoate 2,3-dihydroxybutanedioate (salt)

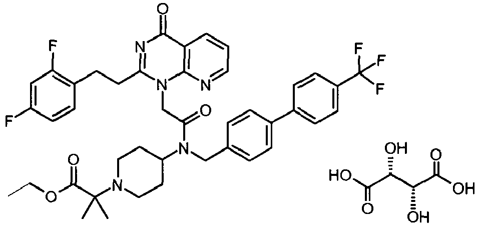

A mixture of [2-[2-(2,4-difluorophenyl)ethyl]-4-oxopyrido[2,4-d]pyrimidin-1(*4H*)-yl]acetic acid (Int. D2) (120mg, 1 equiv), ethyl 2-methyl-2-[4-({[4'-(trifluoromethyl)-4-biphenylyl]methyl}amino)-1-piperidinyl]propanoate (Int. B2) (198mg, 1.3 equiv), DMF (1.4ml) and DIPEA (0.178ml, 3 equiv) was shaken at room temperature for 1.5h, then HATU (200mg, 1.5 equiv) was added with vigorous agitation and shaking continued for 1.5h. A further portion of Intermediate D2 (12mg, 0.1 equiv) was added then shaking was continued for 2 days. Reverse phase HPLC (Preparative Method B) gave ethyl 2-[4-({[2-[2-(2,4-difluorophenyl)ethyl]-4-oxopyrido[2,3-d]pyrimidin-1(*4H*)-yl]acetyl} {[4'-(trifluoromethyl)-4-biphenylyl]methyl}amino)-1-piperidinyl]-2-methylpropanoate as a white solid (170mg).

LCMS Rt = 2.827 minutes; m/z [M+H]⁺ = 776.3

¹H NMR (CDCl₃) Characteristic peaks: δ 1.14-1.43 (9H, m), 1.57-2.05 (4H, m), 2.10-2.46 (2H, m), 2.84-3.11 (3H, m), 3.12-3.34 (3H, m), 3.65/3.85 (1H, m), 4.06-4.27 (2H, m), 4.65/4.85 (2H, s), 5.15/5.45 (2H, s), 6.62-6.89 (2H, m), 7.18-7.34 (1H, m), 7.37-7.82 (9H, m), 8.59-8.77 (2H, m).

This was converted to the bitartrate salt by a method analogous to that described for Example of Synthesis Approach (II).

### Example Synthesis Approach (IV)-18

Ethyl 2-{4-[{[2-[2-(2,4-difluorophenyl)ethyl]-4-oxopyrido[2,3-*d*]pyrimidin-1(*4H*)-yl]acetyl}({4'-[(trifluoromethyl)oxy]-4-biphenylyl}methyl)amino]-1-piperidinyl}-2-methylpropanoate 2,3-dihydroxybutanedioate (salt)

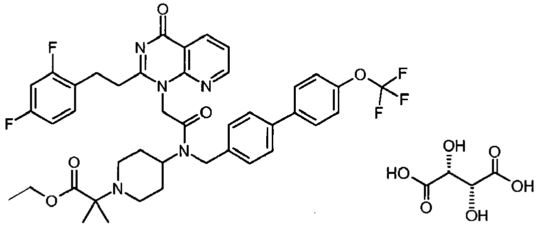

A mixture of [2-[2-(2,4-difluorophenyl)ethyl]-4-oxopyrido[2,4-d]pyrimidin-1(*4H*)-yl]acetic acid (Int. D2) (114mg, 1.1 equiv), ethyl 2-methyl-2-{4-[({4'-[(trifluoromethyl)oxy]-4-biphenylyl}methyl)amino]-1-piperidinyl}propanoate (Int. B4) (139mg, 1 equiv), DMF (1.2ml) and DIPEA (0.16ml, 3 equiv) was shaken at room temperature, then HATU (176mg, 1.5 equiv) was added with vigorous agitation and shaking continued for 2h. Reverse phase HPLC (Preparative Method B) gave ethyl 2-{4-[{[2-[2-(2,4-difluorophenyl)ethyl]-4-oxopyrido[2,3-*d*]pyrimidin-1(*4H*)-yl]acetyl}({4'-[(trifluoromethyl)oxy]-4-biphenylyl}-methyl)amino]-1-piperidinyl}-2-methylpropanoate as a white solid (149mg).

LCMS Rt = 2.801 minutes; m/z [M+H]⁺ = 792.3

¹H NMR (CDCl₃) δ 1.18-1.40 (9H, m), 1.61-2.02 (4H, m), 2.20-2.44 (2H, m), 2.83-3.35 (6H, br m), 3.79/4.57 (1H, 2x br m), 4.07-4.27 (2H, m), 4.68/4.81 (2H, 2x s), 5.14/5.46 (2H, 2x br m), 6.62-6.90 (2H, 2x m), 7.18-7.63 (10H, m), 8.59-8.75 (2H, m).

This was converted to the bitartrate salt by a method analogous to that described for Example of Synthesis Approach (II).

### Example Synthesis Approach (IV)-19

1-Methylethyl 2-[4-({[2-[2-(2,4-difluorophenyl)ethyl]-4-oxopyrido[2,3-*d*]pyrimidin-1(*4H*)-yl]acetyl} {[4'-(trifluoromethyl)-4-biphenylyl]methyl}amino)-1-piperidinyl]-2-methylpropanoate 2,3-dihydroxybutanedioate (salt)

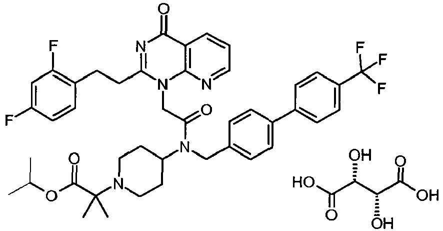

A mixture of 1-methylethyl 2-methyl-2-[4-({[4'-(trifluoromethyl)-4-biphenylyl]methyl}amino)-1-piperidinyl]propanoate (Int. B3) (70 mg, 1 equiv), [2-[2-(2,4-difluorophenyl)ethyl]-4-oxopyrido[2,4-d]pyrimidin-1(*4H*)-yl]acetic acid (Int. D2) (52.2mg, 1 equiv), HATU (69mg, 1.2 equiv), DIPEA (0.04ml, 1.5 equiv) and DMF (1.0ml) was stirred at room temperature for 10min. The crude reaction mixture was applied directly to reverse-phase HPLC (Preparative Method A) to obtain 1-methylethyl 2-[4-({[2-[2-(2,4-difluorophenyl)ethyl]-4-oxopyrido[2,3-*d*]pyrimidin-1(*4H*)-yl]acetyl} {[4'-(trifluoromethyl)-4-biphenylyl]methyl}amino)-1-piperidinyl]-2-methylpropanoate (20mg).

LCMS Rt = 2.910 minutes; m/z [M+H]⁺ = 790.4

¹H NMR (d₆-DMSO) δ 1.08-1.27 (12H, m), 1.40-1.90 (4H, m), 2.03-2.35 (2H, m), 2.85-3.24 (6H, m), 3.95/4.17 (1H, 2x br m), 4.61 (1H, s), 4.80-4.97 (2H, m), 5.36/5.67 (2H, 2x s), 6.96-7.10 (1H, m), 7.13-7.28 (1H, m), 7.28-7.38 (1H, m), 7.39-7.54 (1H, m), 7.54-7.68 (3H, m), 7.72-7.98 (5H, m), 8.43-8.52 (1H, m), 8.86-8.95 (1H, m)

This was converted to the bitartrate salt by a method analogous to that described for Example of Synthesis Approach (II).

### Example Synthesis Approach (IV)-20

### 1-Methylethyl 2-{4-[{[2-[2-(2,4-difluorophenyl)ethyl]-4-oxopyrido[2,3-d]pyrimidin-1(4H)-yl]acetyl}({4'-[(trifluoromethyl)oxy]-4-biphenylyl}methyl)amino]-1-piperidinyl}-2-methylpropanoate 2,3-dihydroxybutanedioate (salt)

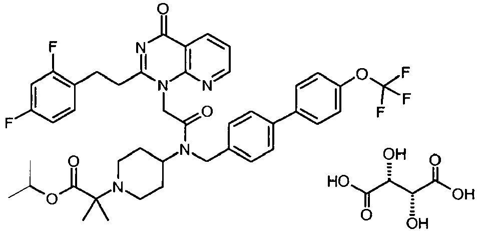

A mixture of 1-methylethyl 2-methyl-2-{4-[({4'-[(trifluoromethyl)oxy]-4-biphenylyl}methyl)amino]-1-piperidinyl}propanoate (Int. B5) (80 mg, 1 equiv), [2-[2-(2,3-difluorophenyl)ethyl]-4-oxopyrido[2,4-d]pyrimidin-1(*4H*)-yl]acetic acid (Int. D2) (57mg, 1 equiv), HATU (76mg, 1.2 equiv), DIPEA (0.04ml, 1.5 equiv) and DMF (1.0ml) was stirred at room temperature for 10min. The crude reaction mixture was applied directly to reverse-phase HPLC (Preparative Method A) to obtain 1-methylethyl 2-{4-[{[2-[2-(2,4-difluorophenyl)ethyl]-4-oxopyrido[2,3-*d*]pyrimidin-1(*4H*)-yl]acetyl}({4'-[(trifluoromethyl)-oxy]-4-biphenylyl}methyl)amino]-1-piperidinyl}-2-methylpropanoate (47mg).

LCMS Rt = 2.909 minutes; m/z [M+H]⁺ = 806.4

¹H NMR (d₆-DMSO) δ 1.09-1.27 (12H, m), 1.50-1.90 (4H, m), 2.03-2.17 (1H, m), 2.20-2.37 (1H, m), 2.88-3.18 (6H, m), 3.94/4.17 (1H, 2x m), 4.60 (1H, s), 4.74-4.96 (2H, m), 5.36/5.66 (2H, 2x br s), 6.96-7.09 (1H, m), 7.14-7.32 (2H, m), 7.39-7.55 (4H, m), 7.55-7.66 (2H, m), 7.66-7.87 (3H, m), 8.43-8.54 (1H, m), 8.85-8.96 (1H, m).

This was converted to the bitartrate salt by a method analogous to that described for Example of Synthesis Approach (II).

### Example Synthesis Approach (IV)-21

### Methyl 2-{4-[{[2-[2-(2,3-difluorophenyl)ethyl]-4-oxopyrido[2,3-d]pyrimidin-1(4H)-yl]acetyl}({4'-[(trifluoromethyl)oxy]-4-biphenylyl}methyl)amino]-1-piperidinyl}-2-methylpropanoate dihydroxybutanedioate (salt)

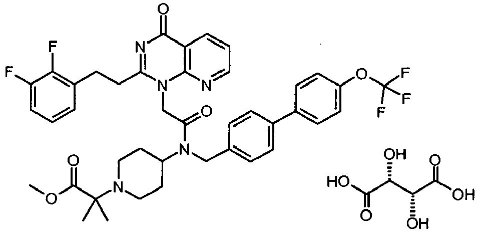

A mixture of methyl 2-methyl-2-{4-[({4'-[(trifluoromethyl)oxy]-4-biphenylyl}methyl)amino]-1 piperidinyl}propanoate (Int. B6) (145 mg, 1 equiv), [2-[2-(2,3-difluorophenyl)ethyl]-4-oxopyrido[2,3-*d*]pyrimidin-1(*4H*)-yl]acetic acid (Int. D1) (122mg, 1 equiv), DIPEA (0.084ml, 1.5 equiv) and DMF (2.0ml) was stirred at room temperature for 5min. The HATU (160mg, 1.3 equiv) was added in 1 portion and stirred an additional 1 hour under nitrogen. The crude reaction mixture was applied directly to reverse-phase HPLC (Preparative Method A) to obtain methyl 2-{4-[{[2-[2-(2,3-difluorophenyl)ethyl]-4-oxopyrido[2,3-*d*]pyrimidin-1(*4H*)-yl]acetyl}({4'-[(trifluoromethyl)oxy]-4-biphenylyl}methyl)amino]-1-piperidinyl}-2-methylpropanoate (116mg).

LCMS Rt = 2.721 minutes; m/z [M+H]⁺ = 778.3

¹H NMR (CDCl₃) Characteristic peaks: δ 1.53-1.62 (6H, m), 3.46-5.99 (22H, m), 7.01-7.21 (3H, m), 7.30-7.43 (3H, m), 7.50-7.78 (6H, m), 8.54-8.60 (1H, m), 8.86-8.94 (1H, m).

This was converted to the bitartrate salt by a method analogous to that described for Example of Synthesis Approach (II).

### Examples Synthesis Approach (IV)-22

### 2-[4-({[2-[2-(2,3-Difluorophenyl)ethyl]-4-oxopyrido[2,3-d]pyrimidin-1(4H)-yl]acetyl} {[4'-(trifluoromethyl)-4-biphenylyl]methyl} amino)-1-piperidinyl]-2-methylpropanoic acid trifluoroacetate

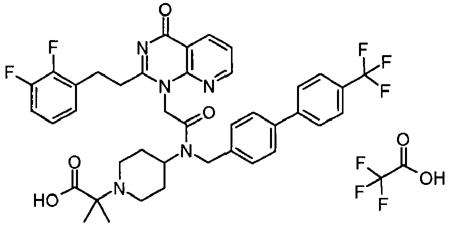

A mixture of 1,1-dimethylethyl 2-methyl-2-[4-({[4'-(trifluoromethyl)-4-biphenylyl]methyl}amino)-1-piperidinyl]propanoate (Int. B7) (150 mg, 1 equiv), [2-[2-(2,3-difluorophenyl)ethyl]-4-oxopyrido[2,3-*d*]pyrimidin-1(*4H*)-yl]acetic acid (Int. D1) (130mg, 1.2 equiv), DIPEA (0.164ml, 3 equiv) and DMF (1.0ml) was stirred at room temperature for 5min. HATU (180mg, 1.5 equiv) was added in 1 portion and stirred an additional 5 min. The crude reaction mixture was concentrated, filtered through a plug of silica eluted with acetone and evaporated to obtain crude 1,1-dimethylethyl 2-[4-({[2-[2-(2,3-difluorophenyl)ethyl]-4-oxopyrido[2,3-*d*]pyrimidin-1(*4H*)-yl]acetyl} {[4'-(trifluoromethyl)-4-biphenylyl]methyl}amino)-1-piperidinyl]-2-methylpropanoate.

LCMS Rt = 2.823 minutes; m/z [M+H]⁺ = 804.4

This intermediate, without isolation, was dissolved in a 1:1 mixture of TFA and DCM and stirred at RT for 4h. Evaporation and prepative HPLC (Method A) gave the desired 2-[4-({[2-[2-(2,3-difluorophenyl)ethyl]-4-oxopyrido[2,3-*d*]pyrimidin-1(*4H*)-yl]acetyl} {[4'-(trifluoromethyl)-4-biphenylyl]methyl}amino)-1-piperidinyl]-2-methylpropanoic acid trifluoroacetate (70mg).

LCMS Rt = 2.554 minutes; m/z [M+H]⁺ = 748.2

¹H NMR (d₆-DMSO) d 1.44 (3H, s), 1.51 (3H, s), 1.70-2.30 (4H, m), 2.41-2.56 (2H, m), 2.94-3.54 (6H, m), 4.44-4.95 (3H, m), 5.42/5.76 (2H, 2x br s), 7.07-7.38 (4H, m), 7.54-7.75 (3H, m), 7.76-7.99 (5H, m), 8.42-8.54 (1H, m), 8.85-8.98 (1H, m).

Other salts can be prepared by conventional means. The free base can also be prepared by conventional means.

In some embodiments, compounds useful as inhibitors of Lp-PLA2 useful in the methods as described herein are:

1-(N-(2-(diethylamino)ethyl)-N-(4-(4-trifluoromethylphenyl)benzyl)-aminocarbonylmethyl)-2-(4-fluorobenzyl)thio-5,6-trimethylenepyrimidin-4-one, also referred to as "SB480848" or the USAN name "darapladib" which is a pyrimidinone-based compound and a reversible inhibitor of Lp-PLA₂ and is used in the Examples herein,

*N*-(2-diethylaminoethyl)-2-[2-(2-(2,3-difluorophenyl)ethyl)-4-oxo-4,5,6,7-tetrahydrocyclopentapyrimidin-1-yl]-*N*-(4'-trifluoromethyl-biphenyl-4-ylmethyl)acetamide;

*N*-(1-(2-Methoxyethyl)piperidin-4-yl)-2-[2-(2,3-difluorobenzylthio)-4-oxo-4*H-*quinolin-1-yl]-*N*-(4'-trifluoromethylbiphenyl-4-ylmethyl)acetamide; and

methyl 2-[4-({[2-[2-(2,3-difluorophenyl)ethyl]-4-oxopyrido[2,3-d]pyrimidin-1(*4H*)-yl]acetyl} {[4'-(trifluoromethyl)-4-biphenylyl]methyl} amino)-1-piperidinyl]-2-methylpropanoate.

Pharmaceutically acceptable salts of 1-(N-(2-(diethylamino)ethyl)-N-(4-(4-trifluoromethylphenyl)benzyl)-aminocarbonylmethyl)-2-(4-fluorobenzyl)thio-5,6-trimethylenepyrimidin-4-one, AKA SB480848, and used in the Examples herein; N-(2-diethylaminoethyl)-2-[2-(2-(2,3-difluorophenyl)ethyl)-4-oxo-4,5,6,7-tetrahydrocyclopentapyrimidin-1-yl]-*N*-(4'-trifluoromethyl-biphenyl-4-ylmethyl)acetamide; *N*-(1-(2-Methoxyethyl)piperidin-4-yl)-2-[2-(2,3-difluorobenzylthio)-4-oxo-*4H*-quinolin-1-yl]-*N*-(4'-trifluoromethylbiphenyl-4-ylmethyl)acetamide; and methyl 2-[4-({[2-[2-(2,3-difluorophenyl)ethyl]-4-oxopyrido[2,3-*d*]pyrimidin-1(*4H*)acetyl} {[4'-(trifluoromethyl)-4-biphenylyl]methyl}amino)-1-piperidinyl]-2-methylpropanoate are also useful as inhibitors of Lp-PLA₂ for use in the methods as described herein.

### Nucleic acid inhibitors of Lp-PLA₂

Agents that inhibit Lp-PLA₂ may be nucleic acids. Nucleic acid inhibitors of Lp-PLA₂ may be, for example, RNA interference-inducing molecules, for example but are not limited to siRNA, dsRNA, stRNA, shRNA and modified versions thereof, where the RNA interference molecule silences the gene expression of Lp-PLA₂. The nucleic acid inhibitor of Lp-PLA₂ may be an anti-sense oligonucleic acid, or a nucleic acid analogue, for example but are not limited to DNA, RNA, peptide-nucleic acid (PNA), pseudo-complementary PNA (pcPNA), or locked nucleic acid (LNA) and the like. The nucleic acid may be DNA or RNA, and nucleic acid analogues, for example PNA, pcPNA and LNA. A nucleic acid can be single or double stranded, and can be selected from a group comprising nucleic acid encoding a protein of interest, oligonucleotides, PNA, etc. Such nucleic acid sequences include, for example, but are not limited to, nucleic acid sequence encoding proteins that act as transcriptional repressors, antisense molecules, ribozymes, small inhibitory nucleic acid sequences, for example but are not limited to RNAi, shRNAi, siRNA, micro RNAi (mRNAi), antisense oligonucleotides etc.

Single-stranded RNA (ssRNA), a form of RNA endogenously found in eukaryotic cells can be used to form an RNAi molecule. Cellular ssRNA molecules include messenger RNAs (and the progenitor pre-messenger RNAs), small nuclear RNAs, small nucleolar RNAs, transfer RNAs and ribosomal RNAs. Double-stranded RNA (dsRNA) induces a size-dependent immune response such that dsRNA larger than 30bp activates the interferon response, while shorter dsRNAs feed into the cell's endogenous RNA interference machinery downstream of the Dicer enzyme.

Lp-PLA₂ can be reduced by inhibition of the expression of Lp-PLA₂ polypeptide or by "gene silencing" methods commonly known by persons of ordinary skill in the art.

RNA interference (RNAi) provides a powerful approach for inhibiting the expression of selected target polypeptides. RNAi uses small interfering RNA (siRNA) duplexes that target the messenger RNA encoding the target polypeptide for selective degradation. siRNA-dependent post-transcriptional silencing of gene expression involves cutting the target messenger RNA molecule at a site guided by the siRNA.

RNA interference (RNAi) is an evolutionally conserved process whereby the expression or introduction of RNA of a sequence that is identical or highly similar to a target gene results in the sequence specific degradation or specific post-transcriptional gene silencing (PTGS) of messenger RNA (mRNA) transcribed from that targeted gene (*see* Coburn, G. and Cullen, B. (2002) J. of Virology 76(18):9225), thereby inhibiting expression of the target gene. The RNA may be double stranded RNA (dsRNA). This process has been described in plants, invertebrates, and mammalian cells. In nature, RNAi is initiated by the dsRNA-specific endonuclease Dicer, which promotes processive cleavage of long dsRNA into double-stranded fragments termed siRNAs. siRNAs are incorporated into a protein complex (termed "RNA induced silencing complex," or "RISC") that recognizes and cleaves target mRNAs. RNAi can also be initiated by introducing nucleic acid molecules, e.g., synthetic siRNAs or RNA interfering agents, to inhibit or silence the expression of target genes. As used herein, "inhibition of target gene expression" includes any decrease in expression or protein activity or level of the target gene or protein encoded by the target gene as compared to a situation wherein no RNA interference has been induced. The decrease can be of at least 30%, 40%, 50%, 60%, 70%, 80%, 90%, 95% or 99% or more as compared to the expression of a target gene or the activity or level of the protein encoded by a target gene which has not been targeted by an RNA interfering agent.

"Short interfering RNA" (siRNA), also referred to herein as "small interfering RNA" is defined as an agent which functions to inhibit expression of a target gene, *e.g.,* by RNAi. An siRNA can be chemically synthesized, can be produced by *in vitro* transcription, or can be produced within a host cell. siRNA may be a double stranded RNA (dsRNA) molecule of about 15 to about 40 nucleotides in length, preferably about 15 to about 28 nucleotides, more preferably about 19 to about 25 nucleotides in length, and more preferably about 19, 20, 21, 22, or 23 nucleotides in length, and can contain a 3' and/or 5' overhang on each strand having a length of about 0, 1, 2, 3, 4, or 5 nucleotides. The length of the overhang is independent between the two strands, *i.e.,* the length of the overhang on one strand is not dependent on the length of the overhang on the second strand. Preferably the siRNA is capable of promoting RNA interference through degradation or specific post-transcriptional gene silencing (PTGS) of the target messenger RNA (mRNA).

siRNAs also include small hairpin (also called stem loop) RNAs (shRNAs). These shRNAs may be composed of a short (*e.g.,* about 19 to about 25 nucleotide) antisense strand, followed by a nucleotide loop of about 5 to about 9 nucleotides, and the analogous sense strand. Alternatively, the sense strand can precede the nucleotide loop structure and the antisense strand can follow. These shRNAs can be contained in plasmids, retroviruses, and lentiviruses and expressed from, for example, the pol III U6 promoter, or another promoter (*see, e.g.,* Stewart, et al. (2003) RNA Apr;9(4):493-501).

The target gene or sequence of the RNA interfering agent can be a cellular gene or genomic sequence, e.g. the Lp-PLA₂ sequence. An siRNA can be substantially homologous to the target gene or genomic sequence, or a fragment thereof. As used in this context, the term "homologous" is defined as being substantially identical, sufficiently complementary, or similar to the target mRNA, or a fragment thereof, to effect RNA interference of the target. In addition to native RNA molecules, RNA suitable for inhibiting or interfering with the expression of a target sequence include RNA derivatives and analogs. Preferably, the siRNA is identical to its target.

The siRNA preferably targets only one sequence. Each of the RNA interfering agents, such as siRNAs, can be screened for potential off-target effects by, for example, expression profiling. Such methods are known to one skilled in the art and are described, for example, in Jackson et al, Nature Biotechnology 6:635-637, 2003. In addition to expression profiling, one can also screen the potential target sequences for similar sequences in the sequence databases to identify potential sequences which can have off-target effects. For example, according to Jackson et al. (Id.) 15, or perhaps as few as 11 contiguous nucleotides of sequence identity are sufficient to direct silencing of non-targeted transcripts. Therefore, one can initially screen the proposed siRNAs to avoid potential off-target silencing using the sequence identity analysis by any known sequence comparison methods, such as BLAST.

siRNA molecules need not be limited to those molecules containing only RNA, but, for example, further encompasses chemically modified nucleotides and non-nucleotides, and also include molecules wherein a ribose sugar molecule is substituted for another sugar molecule or a molecule which performs a similar function. Moreover, a non-natural linkage between nucleotide residues can be used, such as a phosphorothioate linkage. For example, siRNA containing D-arabinofuranosyl structures in place of the naturally-occurring D-ribonucleosides found in RNA can be used in RNAi molecules (U.S. Pat. No. 5,177,196). Other examples include RNA molecules containing the o-linkage between the sugar and the heterocyclic base of the nucleoside, which confers nuclease resistance and tight complementary strand binding to the oligonucleotidesmolecules similar to the oligonucleotides containing 2'-O-methyl ribose, arabinose and particularly D-arabinose (U.S. Pat. No. 5,177,196).

The RNA strand can be derivatized with a reactive functional group of a reporter group, such as a fluorophore. Particularly useful derivatives are modified at a terminus or termini of an RNA strand, typically the 3' terminus of the sense strand. For example, the 2'-hydroxyl at the 3' terminus can be readily and selectively derivatized with a variety of groups.

Other useful RNA derivatives incorporate nucleotides having modified carbohydrate moieties, such as 2'O-alkylated residues or 2'-O-methyl ribosyl derivatives and 2'-O-fluoro ribosyl derivatives. The RNA bases can also be modified. Any modified base useful for inhibiting or interfering with the expression of a target sequence can be used. For example, halogenated bases, such as 5-bromouracil and 5-iodouracil can be incorporated. The bases can also be alkylated, for example, 7-methylguanosine can be incorporated in place of a guanosine residue. Non-natural bases that yield successful inhibition can also be incorporated.

The most preferred siRNA modifications include 2'-deoxy-2'-fluorouridine or locked nucleic acid (LNA) nucleotides and RNA duplexes containing either phosphodiester or varying numbers of phosphorothioate linkages. Such modifications are known to one skilled in the art and are described, for example, in Braasch et al., Biochemistry, 42: 7967-7975, 2003. Most of the useful modifications to the siRNA molecules can be introduced using chemistries established for antisense oligonucleotide technology. Preferably, the modifications involve minimal 2'-O-methyl modification, preferably excluding such modification. Modifications also preferably exclude modifications of the free 5'-hydroxyl groups of the siRNA.

siRNA and miRNA molecules having various "tails" covalently attached to either their 3'- or to their 5'-ends, or to both, are also known in the art and can be used to stabilize the siRNA and miRNA molecules delivered using the methods described herein. Generally speaking, intercalating groups, various kinds of reporter groups and lipophilic groups attached to the 3' or 5' ends of the RNA molecules are well known to one skilled in the art and are useful according to the methods described herein. Descriptions of syntheses of 3'-cholesterol or 3'-acridine modified oligonucleotides applicable to preparation of modified RNA molecules useful according to the described methods can be found, for example, in the articles: Gamper, H. B., Reed, M. W., Cox, T., Virosco, J. S., Adams, A. D., Gall, A., Scholler, J. K., and Meyer, R. B. (1993) Facile Preparation and Exonuclease Stability of 3'-Modified Oligodeoxynucleotides. Nucleic Acids Res. 21 145-150; and Reed, M. W., Adams, A. D., Nelson, J. S., and Meyer, R. B.,Jr. (1991) Acridine and Cholesterol-Derivatized Solid Supports for Improved Synthesis of 3'-Modified Oligonucleotides. Bioconjugate Chem. 2 217-225 (1993).

Other siRNAs useful for targeting Lp-PLA₂ expression can be readily designed and tested. Accordingly, siRNAs useful for the methods described herein include siRNA molecules of about 15 to about 40 or about 15 to about 28 nucleotides in length, which are homologous to an Lp-PLA₂ gene. Preferably, the Lp-PLA₂ targeting siRNA molecules have a length of about 19 to about 25 nucleotides. More preferably, the Lp-PLA₂ targeting siRNA molecules have a length of about 19, 20, 21, or 22 nucleotides. The Lp-PLA₂ targeting siRNA molecules can also comprise a 3' hydroxyl group. The Lp-PLA₂ targeting siRNA molecules can be single-stranded or double stranded; such molecules can be blunt ended or comprise overhanging ends (*e.g.,* 5', 3'). The RNA molecule may be double stranded and either blunt ended or comprises overhanging ends.

At least one strand of the Lp-PLA₂ targeting RNA molecule may have a 3' overhang from about 0 to about 6 nucleotides (e.g., pyrimidine nucleotides, purine nucleotides) in length. The 3' overhang may be from about 1 to about 5 nucleotides, from about 1 to about 3 nucleotides and from about 2 to about 4 nucleotides in length. The Lp-PLA₂ targeting RNA molecule may be double stranded - one strand having a 3' overhang and the other strand can be blunt-ended or have an overhang. Where the Lp-PLA₂ targeting RNA molecule is double stranded and both strands comprise an overhang, the length of the overhangs can be the same or different for each strand. The RNA may comprise about 19, 20, 21, or 22 nucleotides which are paired and which have overhangs of from about 1 to about 3, particularly about 2, nucleotides on both 3' ends of the RNA. The 3' overhangs can be stabilized against degradation. The RNA may be stabilized by including purine nucleotides, such as adenosine or guanosine nucleotides. Alternatively, substitution of pyrimidine nucleotides by modified analogues, *e.g.,* substitution of uridine 2 nucleotide 3' overhangs by 2'-deoxythymidine is tolerated and does not affect the efficiency of RNAi. The absence of a 2' hydroxyl significantly enhances the nuclease resistance of the overhang in tissue culture medium.

Lp-PLA₂ mRNA has been successfully targeted using siRNAs and such siRNA or vectors for preparing them are commercially available, for example from Invitrogen. Assessment of the expression and/or knock down of Lp-PLA₂ protein using such Lp-PLA₂ siRNAs can be determined using commercially available kits, for example but are not limited to PLAC assay from diaDexus. Others can be readily prepared by those of skill in the art based on the known sequence of the target mRNA. To avoid doubt, the sequence of a human Lp-PLA₂ cDNA is provided at, for example, GenBank Accession Nos. : U20157 (SEQ ID NO:1) or NM_005084 (SEQ ID NO:2). The sequence at U20157 is the following (SEQ ID NO:1):

1 gctggtcgga ggctcgcagt gctgtcggcg agaagcagtc gggtttggag cgcttgggtc
61 gcgttggtgc gcggtggaac gcgcccaggg accccagttc ccgcgagcag ctccgcgccg
121 cgcctgagag actaagctga aactgctgct cagctcccaa gatggtgcca cccaaattgc
181 atgtgctttt ctgcctctgc ggctgcctgg ctgtggttta tccttttgac tggcaataca
241 taaatcctgt tgcccatatg aaatcatcag catgggtcaa caaaatacaa gtactgatgg
301 ctgctgcaag ctttggccaa actaaaatcc cccggggaaa tgggccttat tccgttggtt
361 gtacagactt aatgtttgat cacactaata agggcacctt cttgcgttta tattatccat
421 cccaagataa tgatcgcctt gacacccttt ggatcccaaa taaagaatat ttttgggtc
481 ttagcaaatt tcttggaaca cactggctta tgggcaacat tttgaggtta ctctttggtt
541 caatgacaac tcctgcaaac tggaattccc ctctgaggcc tggtgaaaaa tatccacttg
601 ttgttttttc tcatggtctt ggggcattca ggacacttta ttctgctatt ggcattgacc
661 tggcatctca tgggtttata gttgctgctg tagaacacag agatagatct gcatctgcaa
721 cttactattt caaggaccaa tctgctgcag aaatagggga caagtcttgg ctctacctta
781 gaaccctgaa acaagaggag gagacacata tacgaaatga gcaggtacgg caaagagcaa
841 aagaatgttc ccaagctctc agtctgattc ttgacattga tcatggaaag ccagtgaaga
901 atgcattaga tttaaagttt gatatggaac aactgaagga ctctattgat agggaaaaaa
961 tagcagtaat tggacattct tttggtggag caacggttat tcagactctt agtgaagatc
1021 agagattcag atgtggtatt gccctggatg catggatgtt tccactgggt gatgaagtat
1081 attccagaat tcctcagccc ctctttttta tcaactctga atatttccaa tatcctgcta
1141 atatcataaa aatgaaaaaa tgctactcac ctgataaaga aagaaagatg attacaatca
1201 ggggttcagt ccaccagaat tttgctgact tcacttttgc aactggcaaa ataattggac
1261 acatgctcaa attaaaggga gacatagatt caaatgtagc tattgatctt agcaacaaag
1321 cttcattagc attcttacaa aagcatttag gacttcataa agattttgat cagtgggact
1381 gcttgattga aggagatgat gagaatctta ttccagggac caacattaac acaaccaatc
1441 aacacatcat gttacagaac tcttcaggaa tagagaaata caattaggat taaaataggt
1501 ttttt

siRNA sequences are chosen to maximize the uptake of the antisense (guide) strand of the siRNA into RISC and thereby maximize the ability of RISC to target human Lp-PLA₂ mRNA for degradation. This can be accomplished by scanning for sequences that have the lowest free energy of binding at the 5'-terminus of the antisense strand. The lower free energy leads to an enhancement of the unwinding of the 5'- end of the antisense strand of the siRNA duplex, thereby ensuring that the antisense strand will be taken up by RISC and direct the sequence-specific cleavage of the human Lp-PLA₂ mRNA.

The siRNA or modified siRNA may be delivered in a pharmaceutically acceptable carrier. Additional carrier agents, such as liposomes, can be added to the pharmaceutically acceptable carrier.

The siRNA may be delivered by delivering a vector encoding small hairpin RNA (shRNA) in a pharmaceutically acceptable carrier to the cells in an organ of an individual. The shRNA is converted by the cells after transcription into siRNA capable of targeting, for example, Lp-PLA₂. The vector can be a regulatable vector, such as tetracycline inducible vector.

The RNA interfering agents used in the methods described herein are taken up actively by cells *in vivo* following intravenous injection, *e.g.,* hydrodynamic injection, without the use of a vector, illustrating efficient *in vivo* delivery of the RNA interfering agents.

Other strategies for delivery of the RNA interfering agents, *e.g.,* the siRNAs or shRNAs, can also be employed, such as, for example, delivery by a vector, *e.g.,* a plasmid or viral vector, *e.g.,* a lentiviral vector. Such vectors can be used as described, for example, in Xiao-Feng Qin et al. Proc. Natl. Acad. Sci. U.S.A., 100: 183-188. Other delivery methods include delivery of the RNA interfering agents, *e.g.,* the siRNAs or shRNAs, using a basic peptide by conjugating or mixing the RNA interfering agent with a basic peptide, *e.g.,* a fragment of a TAT peptide, mixing with cationic lipids or formulating into particles.

As noted, the dsRNA, such as siRNA or shRNA can be delivered using an inducible vector, such as a tetracycline inducible vector. Methods described, for example, in Wang et al. Proc. Natl. Acad. Sci. 100: 5103-5106, using pTet-On vectors (BD Biosciences Clontech, Palo Alto, CA) can be used. A vector can be a plasmid vector, a viral vector, or any other suitable vehicle adapted for the insertion and foreign sequence and for the introduction into eukaryotic cells. The vector can be an expression vector capable of directing the transcription of the DNA sequence of the agonist or antagonist nucleic acid molecules into RNA. Viral expression vectors can be selected from a group comprising, for example, retroviruses, lentiviruses, Epstein Barr virus-, bovine papilloma virus, adenovirus- and adeno-associated-based vectors or hybrid virus of any of the above. The vector may be episomal. The use of a suitable episomal vector provides a means of maintaining the antagonist nucleic acid molecule in the subject in high copy number extra chromosomal DNA thereby eliminating potential effects of chromosomal integration.

RNA interference molecules and nucleic acid inhibitors useful in the methods as described herein can be produced using any known techniques such as direct chemical synthesis, through processing of longer double stranded RNAs by exposure to recombinant Dicer protein or Drosophila embryo lysates, through an in vitro system derived from S2 cells, using phage RNA polymerase, RNA-dependant RNA polymerase, and DNA based vectors. Use of cell lysates or in vitro processing can further involve the subsequent isolation of the short, for example, about 21-23 nucleotide, siRNAs from the lysate, etc. Chemical synthesis usually proceeds by making two single stranded RNA-oligomers followed by the annealing of the two single stranded oligomers into a double stranded RNA. Other examples include methods disclosed in WO 99/32619 and WO 01/68836 that teach chemical and enzymatic synthesis of siRNA. Moreover, numerous commercial services are available for designing and manufacturing specific siRNAs (see, e.g., QIAGEN Inc., Valencia, CA and AMBION Inc., Austin, TX)

The agent may be protein or polypeptide or RNAi agent that inhibits expression of Lp-PLA and/or activity of the Lp-PLA₂ protein. In this case cells can be modified (e.g., by homologous recombination) to provide increased expression of such an agent, for example by replacing, in whole or in part, the naturally occurring promoter with all or part of a heterologous promoter so that the cells express the natural inhibitor agent of Lp-PLA₂, for example protein or miRNA inhibitor of Lp-PLA₂ at higher levels. The heterologous promoter is inserted in such a manner that it is operatively linked to the desired nucleic acid encoding the agent. See, for example, PCT International Publication No. WO 94/12650 by Transkaryotic Therapies, Inc., PCT International Publication No. WO 92/20808 by Cell Genesys, Inc., and PCT International Publication No. WO 91/09955 by Applied Research Systems. Cells also can be engineered to express an endogenous gene comprising the agent under the control of inducible regulatory elements, in which case the regulatory sequences of the endogenous gene can be replaced by homologous recombination. Gene activation techniques are described in U.S. Patent No. 5,272,071 to Chappel; U.S. Patent No. 5,578,461 to Sherwin et al.; PCT/US92/09627 (WO93/09222) by Selden et al.; and PCT/US90/06436 (WO91/06667) by Skoultchi et al. The agent can be prepared by culturing transformed host cells under culture conditions suitable to express the miRNA. The resulting expressed agent can then be purified from such culture (i.e., from culture medium or cell extracts) using known purification processes, such as gel filtration and ion exchange chromatography. The purification of the peptide or nucleic acid agent inhibitor of Lp-PLA₂ can also include an affinity column containing agents which will bind to the protein; one or more column steps over such affinity resins as concanavalin A-agarose, heparin-toyopearl^{™} or Cibacrom blue 3GA Sepharose; one or more steps involving hydrophobic interaction chromatography using such resins as phenyl ether, butyl ether, or propyl ether; immunoaffnity chromatography, or complementary cDNA affinity chromatography.

The nucleic acid inhibitors of Lp-PLA₂ can be obtained synthetically, for example, by chemically synthesizing a nucleic acid by any method of synthesis known to the skilled artisan. The synthesized nucleic acid inhibitors of Lp-PLA₂ can then be purified by any method known in the art. Methods for chemical synthesis of nucleic acids include, but are not limited to, in vitro chemical synthesis using phosphotriester, phosphate or phosphoramidite chemistry and solid phase techniques, or via deoxynucleoside H-phosphonate intermediates (see U.S. Patent No. 5,705,629 to Bhongle).

In some circumstances, for example, where increased nuclease stability is desired, nucleic acids having nucleic acid analogs and/or modified internucleoside linkages can be preferred. Nucleic acids containing modified internucleoside linkages can also be synthesized using reagents and methods that are well known in the art. For example, methods of synthesizing nucleic acids containing phosphonate phosphorothioate, phosphorodithioate, phosphoramidate methoxyethyl phosphoramidate, formacetal, thioformacetal, diisopropylsilyl, acetamidate, carbamate, dimethylene-sulfide (-CH₂-S-CH₂), diinethylene-sulfoxide (-CH₂-SO-CH₂), dimethylene-sulfone (-CH₂-SO₂-CH₂), 2'-O-alkyl, and 2'-deoxy-2'-fluoro' phosphorothioate internucleoside linkages are well known in the art (see Uhlmann et al., 1990, Chem. Rev. 90:543-584; Schneider et al., 1990, Tetrahedron Lett. 31:335 and references cited therein). U.S. Patent Nos. 5,614,617 and 5,223,618 to Cook, et al., 5,714, 606 to Acevedo, et al, 5,378,825 to Cook, et al., 5,672,697 and 5,466, 786 to Buhr, et al., 5, 777,092 to Cook, et al., 5,602,240 to De Mesmacker, et al., 5,610,289 to Cook, et al. and 5,858,988 to Wang, also describe nucleic acid analogs for enhanced nuclease stability and cellular uptake.

Synthetic siRNA molecules, including shRNA molecules, can be obtained using a number of techniques known to those of skill in the art. For example, the siRNA molecule can be chemically synthesized or recombinantly produced using methods known in the art, such as using appropriately protected ribonucleoside phosphoramidites and a conventional DNA/RNA synthesizer (see, *e.g.,* Elbashir, S.M. et al. (2001) Nature 411:494-498; Elbashir, S.M., W. Lendeckel and T. Tuschl (2001) Genes & Development 15:188-200; Harborth, J. et al. (2001) J. Cell Science 114:4557-4565; Masters, J.R. et al. (2001) Proc. Natl. Acad. Sci., USA 98:8012-8017; and Tuschl, T. et al. (1999) Genes & Development 13:3191-3197). Alternatively, several commercial RNA synthesis suppliers are available including, but are not limited to, Proligo (Hamburg, Germany), Dharmacon Research (Lafayette, CO, USA), Pierce Chemical (part of Perbio Science, Rockford, IL , USA), Glen Research (Sterling, VA, USA), ChemGenes (Ashland, MA, USA), and Cruachem (Glasgow, UK). As such, siRNA molecules are not overly difficult to synthesize and are readily provided in a quality suitable for RNAi. In addition, dsRNAs can be expressed as stem loop structures encoded by plasmid vectors, retroviruses and lentiviruses (Paddison, P.J. et al. (2002) Genes Dev. 16:948-958; McManus, M.T. et al. (2002) RNA 8:842-850; Paul, C.P. et al. (2002) Nat. Biotechnol. 20:505-508; Miyagishi, M. et al. (2002) Nat. Biotechnol. 20:497-500; Sui, G. et al. (2002) Proc. Natl. Acad. Sci., USA 99:5515-5520; Brummelkamp, T. et al. (2002) Cancer Cell 2:243; Lee, N.S., et al. (2002) Nat. Biotechnol. 20:500-505; Yu, J.Y., et al. (2002) Proc. Natl. Acad. Sci., USA 99:6047-6052; Zeng, Y., et al. (2002) Mol. Cell 9:1327-1333; Rubinson, D.A., et al. (2003) Nat. Genet. 33:401-406; Stewart, S.A., et al. (2003) RNA 9:493-501). These vectors generally have a *polIII* promoter upstream of the dsRNA and can express sense and antisense RNA strands separately and/or as a hairpin structures. Within cells, Dicer processes the short hairpin RNA (shRNA) into effective siRNA.

The targeted region of the siRNA molecule can be selected from a given target gene sequence, e.g., a Lp-PLA₂ coding sequence, beginning from about 25 to 50 nucleotides, from about 50 to 75 nucleotides, or from about 75 to 100 nucleotides downstream of the start codon. Nucleotide sequences can contain 5' or 3' UTRs and regions nearby the start codon. One method of designing a siRNA molecule involves identifying the 23 nucleotide sequence motif AA(N19)TT (where N can be any nucleotide), and selecting hits with at least 25%, 30%, 35%, 40%, 45%, 50%, 55%, 60%, 65%, 70% or 75% G/C content. The "TT" portion of the sequence is optional. Alternatively, if no such sequence is found, the search can be extended using the motif NA(N21), where N can be any nucleotide. In this situation, the 3' end of the sense siRNA can be converted to TT to allow for the generation of a symmetric duplex with respect to the sequence composition of the sense and antisense 3' overhangs. The antisense siRNA molecule can then be synthesized as the complement to nucleotide positions 1 to 21 of the 23 nucleotide sequence motif. The use of symmetric 3' TT overhangs can be advantageous to ensure that the small interfering ribonucleoprotein particles (siRNPs) are formed with approximately equal ratios of sense and antisense target RNA-cleaving siRNPs (Elbashir *et al.* (2001) *supra* and Elbashir *et al.* 2001 *supra*). Analysis of sequence databases, including but are not limited to the NCBI, BLAST, Derwent and GenSeq as well as commercially available oligosynthesis software such as Oligoengine^{®}, can also be used to select siRNA sequences against EST libraries to ensure that only one gene is targeted.

Delivery of RNA Interfering Agents: Methods of delivering RNA interfering agents, *e.g.,* an siRNA, or vectors containing an RNA interfering agent, to the target cells (*e.g.,* cells of the brain or other desired target cells, for cells in the central and peripheral nervous systems), can include, for example (i) injection of a composition containing the RNA interfering agent, *e.g.,* an siRNA, or (ii) directly contacting the cell, *e.g.,* a cell of the brain, with a composition comprising an RNA interfering agent, *e.g.,* an siRNA. RNA interfering agents, *e.g.,* an siRNA can be injected directly into any blood vessel, such as vein, artery, venule or arteriole, via, *e.g.,* hydrodynamic injection or catheterization. Lp-PLA2 agents such as Lp-PLA2 siRNA can delivered to specific organs, for example the liver, bone marrow or systemic administration.

Administration can be by a single injection or by two or more injections. The RNA interfering agent is delivered in a pharmaceutically acceptable carrier. One or more RNA interfering agents can be used simultaneously. The RNA interfering agents, *e.g.,* the siRNAs targeting Lp-PLA₂ mRNA, can be delivered singly, or in combination with other RNA interfering agents, *e.g.,* siRNAs, such as, for example siRNAs directed to other cellular genes. Lp-PLA₂ siRNAs can also be administered in combination with other pharmaceutical agents which are used to treat or prevent neurodegenerative diseases or disorders.

Specific cells can be targeted with RNA interference, limiting potential side effects of RNA interference caused by non-specific targeting of RNA interference. The method can use, for example, a complex or a fusion molecule comprising a cell targeting moiety and an RNA interference binding moiety that is used to deliver RNA interference effectively into cells. For example, an antibody-protamine fusion protein when mixed with an siRNA, binds siRNA and selectively delivers the siRNA into cells expressing an antigen recognized by the antibody, resulting in silencing of gene expression only in those cells that express the antigen. The siRNA or RNA interference-inducing molecule binding moiety is a protein or a nucleic acid binding domain or fragment of a protein, and the binding moiety is fused to a portion of the targeting moiety. The location of the targeting moiety can be either in the carboxyl-terminal or amino-terminal end of the construct or in the middle of the fusion protein.

A viral-mediated delivery mechanism can also be employed to deliver siRNAs to cells *in vitro* and *in vivo* as described in Xia, H. et al. (2002) Nat Biotechnol 20(10):1006). Plasmid- or viral-mediated delivery mechanisms of shRNA can also be employed to deliver shRNAs to cells *in vitro* and *in vivo* as described in Rubinson, D.A., et al. ((2003) Nat. Genet. 33:401-406) and Stewart, S.A., et al. ((2003) RNA 9:493-501).

RNA interfering agents, for *e.g.,* an siRNA, can also be introduced into cells via the vascular or extravascular circulation, the blood or lymph system, and the cerebrospinal fluid.

The dose of the particular RNA interfering agent will be in an amount necessary to effect RNA interference, *e.g.,* post translational gene silencing (PTGS), of the particular target gene, thereby leading to inhibition of target gene expression or inhibition of activity or level of the protein encoded by the target gene.

It is also known that RNAi molecules do not have to match perfectly to their target sequence. Preferably, however, the 5' and middle part of the antisense (guide) strand of the siRNA is perfectly complementary to the target nucleic acid sequence.

Accordingly, the RNAi molecules functioning as nucleic acid inhibitors of Lp-PLA₂ are for example, but are not limited to, unmodified and modified double stranded (ds) RNA molecules including short-temporal RNA (stRNA), small interfering RNA (siRNA), short-hairpin RNA (shRNA), microRNA (miRNA), double-stranded RNA (dsRNA), (see, e.g. Baulcombe, Science 297:2002-2003, 2002). The dsRNA molecules, e.g. siRNA, also can contain 3' overhangs, preferably 3'UU or 3'TT overhangs. The siRNA molecules may not include RNA molecules that comprise ssRNA greater than about 30-40 bases, about 40-50 bases, about 50 bases or more. The siRNA molecules may be double stranded for more than about 25%, more than about 50%, more than about 60%, more than about 70%, more than about 80%, more than about 90% of their length. A nucleic acid inhibitor of Lp-PLA₂ may be any agent which binds to and inhibits the expression of Lp-PLA₂ mRNA, where the expression of Lp-PLA₂ mRNA or a product of transcription of nucleic acid encoded by SEQ ID NO:1 or 2 is inhibited.

Agents inhibiting Lp-PLA₂ may be catalytic nucleic acid constructs, such as, for example ribozymes, which are capable of cleaving RNA transcripts and thereby preventing the production of wildtype protein. Ribozymes are targeted to and anneal with a particular sequence by virtue of two regions of sequence complementary to the target flanking the ribozyme catalytic site. After binding, the ribozyme cleaves the target in a site specific manner. The design and testing of ribozymes which specifically recognize and cleave sequences of the gene products described herein, for example for cleavage of Lp-PLA₂ or homologues or variants thereof can be achieved by techniques well known to those skilled in the art (for example Lleber and Strauss, (1995) Mol Cell Biol 15:540.551).

### Proteins and peptide inhibitors of Lp-PLA₂

Agents that inhibit Lp-PLA₂ may be proteins and/or peptide inhibitors or fragments of inhibitors of Lp-PLA₂, for example, but are not limited to mutated proteins; therapeutic proteins and recombinant proteins. Proteins and peptides inhibitors can also include for example mutated proteins, genetically modified proteins, peptides, synthetic peptides, recombinant proteins, chimeric proteins, antibodies, humanized proteins, humanized antibodies, chimeric antibodies, modified proteins and fragments thereof.

Agents that inhibit Lp-PLA₂ may be dominant negative variants of Lp-PLA₂, for example a non-functional variant of Lp-PLA₂.

### Antibodies

Inhibitors of genes and/or gene products useful in the methods described herein include, for example, antibodies, including monoclonal, chimeric humanized, and recombinant antibodies and antigen-binding fragments thereof. Neutralizing antibodies can be used as inhibitors of the Lp-PLA₂ enzyme. Antibodies are readily raised in animals such as rabbits or mice by immunization with the antigen. Immunized mice are particularly useful for providing sources of B cells for the manufacture of hybridomas, which in turn are cultured to produce large quantities of monoclonal antibodies.

The inhibitor to the gene products identified herein can be an antibody molecule or the epitope-binding moiety of an antibody molecule and the like. Antibodies provide high binding avidity and unique specificity to a wide range of target antigens and haptens. Monoclonal antibodies useful in the methods described herein include whole antibody and fragments thereof and are generated in accordance with conventional techniques, such as hybridoma synthesis, recombinant DNA techniques and protein synthesis.

Useful monoclonal antibodies and fragments can be derived from any species (including humans) or can be formed as chimeric proteins which employ sequences from more than one species. Human monoclonal antibodies or "humanized" murine antibody are also used in accordance with the described methods. For example, murine monoclonal antibody can be "humanized" by genetically recombining the nucleotide sequence encoding the murine Fv region (i.e., containing the antigen binding sites) or the complementarily determining regions thereof with the nucleotide sequence encoding a human constant domain region and an Fc region. Humanized targeting moieties are recognized to decrease the immunoreactivity of the antibody or polypeptide in the host recipient, permitting an increase in the half-life and a reduction the possibly of adverse immune reactions in a manner similar to that disclosed in European Patent Application No. 0,411,893 A2. The murine monoclonal antibodies should preferably be employed in humanized form. Antigen binding activity is determined by the sequences and conformation of the amino acids of the six complementarily determining regions (CDRs) that are located (three each) on the light and heavy chains of the variable portion (Fv) of the antibody. The 25-kDa single-chain Fv (scFv) molecule, composed of a variable region (VL) of the light chain and a variable region (VH) of the heavy chain joined via a short peptide spacer sequence, is the smallest antibody fragment developed to date. Techniques have been developed to display scFv molecules on the surface of filamentous phage that contain the gene for the scFv. scFv molecules with a broad range of antigenic-specificities can be present in a single large pool of scFv-phage library. Some examples of high affinity monoclonal antibodies and chimeric derivatives thereof are described in the European Patent Application EP 186,833; PCT Patent Application WO 92/16553; and US Patent No. 6,090,923.

Chimeric antibodies are immunoglobulin molecules characterized by two or more segments or portions derived from different animal species. Generally, the variable region of the chimeric antibody is derived from a non-human mammalian antibody, such as murine monoclonal antibody, and the immunoglobulin constant region is derived from a human immunoglobulin molecule. Preferably, both regions and the combination have low immunogenicity as routinely determined.

One limitation of scFv molecules is their monovalent interaction with target antigen. One of the easiest methods of improving the binding of a scFv to its target antigen is to increase its functional affinity through the creation of a multimer. Association of identical scFv molecules to form diabodies, triabodies and tetrabodies can comprise a number of identical Fv modules. These reagents are therefore multivalent, but monospecific. The association of two different scFv molecules, each comprising a VH and VL domain derived from different parent Ig will form a fully functional bispecific diabody. A unique application of bispecific scFvs is to bind two sites simultaneously on the same target molecule via two (adjacent) surface epitopes. These reagents gain a significant avidity advantage over a single scFv or Fab fragments. A number of multivalent scFv-based structures has been engineered, including for example, miniantibodies, dimeric miniantibodies, minibodies, (scFv)₂, diabodies and triabodies. These molecules span a range of valence (two to four binding sites), size (50 to 120 kDa), flexibility and ease of production. Single chain Fv antibody fragments (scFvs) are predominantly monomeric when the VH and VL domains are joined by, polypeptide linkers of at least 12 residues. The monomer scFv is thermodynamically stable with linkers of 12 and 25 amino acids length under all conditions. The noncovalent diabody and triabody molecules are easy to engineer and are produced by shortening the peptide linker that connects the variable heavy and variable light chains of a single scFv molecule. The scFv dimers are joined by amphipathic helices that offer a high degree of flexibility and the miniantibody structure can be modified to create a dimeric bispecific (DiBi) miniantibody that contains two miniantibodies (four scFv molecules) connected via a double helix. Gene-fused or disulfide bonded scFv dimers provide an intermediate degree of flexibility and are generated by straightforward cloning techniques adding a C-terminal Gly4Cys sequence. scFv-CH3 minibodies are comprised of two scFv molecules joined to an IgG CH3 domain either directly (LD minibody) or via a very flexible hinge region (Flex minibody). With a molecular weight of approximately 80 kDa, these divalent constructs are capable of significant binding to antigens. The Flex minibody exhibits impressive tumor localization in mice. Bi- and tri-specific multimers can be formed by association of different scFv molecules. Increase in functional affinity can be reached when Fab or single chain Fv antibody fragments (scFv) fragments are complexed into dimers, trimers or larger aggregates. The most important advantage of multivalent scFvs over monovalent scFv and Fab fragments is the gain in functional binding affinity (avidity) to target antigens. High avidity requires that scFv multimers are capable of binding simultaneously to separate target antigens. The gain in functional affinity for scFv diabodies compared to scFv monomers is significant and is seen primarily in reduced off-rates, which result from multiple binding to two or more target antigens and to rebinding when one Fv dissociates. When such scFv molecules associate into multimers, they can be designed with either high avidity to a single target antigen or with multiple specificities to different target antigens. Multiple binding to antigens is dependent on correct alignment and orientation in the Fv modules. For full avidity in multivalent scFvs target, the antigen binding sites must point towards the same direction. If multiple binding is not sterically possible then apparent gains in functional affinity are likely to be due the effect of increased rebinding, which is dependent on diffusion rates and antigen concentration. Antibodies conjugated with moieties that improve their properties are also contemplated. For example, antibody conjugates with PEG that increases their half-life in vivo can be used. Immune libraries are prepared by subjecting the genes encoding variable antibody fragments from the B lymphocytes of naive or immunized animals or patients to PCR amplification. Combinations of oligonucleotides which are specific for immunoglobulin genes or for the immunoglobulin gene families are used. Immunoglobulin germ line genes can be used to prepare semisynthetic antibody repertoires, with the complementarity-determining region of the variable fragments being amplified by PCR using degenerate primers. These single-pot libraries have the advantage that antibody fragments against a large number of antigens can be isolated from one single library. The phage-display technique can be used to increase the affinity of antibody fragments, with new libraries being prepared from already existing antibody fragments by random, codon-based or site-directed mutagenesis, by shuffling the chains of individual domains with those of fragments from naive repertoires or by using bacterial mutator strains.

Alternatively, a SCID-hu mouse, for example the model developed by Genpharm, can be used to produce antibodies, or fragments thereof. A new type of high avidity binding molecule, termed peptabody, created by harnessing the effect of multivalent interaction is contemplated. A short peptide ligand was fused via a semirigid hinge region with the coiled-coil assembly domain of the cartilage oligomeric matrix protein, resulting in a pentameric multivalent binding molecule. Ligands and/or chimeric inhibitors can be targeted to tissue- or tumor-specific targets by using bispecific antibodies, for example produced by chemical linkage of an anti-ligand antibody (Ab) and an Ab directed toward a specific target. To avoid the limitations of chemical conjugates, molecular conjugates of antibodies can be used for production of recombinant bispecific single-chain Abs directing ligands and/or chimeric inhibitors at cell surface molecules. Alternatively, two or more active agents and or inhibitors attached to targeting moieties can be administered, wherein each conjugate includes a targeting moiety, for example, a different antibody. Each antibody is reactive with a different target site epitope (associated with the same or a different target site antigen). The different antibodies with the agents attached accumulate additively at the desired target site. Antibody-based or non- antibody-based targeting moieties can be employed to deliver a ligand or the inhibitor to a target site. Preferably, a natural binding agent for an unregulated or disease associated antigen is used for this purpose.

### Bioassay for Identifying Lp-PLA₂ Inhibitors:

### Screen for inhibition of Lp-PLA₂ protein

The methods described herein relate to use of inhibitors of Lp-PLA₂ for the treatment of vascular dementia, for example Alzheimer's disease and/or treatment of disorders associated with permeability of BBB. Where necessary, agents that inhibit Lp-PLA₂ protein are assessed using a bioassay, as disclosed in U.S. Patent 5,981,252. One such assay is testing the effect of the agent on the recombinant Lp-PLA₂ protein. In one assay, for example, recombinant Lp-PLA₂ is purified to homogeneity from baculovirus infected Sf9 cells, using a zinc chelating column, blue sepharose affinity chromatography and an anion exchange column. Following purification and ultrafiltration, the enzyme can be stored at 6mg/ml at 4°C. Assay buffer comprises Tris-HCl (50 mM), NaCl (150 mM) and 1mM CHAPS, pH 7.4 at room temperature. Activity is measured by an increase in emission at 535 nm on hydrolysis of N-((6-(2,4-dinitrophenyl) amino)hexanoyl)-2-(4,4-difluoro-5,7-dimethyl-4-bora-3a,4a-diaza-s-indacene-3-pentanoyl)-1-hexadecanoyl-sn-glycero-3-phosphoethanolamine, triethylammonium salt (PED6, Molecular Probes catalogue reference D-23739) as substrate, using a fluorometric plate reader with 384 well microtitre plates. Reaction is initiated by the addition of enzyme (approx 400 pM final by weight) and substrate (5 µM final) to inhibitor in a total volume of 10 microlitres.

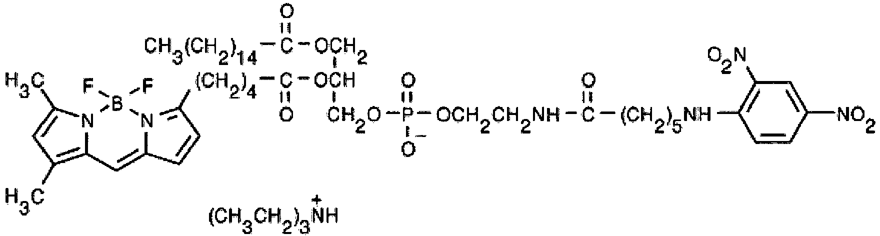

### (PED6)

The compounds as described herein, for example as described in the sections entitled of Examples of synthesis were tested and were found to have IC₅₀ values in the range 0.1 to 10 nM.

### Uses of agents that inhibit Lp-PLA₂ for the treatment of neurodegenerative diseases or disorders

### Disorders associated with BBB permeability:

Without wishing to be bound by theory, the BBB, a functional barrier in addition to an anatomic barrier, is of great importance for the maintenance of a constant environment for optimal CNS function. Most metabolic substrates (i.e. sugars and amino acids) are hydrophilic, and traverse the BBB only by specific carrier-mediated transport systems, which are expressed at both the luminal and abluminal sides of BBB endothelial cells. There are marked apical/basal differences in the distribution of carriers and enzymes, which distinguishes the specialized BBB endothelium from peripheral endothelium. Substantial progress has been made in the understanding of the pathophysiology and mechanisms involved in the attenuation of BBB permeability. Clinical implications of the "sick BBB" occurs in many diseases that affect the brain, with the BBB becoming disrupted, or modified, in such a way that there is a dramatic increase in vascular permeability. Without being bound by theory, several ways exist in which various molecules can pass the endothelium. These include intercellular routes, vesicular transport, or direct transcellular penetration through damaged endothelium.

An abnormal BBB or BBB dysfunction can be a cause or consequence of a particular disease process, for example neurodegenerative diseases and disorders. Diseases in which increased BBB permeability has been reported include neoplasia, ischemia, hypertension, dementia, epilepsy, infection, multiple sclerosis, and trauma. The effect of a disease on BBB function will secondarily affect the cerebral blood flow and vascular tone in the brain, which further influences transport across the BBB.

As described herein, inhibiting Lp-PLA₂ using the agents as described herein is useful for the treatment of disorders or diseases where BBB permeability occurs and/or BBB breakdown occurs. Inhibiting Lp-PLA₂ using the agents, for example, agents as described herein is useful for the treatment or prevention of disorders or diseases where maintaining an intact BBB is desired.

Agents inhibiting Lp-PLA₂ as described herein may be useful in preventing and treating neurodegenerative diseases and disorders. Examples of neurological diseases and neurodegenerative diseases and disorders include, but are not limited to Alzhiemer's disease (AD), Parkinson's disease (PD), Huntington's disease (HD), vascular dementia, aging and mild-cogntitive impairment.

Agents inhibiting Lp-PLA₂ as described herein may be useful in preventing and treating diseases where BBB permeability has been determined, or can be determined, to play a role, such as, for example but are not limited to: hyperosmolarity; acidic pH; burn encephalopathy; lead encephalopathy; autoimmune encephalitis; multiple sclerosis; post-ischemia reperfusion; acute hypertension; microwave irradiation; hepatic encephalopathy; seizures; tumors; development; hypervolemia; hypothermia; post-radiation; hyperbaric conditions; meningitis; lymphostatic encephalopathy; diabetes; Wernickes-Korsakoff syndrome; familial mental retardation and amyotrophic lateral sclerosis (ALS).

Subjects amenable to treatment by agents inhibiting Lp-PLA₂ as described herein include subjects at risk of disease but not showing symptoms (for example asymptomatic subjects), as well as subjects presently showing symptoms. In the case of Alzheimer's disease, virtually anyone is at risk of suffering from Alzheimer's disease if he or she lives long enough. Therefore, the present methods can be administered prophylactically to the general population without any assessment of the risk of the subject patient. The methods as described herein are especially useful for individuals who do have a known genetic risk of Alzheimer's disease. Such individuals include those having relatives who have experienced this disease, and those whose risk is determined by analysis of genetic or biochemical markers, as described herein.

### Vascular dementia diseases

Vascular dementia is comprised of a number of heterogeneous pathological conditions, which results from ischemic or hemorrhagic brain lesions as well as from lesions that develop during protracted hypoperfusion.

The subcortical ischemic form of vascular dementia is a common type of vascular cognitive impairment and dementia, and one of the major causes of cognitive decline in elderly people. Subcortical ischemic vascular dementia mainly results from small-vessel disease, which causes lacunes and extensive white matter lesions, and can be compared to large vessel dementia or cortical vascular dementia (Roman GC, Neurology. 1993;43:250-260, Roman GC Lancet Neurol. 2002;1:426-436). The ischemic lesions in subcortical ischemic vascular dementia particularly affect the frontal-subcortical circuits, an observation that explains the major cognitive and clinical neurological effects of vascular dementia (Ishii N, Neurologyl 986; 36: 340-45, Cummings JL, Arch Neurol 1993; 50:873-80). Subcortical ischemic vascular dementia is also caused by persistent hypertension (de Leeuw FE, Brain. 2002;125:765-772) and hypoperfusion due to congestive heart failure (Roman GC. Neurol Res. 2004;26:454-458), atrial fibrillation (de Leeuw FE, Neurology. 2000;54:1795- 1801), and obstructive sleep apnea (Kamba M, J. Neurol. Neurosurg. Psychiatry. 2001; 71; 334-339).

Ischemic white matter lesions, a common finding in elderly people, are the characteristic pathological changes in subcortical ischemic vascular dementia and cognitive impairment, and cognitive dysfunctions are related to lesion severity (Hachinski VC, Arch Neurol. 1987;4:21-23, Pantoni L, Alzheimer Dis. Assoc. Disord. 1999;13(suppl 3):S49-S54, de Groot JC, Neurology2001; 56:1539-1545). Cerebrovascular white matter lesions constitute the core pathology in several types of vascular dementia, such as Binswanger's disease, cerebral amyloid angiopathy, and cerebral autosomal dominant arteriopathy with subcortical infarcts and leukoencephalopathy (CADASIL). These cerebrovascular white matter lesions are caused by chronic cerebral hypoperfusion, which result from the severe stenosis of several arteries or arterioles mainly in deep white matter (Pantoni L, Stroke 1997;28:652-659, de Groot JC, Neurology2001; 56:1539-1545, Roman GC, Neurol. Res. 2004;26:454-458, Capizzano AA, Am J Neuroradiol 2000;21:621-63 0).

### Alzheimer's Disease

Alzheimer's disease (AD) is a progressive disease resulting in senile dementia. See generally Selkoe, TINS 16, 403-409 (1993); Hardy et al., WO 92/13069; Selkoe, J. Neuropathol. Exp. Neurol. 53, 438-447 (1994); Duff et al., Nature 373, 476-477 (1995); Games et al., Nature 373, 523 (1995). Broadly speaking the disease falls into two categories: late onset, which occurs in old age (65 + years) and early onset, which develops well before the senile period, i.e., between 35 and 60 years. In both types of disease, the pathology is the same but the β abnormalities tend to be more severe and widespread in cases beginning at an earlier age. The disease is characterized at the macroscopic level by significant brain shrinkage away from the cranial vault as seen in MRI images as a direct result of neuronal loss and by two types of macroscopic lesions in the brain, senile plaques and neurofibrillary tangles. Senile plaques are areas comprising disorganized neuronal processes up to 150 µm across and extracellular amyloid deposits, which are typically concentrated at the center and visible by microscopic analysis of sections of brain tissue. Neurofibrillary tangles are intracellular deposits of tau protein consisting of two filaments twisted about each other in pairs.

The principal constituent of the plaques is a peptide termed Aβ or β-amyloid peptide. Aβ peptide is an internal fragment of 39-43 amino acids of a precursor protein termed amyloid precursor protein (APP). Several mutations within the APP protein have been correlated with the presence of Alzheimer's disease. See, e.g., Goate et al., Nature 349, 704) (1991) (valine⁷¹⁷ to isoleucine); Chartier Harlan et al. Nature 353, 844 (1991)) (valine⁷¹⁷ to glycine); Murrell et al., Science 254, 97 (1991) (valine⁷¹⁷ to phenylalanine); Mullan et al., glycine); Murrell et al., Science 254, 97 (1991) (valine⁷¹⁷ to phenylalanine); Mullan et al., Nature Genet. 1, 345 (1992) (a double mutation changing lysine⁵⁹⁵-methionine⁵⁹⁶ to asparagine⁵⁹⁵-leucine⁵⁹⁶). Such mutations are thought to cause Alzheimer's disease by increased or altered processing of APP to Aβ, particularly processing of APP to increased amounts of the long form of Aβ (i.e., Aβ 1-42 and Aβ 1-43). Mutations in other genes, such as the presenilin genes, PS1 and PS2, are thought indirectly to affect processing of APP to generate increased amounts of long form Aβ (see Hardy, TINS 20, 154 (1997)). These observations indicate that Aβ, and particularly its long form, is a causative element in Alzheimer's disease.

Aβ, also known as β-amyloid peptide, or A4 peptide (see U.S. Pat. No. 4,666,829; Glenner & Wong, Biochem. Biophys. Res. Commun. 120, 1131 (1984)), is a peptide of 39-43 amino acids, is the principal component of characteristic plaques of Alzheimer's disease. Aβ is generated by processing of a larger protein APP by two enzymes, termed β and γ secretases (see Hardy, TINS 20, 154 (1997)). Known mutations in APP associated with Alzheimer's disease occur proximate to the site of β or γ-secretase, or within Aβ. For example, position 717 is proximate to the site of γ -secretase cleavage of APP in its processing to Aβ, and positions 670/671 are proximate to the site of β-secretase cleavage. It is believed that the mutations cause AD disease by interacting with the cleavage reactions by which Aβ is formed so as to increase the amount of the 42/43 amino acid form of Aβ generated.

Aβ has the unusual property that it can fix and activate both classical and alternate complement cascades. In particular, it binds to Clq and ultimately to C3bi. This association facilitates binding to macrophages leading to activation of B cells. In addition, C3bi breaks down further and then binds to CR2 on B cells in a T cell dependent manner leading to a 10,000 increase in activation of these cells. This mechanism causes Aβ to generate an immune response in excess of that of other antigens.

Most therapeutic strategies for Alzheimer's disease are aimed at reducing or eliminating the deposition of Aβ42 in the brain, typically via reduction in the generation of Aβ42 from APP and/or some means of lowering existing Aβ42 levels from sources that directly contribute to the deposition of this peptide in the brain (De Felice and Ferreira, 2002). A partial list of aging-associated causative factors in the development of sporadic Alzheimer's disease includes a shift in the balance between Aβ peptide production and its clearance from neurons that favors intracellular accumulation, increased secretion of Aβ peptides by neurons into the surrounding extracellular space, increased levels of oxidative damage to these cells, and global brain hypoperfusion and the associated compensatory metabolic shifts in affected neurons (Cohen et al., 1988; Higgins et al., 1990; Kalaria, 2000; Nalivaevaa et al., 2004; Teller et al., 1996; Wen et al., 2004).

The Aβ42 that deposits within neurons and plaques could also originate from outside of the neurons (exogenous Aβ42) during Alzheimer's disease pathogenesis. Levels of soluble Aβ peptides in the blood are known to be much higher than in the interstitial space and CSF in the brains of healthy individuals (Seubert et al., 1992) with blood as a source of exogenous Aβ peptides that eventually deposit in the Alzheimer's disease brain (Zlokovic et al., 1993). However, except for trace amounts of Aβ that are actively transported across endothelial cells, it is well-known that access of blood-borne Aβ peptides to brain tissue in normal healthy individuals is effectively blocked by the integrity of the blood-brain barrier (BBB) (Kandimalla et al., 2005; Poduslo et al., 1999). The BBB is a complex structure composed of cerebral endothelial cells resting on a basal lamina that is further supported by the foot processes of local astrocytes (Gloor et al., 2001; Risau et al., 1998). It closely regulates the passage of blood components into the brain tissue and is highly impermeable to nearly all proteins and other macromolecules while, at the same time, allowing the selective entry of essential molecules (Mayhan, 2001). Much evidence has revealed that aging is associated with degenerative changes to blood vessels that may compromise the integrity of the BBB. For example, a number of relatively common neurodegenerative diseases in the elderly, including vascular dementia, and AD originate, at least in part, from cerebrovascular pathologies that develop within the microvasculature of the brain (Breteler, 2000; Buee et al., 1997; de la Torre, 1997; Esiri et al., 1999; Kalaria et al., 1996). A link between the neurovasculature and neurodegenerative disease is the well-known fact that Alzheimer pathology, including amyloid plaques and neurofibrillary tangles, develops subsequently within the vicinity of stroke lesions (Jellinger, 2002; Kalaria, 1996; Kalaria, 2002; Natte et al., 1998).

Genetic markers of risk toward Alzheimer's disease include mutations in the APP gene, particularly mutations at position 717 and positions 670 and 671 referred to as the Hardy and Swedish mutations respectively (see Hardy, TINS, supra). Other markers of risk are mutations in the presenilin genes, PS1 and PS2, and ApoE4, family history of Alzheimer's disease, hypercholesterolemia or atherosclerosis. Subjects presently suffering from Alzheimer's disease can be recognized from characteristic dementia, as well as the presence of risk factors described above. In addition, a number of diagnostic tests are available for identifying subjects who have Alzheimer's disease. These include measurement of CSF tau and Aβ42 levels. Elevated tau and increased Aβ42 levels signify the presence of Alzheimer's disease. Individuals suffering from Alzheimer's disease can also be diagnosed by MMSE or ADRDA criteria. The tissue sample for analysis is typically blood, plasma, serum, mucus or cerebral spinal fluid from the patient. The sample is analyzed for indicia of an immune response to any forms of Aβ peptide, typically Aβ 42. The immune response can be determined from the presence of, e.g., antibodies or T-cells that specifically bind to Aβ peptide. ELISA methods of detecting antibodies specific to Aβ are described in the Examples section.

In asymptomatic patients, treatment can begin at any age (e.g., 10, 20, 30). Usually, however, it is not necessary to begin treatment until a patient reaches 40, 50, 60 or 70. Treatment typically entails multiple dosages over a period of time. Treatment can be monitored by assaying presence of Aβ peptide in the CSF or the permeability of the BBB over time. If the Aβ peptide is still present in the CSF or the BBB remains permeable or defective, additional treatment with agents inhibiting Lp-PLA₂ as described herein are recommended, and/or treatment of additional therapies for Alzheimer's disease. In the case of potential Down's syndrome patients, treatment can begin antenatally by administering therapeutic agent to the mother or shortly after birth.

Agents that inhibit Lp-PLA₂ as described herein may also be useful in the treatment of other neurodegenerative disorders or cognitive impairment disorders in general: for example, dementia, depression, confusion, Creutzfeldt-Jakob or mad cow disease, Huntington's disease, loss of motor coordination, multiple sclerosis, Parkinson's disease, Pick disease and other brain storage disorders (e.g., amyloidosis, gangliosidosis, lipid storage disorders, mucopolysaccharidosis), syncope, and vascular dementia. Thus, treatment can be directed to a subject who is affected with unsymptomatic by the neurodegenerative disease; it can improve cognitive function. The efficacy of treatment can be determined by monitoring cerebral blood flow (CBF) and/or blood-brain barrier (BBB) function. For example, measuring the presence of Tau or Aβ in the CSF. Markers for BBB function can be used, for example, as disclosed in U.S. Patent 6,884,591 BBB function can be monitored using imaging techniques known by persons of ordinary skill in the art, for example using MRI machines with high contrast capability. Vascular injection of specific MRI contrasting agents immediately prior to a MRI is performed, where the contrasting agent remains within the blood vessels in a subject with a normal BBB, whereas the contrasting agent penetrates into the brain tissue and appears as a "cloud" on the MRI in individuals with a permeable BBB. Using this method, quantitative measurements of the location and the extent of the BBB breakdown can be determined using MRI software. Accordingly, efficacy of treatment with an inhibitor of Lp-PLA₂ can be determined by detecting a measurable reduction in the "cloud" and/or reduction to the extent of BBB breakdown after administration of the Lp-PLA₂ inhibitors as compared to prior to administration as described herein.

Some methods entail determining a baseline value of, for example the level of beta amyloid in the CSF of a subject before administering a dosage of agent, and comparing this with a value for beta amyloid in the CSF after treatment. A decrease, for example a 10% decrease in the level of beta amyloid in the CSF indicates a positive treatment outcome (i.e., that administration of the agent has achieved or augmented a decrease in beta amyloid in the CSF). If the value for level of beta amyloid in the CSF does not change significantly, or increases, a negative treatment outcome is indicated. In general, subjects undergoing an initial course of treatment with an agent are expected to show a decrease in beta amyloid in the CSF with successive dosages of an agent as described herein.

In other methods to determine efficacy of treatment, a control value (i.e., a mean and standard deviation) of beta amyloid is determined for a control population. Typically the individuals in the control population have not received prior treatment and do not suffer from Alzheimer's disease. Measured values of beta amyloid in the CSF in a subject after administering an inhibitor agent of Lp-PLA₂ as described herein are then compared with the control value. A decrease in the beta amyloid in the CSF of the subject relative to the control value (i.e. a decrease of at least 10% of beta amyloid in a subject) signals a positive treatment outcome. A lack of significant decrease signals a negative treatment outcome.

In other methods, a control value of, for example beta amyloid in the CSF is determined from a control population of subjects who have undergone treatment with a therapeutic agent that is effective at reducing beta amyloid in the CSF. Measured values of CSF beta amyloid in the subject are compared with the control value.

In other methods, a subject who is not presently receiving treatment by agents that inhibit Lp-PLA₂ as described herein, but has undergone a previous course of treatment is monitored for beta amyloid in the CSF to determine whether a resumption of treatment is required. The measured value of CSF beta amyloid in the test subject can be compared with a level of the CSF beta amyloid in the previously achieved in the subject after a previous course of treatment. A significant decrease in CSF beta amyloid relative to the previous measurement (i.e., a decrease of at least 10%) is an indication that treatment can be resumed. Alternatively, the level of beta amyloid in the CSF in the subject can be compared with a control level of CSF beta amyloid determined in a population of subjects after undergoing a course of treatment. Alternatively, the level of CSF beta amyloid in a subject can be compared with a control value in populations of prophylactically treated subjects who remain free of symptoms of disease, in particular free of symptoms of BBB permeability, or populations of therapeutically treated subjects who show amelioration of disease symptoms.

### Methods to identify subjects for risk of or having Alzheimer's disease.

Subjects amenable to treatment using the methods as described herein include subjects at risk of a neurodegenerative disease, for example Alzheimer's Disease but not showing symptoms, as well as subjects showing symptoms of the neurodegenerative disease, for example subjects with symptoms of Alzheimer's Disease.

Subjects can be screened for their likelihood of having or developing Alzheimer's Disease based on a number of biochemical and genetic markers.

One can also diagnose a subject with increased risk of developing Alzheimer's Disease using genetic markers for Alzheimer's Disease. Genetic abnormality in a few families has been traced to chromosome 21 (St. George-Hyslop et al., Science 235:885-890, 1987). One genetic marker is, for example mutations in the APP gene, particularly mutations at position 717 and positions 670 and 671 referred to as the Hardy and Swedish mutations respectively (see Hardy, TINS, supra). Other markers of risk are mutations in the presenilin genes, PS1 and PS2, and ApoE4, family history of Alzheimer's Disease, hypercholesterolemia or atherosclerosis. Subjects with APP, PS1 or PS2 mutations are highly likely to develop Alzheimer's disease. ApoE is a susceptibility gene, and subjects with the e4 isoform of ApoE (ApoE4 isoform) have an increased risk of developing Alzheimer's disease. Test for subjects with ApoE4 isoform are disclosed in U.S. Patent 6,027,896. Other genetic links have been associated with increased risk of Alzheimer's disease, for example variances in the neuronal sortilin-related receptor SORL1 may have increased likelihood of developing late-onset Alzheimer's disease (Rogaeva at al, Nat Genet. 2007 Feb;39(2):168-77). Other potential Alzheimer disease susceptibility genes, include, for example ACE, CHRNB2, CST3, ESR1, GAPDHS, IDE, MTHFR, NCSTN, PRNP, PSEN1, TF, TFAM and TNF and be used to identify subjects with increased risk of developing Alzheimer's disease (Bertram et al, Nat Genet. 2007 Jan;39(1):17-23), as well as variances in the alpha-T catenin (VR22) gene (Bertram et al, J Med Genet. 2007 Jan;44(1):e63) and Insulin-degrading enzyme (IDE) and Kim et al, J Biol Chem. 2007;282:7825-32).

One can also diagnose a subject with increased risk of developing Alzheimer's disease on the basis of a simple eye test, where the presence of cataracts and/or Abeta in the lens identifies a subject with increased risk of developing Alzheimer's Disease. Methods to detect Alzheimer's disease include using a quasi-elastic light scattering device (Goldstein et al., Lancet. 2003;12;361:1258-65) from Neuroptix, using Quasi-Elastic Light Scattering (QLS) and Fluorescent Ligand Scanning (FLS) and a Neuroptix™ QEL scanning device, to enable non-invasive quantitative measurements of amyloid aggregates in the eye, to examine and measure deposits in specific areas of the lens as an early diagnostic for Alzheimer's disease. Method to diagnose a subject at risk of developing Alzheimers disease using such a method of non-invasive eye test are disclosed in U.S. Patent 7,107,092.

Individuals presently suffering from Alzheimer's disease can be recognized from characteristic dementia, as well as the presence of risk factors described above. In addition, a number of diagnostic tests are available for identifying individuals who have AD. These include measurement of CSF tau and Ax3b242 levels. Elevated tau and decreased Ax3b242 levels signify the presence of Alzheimer's Disease.

There are two alternative "criteria" which are utilized to clinically diagnose Alzheimer's Disease: the DSM-IIIR criteria and the NINCDS-ADRDA criteria (which is an acronym for National Institute of Neurological and Communicative Disorders and Stroke (NINCDS) and the Alzheimer's Disease and Related Disorders Association (ADRDA); see McKhann et al., Neurology 34:939-944, 1984). Briefly, the criteria for diagnosis of Alzheimer's Disease under DSM-IIIR include (1) dementia, (2) insidious onset with a generally progressive deteriorating course, and (3) exclusion of all other specific causes of dementia by history, physical examination, and laboratory tests. Within the context of the DSM-IIIR criteria, dementia is understood to involve "a multifaceted loss of intellectual abilities, such as memory, judgement, abstract thought, and other higher cortical functions, and changes in personality and behaviour." (DSM-1IR, 1987).

In contrast, the NINCDS-ADRDA criteria sets forth three categories of Alzheimer's Disease, including "probable," "possible," and "definite" Alzheimer's Disease. Clinical diagnosis of "possible" Alzheimer's Disease may be made on the basis of a dementia syndrome, in the absence of other neurologic, psychiatric or systemic disorders sufficient to cause dementia. Criteria for the clinical diagnosis of "probable" Alzheimer's Disease include (a) dementia established by clinical examination and documented by a test such as the Mini-Mental test (Foldstein et al., J. Psych. Res. 12:189-198, 1975); (b) deficits in two or more areas of cognition; (c) progressive worsening of memory and other cognitive functions; (d) no disturbance of consciousness; (e) onset between ages 40 and 90, most often after age 65; and (f) absence of systemic orders or other brain diseases that could account for the dementia. The criteria for definite diagnosis of Alzheimer's Disease include histopathologic evidence obtained from a biopsy, or after autopsy. Since confirmation of definite Alzheimer's Disease requires histological examination from a brain biopsy specimen (which is often difficult to obtain), it is rarely used for early diagnosis of Alzheimer's Disease.

One can also use neuropathologic diagnosis of Alzheimer's Disease, where the numbers of plaques and tangles in the neurocortex (frontal, temporal, and parietal lobes), hippocampus and amygdala are analyzed (Khachaturian, Arch. Neurol. 42:1097-1105; Esiri, "Anatomical Criteria for the Biopsy diagnosis of Alzheimer's Disease," Alzheimer's Disease, Current Research in Early Diagnosis, Becker and Giacobini (eds.), pp. 239-252, 1990).

One can also use quantitative electroencephalographic analysis (EEG) to diagnose Alzheimer's Disease. This method employs Fourier analysis of the beta, alpha, theta, and delta bands (Riekkinen et al., "EEG in the Diagnosis of Early Alzheimer's Disease," Alzheimer's Disease, Current Research in Early Diagnosis, Becker and Giacobini (eds.), pp. 159-167, 1990) for diagnosis of Alzheimer's Disease.

One can also diagnose Alzheimer's Disease by quantifying the degree of neural atrophy, since such atrophy is generally accepted as a consequence of Alzheimer's Disease. Examples of these methods include computed tomographic scanning (CT), and magnetic resonance imaging (MRI) (Leedom and Miller, "CT, MRI, and NMR Spectroscopy in Alzheimer's Disease," Alzheimer's Disease, Current Research in Early Diagnosis, Becker and Giacobini (eds.), pp. 297-313,1990).

One can also diagnose Alzheimer's Disease by assessing decreased cerebral blood flow or metabolism in the posterior temporoparietal cerebral cortex by measuring decreased blood flow or metabolism by positron emission tomography (PET) (Parks and Becker, "Positron Emission Tomography and Neuropsychological Studies in Dementia," Alzheimer's Disease's, Current Research in Early Diagnosis, Becker and Giacobini (eds.), pp. 315-327, 1990), single photon emission computed tomography (SPECT) (Mena et al., "SPECT Studies in Alzheimer's Type Dementia Patients," Alzheimer's Disease, Current Research in Early Diagnosis, Becker and Giacobini (eds.), pp. 339-355, 1990), and xenon inhalation methods (Jagust et al., Neurology 38:909-912; Prohovnik et al., Neurology 38:931-937; and Waldemar et al., Senile Dementias: II International Symposium, pp. 399407, 1988).

One can also immunologically diagnose Alzheimer's disease (Wolozin, "Immunochemical Approaches to the Diagnosis of Alzheimer's Disease," Alzheimer's Disease, Current Research in Early Diagnosis, Becker and Giacobini (eds.), pp. 217-235, 1990). Wolozin and coworkers (Wolozin et al., Science 232:648-650, 1986) produced a monoclonal antibody "Alz50," that reacts with a 68-kDa protein "A68," which is expressed in the plaques and neuron tangles of patients with Alzheimer's disease. Using the antibody Alz50 and Western blot analysis, A68 was detected in the cerebral spinal fluid (CSF) of some Alzheimer's patients and not in the CSF of normal elderly patients (Wolozin and Davies, Ann. Neurol. 22:521-526, 1987).

One can also diagnose Alzheimer's disease using neurochemical markers of Alzheimer's disease. Neurochemical markers which have been associated with Alzheimer's Disease include reduced levels of acetylcholinesterase (Giacobini and Sugaya, "Markers of Cholinergic Dysfunction in Alzheimer's Disease," Alzheimer's Disease, Current Research in Early Diagnosis, Becker and Giacobini (eds.), pp. 137-156, 1990), reduced somatostatin (Tamminga et al., Neurology 37:161-165, 1987), a negative relation between serotonin and 5-hydroxyindoleacetic acid (Volicer et al., Arch Neurol. 42:127-129, 1985), greater probenecid-induced rise in homovanyllic acid (Gibson et al., Arch. Neurol. 42:489-492, 1985) and reduced neuron-specific enolase (Cutler et al., Arch. Neurol. 43:153-154, 1986).

### Methods to identify subjects for risk of or having dementia and/or methods for memory assessment.

Current standard practice can be used to diagnose the various types of dementia and, once diagnosed, to monitor the progression of the disease over an extended period of time. One such method includes at least one of the following; (i) a memory assessment, (ii) an extensive neuropsychological exam, (iii) an examination by a geriatric neurologist and (iv) MRI imaging of the brain. Disease progression is documented by changes in these parameters over time. Changes in the parameters of at least one of these assessments can be used to assess the efficacy of Lp-PLA₂ inhibitor in the subject over time.

A memory assessment can be used, such as the program of the UMDNJ New Jersey Institute for Successful Aging. Adult patients with complaint of short term memory and / or cognitive decline are seen in the Memory Assessment Program, comprising evaluation by Geriatric Neurology, Neuropsychology and Social services. Patients can be both self-referred or directed from community clinicians and physicians on the suspicion of a possible or probable memory disorder or dementia. In such a memory assessment, at the time of the initial evaluation, all of the evaluations such as (i) memory assessment (ii) an extensive neuropsychological exam, (iii) an examination by a geriatric neurologist and (iv) MRI imaging of the brain are performed the same day. The neuropsychology assessment captures a broad inventory of cognitive function which aids in determining the array and severity of deficits. These include assessments of Judgement, Insight, Behavior, Orientation, Executive Control, General Intellectual Functioning, Visualspatial Function, Memory and New Learning Ability. Depression, if present, is identified. The neurological evaluation captures the history of cognitive alteration as well as the general medical history, and typically a complete neurological exam is performed. The neurological examination can also comprise laboratory studies to exclude reversible causes of dementia including Vitamin B12, Folate, Basic Metabolic Profile, CBC, TSH, ALT, AST, C-reactive protein, serum homocysteine, and RPR. The brain imaging provides a structural brain image, such as brain MRI, although one can use other brain imaging methods known by persons of ordinary skill in the art. The data matrix of history, neuropsychologic tests, neurologic examination, laboratory studies and neuroimaging is used to formulate the diagnosis.

Dementia diagnosis can be based the guidelines of the American Academy of Neurology Practice Parameter published in 2001. Diagnosis of Alzheimer's disease can be based on the NINDS-ADRDA criteria. Diagnosis of vascular dementia can be based on State of California AD Diagnostic and Treatment Centers criteria. One can communicate the diagnostic conclusion to the patient and family at a subsequent meeting. If the diagnostic conclusion indicates that the patient or subject has or is likely to have dementia and/or memory loss, a clinician can advise treatment administration with an effective amount of an inhibitor agent of Lp-PLA₂ as described herein. Often presence of a social worker at the subsequent meeting can also aid and direct patient and their family with current and future needs.

Other methods to diagnose a patient at risk of or having a neurodegenerative disease or disorder, such as vascular dementia or Alzheimer's Disease includes measurement of Lp-PLA₂ activity and/or expression using the methods as described herein, for example using the PLAC test commercially available from diaDexus.

### Methods to identify subjects for risk of, or having BBB breakdown or BBB permeability.

Direct detection of BBB breakdown can be assessed using MRI and injection of contrasting agent. Improvements in the resolution of MRIs and in the use of special contrasting agents can be used to detect BBB permeability. In one method, subjects are administered a contrasting agent immediately prior to brain imaging, such as MRI imaging. In cases of intact BBB, the contrasting agents are confined to brain blood vessels whereas, in subjects with a disrupted BBB, the contrasting agent is "sprayed out" into the brain tissue, which can be visualized. Thus, the brain locations, the size of BBB breakdown and extent of BBB compromise, such as extent of vascular leak in subjects can be directly and quantitatively assessed as measurable parameters of BBB permeability. Furthermore, such methods can be used to assess any improvements in these parameters resulting from treatment of the subject with an agent that inhibits Lp-PLA₂. Direct visualization of BBB breakdown and its associated vascular leak into the brain is useful in the methods as described herein for monitoring the beneficial effects of treating a subject with BBB permeability with Lp-PLA₂ inhibitors. An improvement in at least one measurable parameter of BBB permeability, such as location, size of BBB breakdown and extent of vascular leak in subjects administered a Lp-PLA₂ inhibitor indicates a positive outcome from administration of an inhibitor of Lp-PLA₂. Parameters of BBB permeability can be monitored by direct visualization and quantified by MRI-associated image analysis and are useful in the methods as described herein.

### Assessment of inhibitors of Lp-PLA₂ in models of vascular dementia and Alzheimer's disease.

Agents inhibiting Lp-PLA₂ can be assessed in animal models for effect on alleviating BBB permeability. For example, one can use the porcine model of hyperglycemia and hypercholesterolemia (HG/HC) as disclosed herein, where the BBB permeability is impaired, for example the BBB is permeable, in which the BBB permeability can be assessed in the present and absence of inhibitors for Lp-PLA₂ by methods commonly known by persons in the art. Markers for BBB function can be used, for example, as disclosed in U.S. Patent 6,884,591.

Agents inhibiting Lp-PLA₂ can be assessed in animal models for vascular dementia, permitting analysis of the effects of Lp-PLA₂ inhibitory agents on vascular dementia development and treatment, as well as assessment of drug dosages on the development, prognosis and recovery from vascular dementia.

Animal models of vascular dementia includes, for example occlusion of carotid arteries in rats. See, e.g., Sarti et al., Persistent impairment of gait performances and working memory after bilateral common carotid artery occlusion in the adult Wistar rat, BEHAVIOURAL BRAIN RESEARCH 136: 13-20 (2002). Thus, cerebrovascular white matter lesions can be experimentally induced in the rat brain as a result of chronic cerebral hypoperfusion. This model is created by permanent occlusion of both common carotid arteries. For example, Wistar rats can be anesthetized, the bilateral common carotid arteries are exposed through a midline cervical incision and the common carotid arteries are double-ligated with silk sutures bilaterally. The cerebral blood flow (CBF) then initially decreases by about 30 to 50% of the control after ligation. The CBF values later range from 40 to 80 % of control after about 1 week to about 1 month. Blood-brain barrier disruptions have also been observed as well as increased matrix metalloproteinase activity in white matter lesions. These changes appear very similar to those in human cerebrovascular white matter lesions. Moreover, these results suggest that inflammatory and immunologic reactions play a role in the pathogenesis of the white matter changes.

Such physiological changes are correlated with learning and memory problems in the occluded carotid artery rat model. Thus, the gait performance of rats with occluded arteries declines over time in comparison with baseline, for example, at and 90 days, rats with bilateral common carotid artery occlusion have decreased performances on object recognition and Y maze spontaneous alternation test in comparison with sham-operated rats. Thus, this rat model of experimental chronic cerebral hypoperfusion by permanent occlusion of the bilateral common carotid arteries is useful as a model for significant learning impairments along with rarefaction of the white matter. This model is a useful tool to assess the effectiveness of agents that inhibit Lp-PLA₂ on the pathophysiology of chronic cerebral hypoperfusion, and to provide data for determining optimal dosages and dosage regimens for preventing the cognitive impairment and white matter lesions in patients with cerebrovascular disease.

The effectiveness of agents that inhibit Lp-PLA₂ for treating or preventing vascular dementia can therefore be determined by observing the gait performance, memory, learning abilities and the incidence and severity of white matter lesions in rats with carotid artery occlusions. Similarly, the dosage and administration schedule of Lp-PLA₂ inhibitors can be adjusted pursuant to the memory and learning abilities of human patients being treated for vascular dementia.

The optimum dosage of agents that inhibit Lp-PLA₂ may be one that reduces activity and/or expression of Lp-PLA₂, for example, reduced expression of nucleic acid, for example mRNA encoded by Lp-PLA₂ gene or reduced expression or activity of Lp-PLA₂ protein. Alternatively, the optimum dosage of agents that inhibit Lp-PLA₂ may be one that generates the maximum protective effect in preventing a neurodegenerative disease or disorder, or reducing a symptom of a neurodegenerative disease or disorder.

The optimum dosage of agents that inhibit Lp-PLA₂ may be one generating the maximum beneficial effect on damaged tissue caused by arterial occlusion. An effective dosage causes at least a statistically or clinically significant attenuation of at least one marker, symptom, or histological evidence characteristic of vascular dementia. Markers, symptoms and histological evidence characteristic of vascular dementia include memory loss, confusion, disturbances in axonal transport, demyelination, induction of metalloproteinases (MMPs), activation of glial cells, infiltration of lymphocytes, edema and immunological reactions that lead to tissue damage and further vascular injury. Stabilization of symptoms or diminution of tissue damage, under conditions wherein control patients or animals experience a worsening of symptoms or tissue damage, is one indicator of efficacy of a suppressive treatment.

Other diseases neurodegenerative diseases with BBB permeability: Agents inhibiting Lp-PLA₂ using the agents as described herein are useful in preventing and treating neurodegenerative diseases and disorders. Additional examples of neurological diseases and neurodegenerative include, for example, polyglutamine repeat disorders such as Huntington's disease, Spinocerebellar ataxias (e.g., types 1, 2, 3, 6, 7 and 17), Machado- Joseph disease, Spinal and Bulbar muscular atrophy (SBMA or Kennedy's disease), Dentatorubral Pallidoluysian Atrophy (DRPLA) and other neurological conditions arising from polyglutamine expansions, or disease arising from non-coding DNA repeat expansions such as Fragile X syndrome, Fragile XE mental retardation, Friedreich ataxia, myotonic dystrophy, Spinocerebellar ataxias (types 8, 10 and 12) or other neurodegenerative diseases such as spinal muscular atrophy (Werdnig-Hoffinan disease, Kugelberg-Welander disease), Pick's disease, and spongiform encephalopathies.

Additional neurodegenerative diseases for which agents inhibiting Lp-PLA₂ using the agents as described herein may be useful include, for example, age-related memory impairment, agyrophilic grain dementia, Parkinsonism-dementia complex of Guam, autoimmune conditions (eg Guillain- Barre syndrome, Lupus), Biswanger's disease, brain and spinal tumors (including neurofibromatosis), cerebral amyloid angiopathies (Journal of Alzheimer's Disease vol 3, 65- 73 (2001)), cerebral palsy, chronic fatigue syndrome, corticobasal degeneration, conditions due to developmental dysfunction of the CNS parenchyma, conditions due to developmental dysfunction of the cerebrovasculature, dementia - multi infarct, dementia - subcortical, dementia with Lewy bodies, dementia of human immunodeficiency virus (HIV), dementia lacking distinct histology, Dementia Pugilistica, difffies neurofibrillary tangles with calcification, diseases of the eye, ear and vestibular systems involving neurodegeneration (including macular degeneration and glaucoma), Down's syndrome, dyskinesias (Paroxysmal), dystonias, essential tremor, Fahr's syndrome, fronto-temporal dementia and Parkinsonism linked to chromosome 17 (FTDP-17), frontotemporal lobar degeneration, frontal lobe dementia, hepatic encephalopathy, hereditary spastic paraplegia, hydrocephalus, pseudotumor cerebri and other conditions involving CSF dysffinction, Gaucher's disease, Hallervorden- Spatz disease, Korsakoffs syndrome, mild cognitive impairment, monomeric amyotrophy, motor neuron diseases, multiple system atrophy, multiple sclerosis and other demyelinating conditions (eg leukodystrophies), myalgic encephalomyelitis, myoclonus, neurodegeneration induced by chemicals, drugs and toxins, neurological manifestations of AIDS including AIDS dementia, neurological / cognitive manifestations and consequences of bacterial and/or virus infections, including but not restricted to enteroviruses, Niemann-Pick disease, non-Guamanian motor neuron disease with neurofibrillary tangles, non-ketotic hyperglycinemia, olivo-ponto cerebellar atrophy, oculopharyugeal muscular dystrophy, neurological manifestations of Polio myelitis including non-paralytic polio and post-polio- syndrome, primary lateral sclerosis, prion diseases including Creutzfeldt-Jakob disease (including variant form), kuru, fatal familial insomnia, Gerstmann-Straussler-Scheinker disease and other transmissible spongiform encephalopathies, prion protein cerebral amyloid angiopathy, postencephalitic Parkinsonism, progressive muscular atrophy, progressive bulbar palsy, progressive subcortical gliosis, progressive supranuclear palsy, restless leg syndrome, Rett syndrome, Sandhoff disease, spasticity, sporadic fronto-temporal dementias, striatonigral degeneration, subacute sclerosing panencephalitis, sulphite oxidase deficiency, Sydenham's chorea, tangle only dementia, Tay-Sach's disease, Tourette's syndrome, vascular dementia, and Wilson disease.

Additional neurodegenerative diseases for which agents inhibiting Lp-PLA₂ using the agents as described herein may be useful include other dementias not listed above, such as but without limitation, other mixed dementia, frontotemporal dementia, Pick's Disease, progressive supranuclear palsy (PSP), Parkinson's Disease with associated dementia, corticobasal degeneration, multiple system atrophy, HIV-induced dementia, white matter disease-associated dementias, mild cognitive impairment (MCI).

Agents inhibiting Lp-PLA₂ using the agents as described herein may be useful in preventing and treating BBB permeability in a subject. Additional examples of diseases and disorders where BBB permeability occurs include, for example, multiple sclerosis, cerebral amyloid angiopathy, diabetic retinopathy, prion disorders, amyotrophic lateral sclerosis (ALS), Stiff-person Syndrome, Spinocerebellar Ataxias, Friedreich Ataxia, Ataxia Telangiectasia, Bulbospinal Atrophy (Kennedy Syndrome), Spinal Muscular Atrophy, Neuronal storage diseases (lipofuscinoses), Mitochondrial encephalomyopathies, Leukodystrophies, Neural sequelae of spinal shock/blunt trauma, Hypertensive Cerebrovascular disease, such as Lacunar Infarcts, Slit hemorrhages, Hypertensive encephalopathy. BBB permeability also occurs in brain tumors such as those with 'sinusoidal' (aka high nutrient) vascular supply.

BBB permeability also occurs in neurological sequellae associated with Streptococcal infections; pediatric autoimmune neuropsychiatric disorders associated with streptococcal infections (PANDAS), Tourette's syndrome, obsessive compulsive disease (OCD). BBB permeability also occurs in the following diseases and disorders; post-anesthesia neuropsychological dysfunction and neuropsychiatric disorders associated with any vasculopathy (e.g. Lupus, hypertension, etc.). BBB permeability also occurs in subjects with infections (either entry into the CNS, propagation within the CNS, or exit from the CNS upon established infection), for example, HIV, Tertiary Syphilis, Neuroborreliosis (Lyme Disease), Herpes Simplex Virus Type 1 (HSV-1)/ Herpes Simplex Virus Type 2 (HSV-2), Varicella-Zoster Virus (Herpes Zoster), Cytomegalovirus, Poliomyelitis, Rabies, Progressive Multifocal Leukoencephalopathy, Subacute Sclerosing Panencephalitis (post-measles), Protozoal diseases (toxoplasmosis, amebiasis, and trypanosomiasis), Rickettsial infections (typhus and Rocky Mountain spotted fever), Metazoal diseases, Malaria, and Encephalitis/Meningitis. Encephalitis/meningitis may result from sepsis.

Agents inhibiting Lp-PLA₂ using the agents as described herein may be useful in preventing and/or treating BBB permeability occurring in subjects with or at risk of autism. Autism is a largely heritable disorder that is hallmarked by the expression of social deficits, language abnormalities and stereotyped, repetitive behaviors (American Psychiatric Association, 1994). Neuropathological and neuroimaging studies have reported increased brain size and weight (Bailey et al., 1998; Kemper and Bauman, 1998; Sparks et al., 2002; Herbert et al., 2003; Palmen et al., 2004). Many studies of autistic brains have reported an overall reduction in cell size and an increased cell packing density, especially in the hippocampus, subiculum and amygdala (Kemper and Bauman, 1993), indicating resident neurons have small dendritic trees and possible incomplete maturation or arrested development.

Autoimmunity can contribute to the pathogenesis of autism, where the binding of autoantibodies to neurons disrupts the normal pattern of neurodevelopment at critical stages. Autoantibodies to brain have been reported in autistic children (Singh et al., 1988) and several autoimmune factors including brain-specific autoantibodies (Gupta et al., 1996; Singh and Rivas, 2004), impaired lymphocyte function (Warren et al., 1996), abnormal cytokine regulation and viral associations (Singh, 2001). Singh and Rivas (2004) have shown that the serum of autistic children harbors brain-specific autoantibodies, and in 68 autistic children at 4-12 years of age, antibodies to caudate nucleus, cerebral cortex and cerebellum were detected in 49%, 18% and 9%, respectively, of autistic children, but not in normal children.

Another study has shown that children with Tourette syndrome possess anti-striatal antibodies, and experimental influsion of these antibodies into the rat striatum caused neuronal dysfunction similar to Tourette syndrome in rats (Hallet et al., 2000). A strong link between the presence of anti-neuronal autoantibodies and neurological disease has been shown most dramatically in children in cases following streptococcal infections, such as in obsessive compulsive disorder (OCD), Sydenham's chorea, Tourette syndrome, pediatric autoimmune neuropsychiatric disorders associated with streptococcus (PANDAS), paraneoplasia and in elderly patients with systemic lupus erythematosus that show both cognitive and memory loss (Swedo et al., 1989; Kalume et al., 2004; Tanaka et al. 2004).

Access of autoantibodies to neurons within the brain would naturally be restricted by the integrity of the blood-brain barrier (BBB), at least in normal healthy individuals. For antibodies to gain access to brain neurons, the blood-brain barrier can be either defective or developmentally delayed. A link between the establishment of the BBB at or near six months of age, the development of the normal immune repertoire of the neonate and disturbances in neurodevelopment can ultimately lead to autism. During the late fetal period and first few months of life, critical neuronal pathways controlling the reception of environmental signals and individual and appropriate response pathways to these signals are being established and refined. A developmental delay or defect in the formation of the BBB at this time can allow exposure of neurons to blood-borne antineuronal autoantibodies that could impair the normal pattern of neuronal wiring, leading to autism.

### Assessment of inhibitors of Lp-PLA₂ on models of neurodegenerative diseases and/or BBB permeability.

The suitability of an inhibitor of Lp-PLA₂ for the treatment of a neurodegenerative disease can be assessed in any of a number of animal models for neurodegenerative disease. For example, mice transgenic for an expanded polyglutamine repeat mutant of ataxin-1 develop ataxia typical of spinocerebellar ataxia type 1 (SCA- 1) are known (Burright et al., 1995, Cell 82: 937-948; Lorenzetti et al., 2000, Hum. Mol. Genet. 9: 779-785; Watase, 2002, Neuron 34: 905- 919), and can be used to determine the efficacy of an agent inhibitor of Lp-PLA₂ for the treatment or prevention of neurodegenerative disease. Additional animal models, for example, for Huntington's disease (see, e.g., Mangiarini et al., 1996, Cell 87: 493-506, Lin et al., 2001, Hum. Mol. Genet. 10: 137- 144), Alzheimer's disease (Hsiao, 1998, Exp. Gerontol, 33: 883-889; Hsiao et al., 1996, Science 274: 99-102), Parkinson's disease (Kim et al., 2002, Nature 418: 50-56), amyotrophic lateral sclerosis (Zhu et al., 2002, Nature 417: 74-78), Pick's disease (Lee & Trojanowski, 2001, Neurology 56 (Suppl. 4): S26-S30, and spongiform encephalopathies (He et al., 2003, Science 299: 710-712) can be used to evaluate the efficacy of the agents that inhibit Lp-PLA₂ as described herein in a similar manner.

Animal models are not limited to mammalian models. For example, Drosophila strains provide accepted models for a number of neurodegenerative disorders (reviewed in Fortini & IBonini, 2000, Trends Genet. 16: 161-167; Zoghbi & Botas, 2002, Trends Genet. 18: 463-471). These models include not only flies bearing mutated fly genes, but also flies bearing human transgenes, optionally with targeted mutations. Among the Drosophila models available are, for example, spinocerebellar ataxias (e.g., SCA-1 (see, e.g., WO 02/058626), SCA-3 (Warrick et al., 1998, Cell 93: 939-949)), Huntington's disease (Kazemi- Esfarjani & Benzer, 2000, Science 287: 1837-1840), Parkinson's disease (Feany et al, 2000, Nature 404: 394-398; Auluck et al., 2002, Science 295: 809-8 10), age-dependent neurodegeneration (Genetics, 2002,161:4208), Alzheimer's disease (Selkoe et al., 1998, Trends Cell Biol. 8: 447-453; Ye et al., 1999, J. Cell Biol. 146: 1351- 1364), amyotrophic lateral sclerosis (Parkes et al., 1998, Nature Genet. 19: 171-174), and adrenoleukodystrophy.

The use of Drosophila as a model organism has proven to be an important tool in the elucidation of human neurodegenerative pathways, as the Drosophila genome contains many relevant human orthologs that are extremely well conserved in function (Rubin, G.M., et al., Science 287: 2204-2215 (2000)). For example, Drosophila melanogaster carries a gene that is homologous to human APP which is involved in nervous system function. The gene, APP-like (APPL), is approximately 40% identical to APP695, the neuronal isoform (Rosen et al., Proc. Nati. Acad. Sci. U.S.A. 86:2478-2482 (1988)), and like human APP695 is exclusively expressed in the nervous system. Flies deficient for the APPL gene show behavioral defects which can be rescued by the human APP gene, suggesting that the two genes have similar functions in the two organisms (Luo et al., Neuron 9:595-605 (1992)). Drosophila models for Alzheimer's disease are disclosed in U.S. Patent Applications 2004/0244064, 2005/0132425, 2005/0132424, 2005/0132423, 2005/0132422, 200/50132421, 2005/0108779, 2004/0255342, 2004/0255341, 2004/0250302.

In addition, Drosophila models of polyglutamine repeat diseases (Jackson, G.R., et al., Neuron 21:633- 642 (1998); Kazemi-Esfarani, P. and Benzer, S., Science 287:1837-1840 (2000); Fernandez- Funez et al., Nature 408:101-6 (2000)), Parkinson's disease (Feany, M.B. and Bender, W.W., Nature 404:394-398 (2000)) and other diseases have been established which closely mimic the disease state in humans at the cellular and physiological levels, and have been successfully employed in identifying other genes that can be involved in these diseases. The transgenic flies exhibit progressive neurodegeneration which can lead to a variety of altered phenotypes including locomotor phenotypes, behavioral phenotypes (e.g., appetite, mating behavior, and/or life span), and morphological phenotypes (e.g., shape, size, or location of a cell, organ, or appendage; or size, shape, or growth rate of the fly).

Animals administered the compounds are evaluated for symptoms relative to animals not administered the compounds. A measurable change in the severity a symptom (i.e., a decrease in at least one symptom, i.e. 10% or greater decrease), or a delay in the onset of a symptom, in animals treated with an inhibitor of Lp-PLA₂ versus untreated animals is indicative of therapeutic efficacy.

One can assess the animals for memory and learning, for instance by performing behavioral testing. One can use any behavioral test for memory and learning commonly known by person of ordinary skill in the art, for but not limited to the Morris water maze test for rodent animal models. A measurable increase in the ability to perform the Morris water maze test in animals administered an Lp-PLA₂ inhibitor versus untreated animals is indicative of therapeutic efficacy.

The suitability of an inhibitor of Lp-PLA₂ for the treatment of a BBB disease or disorder can be assessed in any of a number of animal models where BBB abnormality and/or BBB permeability occurs. One method that can be used is to assess the ability of blood-borne components, such as Ig or amyloid beta (Aβ) peptides to cross the BBB and interact with neurons in the brain. One method useful in the methods as described herein to assess blood-borne components, such as Ig or amyloid beta (Aβ) peptides crossing the BBB uses fluorescent labeled Abeta42, and is described in Clifford et al., 2007, Brain Research 1142: 223-236,. In this method, the ability of blood-borne Aβ peptides to cross a defective BBB was assessed using fluorescein isothiocyanate (FITC)-labeled Aβ42 and Aβ40 introduced via tail vein injection into mice with a BBB rendered permeable by treatment with pertussis toxin. Both Aβ40 and Aβ42 were shown to cross the permeabilized BBB and bound selectively to certain neuronal subtypes, but not glial cell, with widespread Aβ42-positive neurons in the brain 48 hrs post-injection. As a control, animals with intact BBB (saline-injected controls) blocked entry of blood-borne Aβ peptides into the brain. One can use such an animal model to assess the ability of an inhibitor of Lp-PLA₂ on the prevention or treatment of BBB breakdown or BBB permeability by assessing Aβ42-positive neurons in the brain 48 hrs post-injection of pertussis toxin and FITC-labeled Aβ42 in the presence or absence of an inhibitor of Lp-PLA₂. A decrease in Aβ42-positive neurons in the brain in animals administered an Lp-PLA₂ inhibitor as compared to animals not administered a Lp-PLA₂ inhibitor indicates the Lp-PLA2 inhibitor is a effective at treating and/or preventing BBB permeability.

One can also use other trace molecules in animal models of BBB permeability. One can detect labeled blood-borne components in brain tissue, for example fluorescent-labeled or 35S labeled Abeta peptides, 125I or 35S-labeled immunoglobulin (Ig), Horse radish peroxidase (HRP), evans blue, fluorescent or magnetic microbeads (Molecular Probes) and dextrans of different molecular weight.

Pertussis toxin is derived from *Bordetella pertussis,* is known to effectively increase the permeability of the BBB in mice as early as 24 h after injection and for a period of up to several weeks (Amiel, 1976; Bruckener et al., 2003), and is likely involved in the development of neurological sequellae associated with whooping cough that are linked to disruption of BBB integrity in the epithelium of the choroid plexus and/or cerebral capillary endothelium. Pertussis toxin has also been used to enhance the development of experimental autoimmune encephalomyelitis (EAE), a widely used mouse model for multiple sclerosis (Ben-Nun et al., 1997; Linthicum et al., 1982; Yong et al., 1993).

### Formulations of compositions

Compounds, for example agents inhibiting Lp-PLA₂ as described herein, can be used as a medicament or used to formulate a pharmaceutical composition with one or more of the utilities disclosed herein. They can be administered in vitro to cells in culture, in vivo to cells in the body, or ex vivo to cells outside of an individual that can later be returned to the body of the same individual or another. Such cells can be disaggregated or provided as solid tissue.

Compounds, for example agents inhibiting Lp-PLA₂ as described herein can be used to produce a medicament or other pharmaceutical compositions. Use of agents inhibiting Lp-PLA₂ which further comprise a pharmaceutically acceptable carrier and compositions which further comprise components useful for delivering the composition to an individual are known in the art. Addition of such carriers and other components to the agents as described herein is well within the level of skill in this art.

Pharmaceutical compositions can be administered as a formulation adapted for passage through the blood-brain barrier or direct contact with the endothelium. The compositions may be administered as a formulation adapted for systemic delivery. The compositions may be administered as a formulation adapted for delivery to specific organs, for example but not limited to the liver, bone marrow, or systemic delivery.

Alternatively, pharmaceutical compositions can be added to the culture medium of cells ex vivo. In addition to the active compound, such compositions can contain pharmaceutically-acceptable carriers and other ingredients known to facilitate administration and/or enhance uptake (e.g., saline, dimethyl sulfoxide, lipid, polymer, affinity-based cell specific-targeting systems). The composition can be incorporated in a gel, sponge, or other permeable matrix (e.g., formed as pellets or a disk) and placed in proximity to the endothelium for sustained, local release. The composition can be administered in a single dose or in multiple doses which are administered at different times.

Pharmaceutical compositions can be administered by any known route. By way of example, the composition can be administered by a mucosal, pulmonary, topical, or other localized or systemic route (e.g., enteral and parenteral). The phrases "parenteral administration" and "administered parenterally" as used herein means modes of administration other than enteral and topical administration, usually by injection, and includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrasternal injection, infusion and other injection or infusion techniques, without limitation.The phrases "systemic administration," "administered systemically", "peripheral administration" and "administered peripherally" as used herein mean the administration of the agents as described herein such that it enters the animal's system and, thus, is subject to metabolism and other like processes, for example, subcutaneous administration.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The phrase "pharmaceutically acceptable carrier" as used herein means a pharmaceutically acceptable material, composition or vehicle, such as a liquid or solid filler, diluent, excipient, solvent or encapsulating material, involved in carrying or transporting the subject agents from one organ, or portion of the body, to another organ, or portion of the body. Each carrier must be "acceptable" in the sense of being compatible with the other ingredients of the formulation, for example the carrier does not decrease the impact of the agent on the treatment. In other words, a carrier is pharmaceutically inert.

Suitable choices in amounts and timing of doses, formulation, and routes of administration can be made with the goals of achieving a favorable response in the subject with vascular dementia, for example a subject with Alzheimer's disease or a risk thereof (i.e., efficacy), and avoiding undue toxicity or other harm thereto (i.e., safety). Therefore, "effective" refers to such choices that involve routine manipulation of conditions to achieve a desired effect.

A bolus of the formulation administered to an individual over a short time once a day is a convenient dosing schedule. Alternatively, the effective daily dose can be divided into multiple doses for purposes of administration, for example, two to twelve doses per day. Dosage levels of active ingredients in a pharmaceutical composition can also be varied so as to achieve a transient or sustained concentration of the compound or derivative thereof in an individual, especially in and around vascular endothelium of the brain, and to result in the desired therapeutic response or protection. But it is also within the skill of the art to start doses at levels lower than required to achieve the desired therapeutic effect and to gradually increase the dosage until the desired effect is achieved.

The amount of agents inhibiting Lp-PLA₂ administered is dependent upon factors known to a person skilled in the art such as bioactivity and bioavailability of the compound (e.g., half-life in the body, stability, and metabolism); chemical properties of the compound (e.g., molecular weight, hydrophobicity, and solubility); route and scheduling of administration, and the like. It will also be understood that the specific dose level to be achieved for any particular individual can depend on a variety of factors, including age, gender, health, medical history, weight, combination with one or more other drugs, and severity of disease.

The term "treatment", with respect to treatment of vascular dementia, or Alzheimer's disease or a disease associated with defective BBB refers to, inter alia, preventing the development of the disease, or altering the course of the disease (for example, but not limited to, slowing the progression of the disease), or reversing a symptom of the disease or reducing one or more symptoms and/or one or more biochemical markers in a subject, preventing one or more symptoms from worsening or progressing, promoting recovery or improving prognosis, and/or preventing disease in a subject who is free therefrom as well as slowing or reducing progression of existing disease. For a given subject, improvement in a symptom, its worsening, regression, or progression can be determined by an objective or subjective measure. Modification of one or more biochemical markers, for example permeability, presence of extravascular Ig in the brain, or beta amyloid in the CSF for example can be measured.

Efficacy of treatment can be measured as an improvement in morbidity or mortality (e.g., lengthening of survival curve for a selected population). Prophylactic methods (e.g., preventing or reducing the incidence of relapse) are also considered treatment.

Treatment can also involve combination with other existing modes of treatment, for example existing agents for treatment of Alzheimer's disease, for example but are not limited to ARICEPT or donepezil, COGNEX or tacrine, EXELON or rivastigmine, REMINYL or galantamine, antiamyloid vaccine, Abeta-lowering therapies, mental exercise or stimulation; see for review Zlokovic, Adv. Drug Deliv. Rev. 54:1533-1660, 2002).

Agents that inhibit Lp-PLA₂ as described herein can be combined with other agent, for example therapeutic agent to prevent and/or treat neurodegenerative diseases. Such agents can be any agent currently in use or being developed for the treatment and/or prevention of a neurodegenerative disease or disorder, where the agent can have a prophylactic and/or a curative effect and/or reduce a symptom of a neurodegenerative disorder or disease.

Where inhibitor agents of Lp-PLA₂ as described herein are used for the prevention and/or treatment of Alzheimer's disease and/or vascular dementia, the inhibitor agents of Lp-PLA₂ as described herein can be used in combination with medicaments commonly known by person of ordinary skill in the art that are claimed to be useful as symptomatic treatments of dementia. Examples of such medicaments include, but are not limited to, agents known to modify cholinergic transmission such as M1 muscarinic receptor agonists or allosteric modulators, M2 muscarinic antagonists, acetylcholinesterase inhibitors (such as tetrahydroaminoacridine, donepezil, galantamine and rivastigmine), nicotinic receptor agonists or allosteric modulators (such as α7 agonists or allosteric modulators or α4β2 agonists or allosteric modulators), peroxisome proliferator-activated receptors (PPAR) agonists (such as PPARγ agonists), 5-HT4 receptor partial agonists, histamine H3 antagonists and inverse agonists, 5-HT6 receptor antagonists or 5HT1A receptor antagonists, AMPA positive modulators (alpha-amino-3-hydroxy-5-methylisoxazole-4-propionate, a glutamate receptor subtype) and N-methyl-D-aspartic acid (NMDA) receptor antagonists or modulators (such as memantine).

Where the inhibitor agents of Lp-PLA₂ as described herein are used for the treatment of Alzheimer's disease, the inhibitor agents of Lp-PLA₂ as described herein can be used in combination with those medicaments mentioned above that are claimed to be useful as symptomatic treatments of dementia and/or disease-modifying agents. Disease modifying agents include, for example but are not limited to, gamma secretase inhibitors and modulators, and human beta-secretase (BACE) inhibitors. Disease modifying agents also are, for example but not limited to gamma secretase inhibitors and modulators, beta-secretase (BACE) inhibitors and any other anti-amyloid approaches including active and passive immunization, for example agents identified by the methods as disclosed in U.S. Patent Application 2005/0170359, as well as agents as disclosed in International Patent Applications WO05/07277, WO03/104466 and WO07/028133, and U.S. Patent 6,866,849, 6,913,745.

Thus, combination treatment with one or more agents that inhibit Lp-PLA₂ with one or more other medical procedures can be practiced.

In addition, treatment can also comprise multiple agents to inhibit Lp-PLA₂ expression or activity. For example, other agents include the use of statins with Niacin (see http://www.genengnews.com/news/bnitem.aspx?name=6724568) and fenofibrate (see http://www.genengnews.com/news/bnitem.aspx?name=14817756&taxid=19)

Similarly, diagnosis can be practiced with other diagnostic procedures. For example, endothelium of the vascular system, brain, or spinal cord (e.g., blood or leptomeningeal vessels) can be assayed for a change in gene expression profiles using disease-specific molecular diagnostics kits (e.g., custom made arrays, multiplex QPCR, multiplex proteomic arrays). In addition, a noninvasive diagnostic procedure (e.g., CAT, MRI, SPECT, or PET) can be used in combination to improve the accuracy and/or sensitivity of diagnosis. Early and reliable diagnosis is especially useful to for treatments that are only effective for mild to moderate Alzheimer's disease or only delay its progression.

The amount which is administered to a subject is preferably an amount that does not induce toxic effects which outweigh the advantages which result from its administration. Further objectives are to reduce in number, diminish in severity, and/or otherwise relieve suffering from the symptoms of the disease in the individual in comparison to recognized standards of care.

Production of compounds according to present regulations will be regulated for good laboratory practices (GLP) and good manufacturing practices (GMP) by governmental agencies (e.g., U.S. Food and Drug Administration). This requires accurate and complete record keeping, as well as monitoring of QA/QC. Oversight of patient protocols by agencies and institutional panels is also envisioned to ensure that informed consent is obtained; safety, bioactivity, appropriate dosage, and efficacy of products are studied in phases; results are statistically significant; and ethical guidelines are followed. Similar oversight of protocols using animal models, as well as the use of toxic chemicals, and compliance with regulations is required.

Dosages, formulations, dosage volumes, regimens, and methods for analyzing results aimed at inhibiting Lp-PLA₂ expression and/or activity can vary. Thus, minimum and maximum effective dosages vary depending on the method of administration. Suppression of the clinical and histological changes associated with vascular dementia can occur within a specific dosage range, which, however, varies depending on the organism receiving the dosage, the route of administration, whether agents that inhibit Lp-PLA₂ are administered in conjunction with other co-stimulatory molecules, and the specific regimen of inhibitor of Lp-PLA₂ administration. For example, in general, nasal administration requires a smaller dosage than oral, enteral, rectal, or vaginal administration.

For oral or enteral formulations, tablets can be formulated in accordance with conventional procedures employing solid carriers well-known in the art. Capsules employed for oral formulations can be made from any pharmaceutically acceptable material, such as gelatin or cellulose derivatives. Sustained release oral delivery systems and/or enteric coatings for orally administered dosage forms are also contemplated, such as those described in U.S. Pat. No. 4,704,295, "Enteric Film-Coating Compositions," issued Nov. 3, 1987; U.S. Pat. No. 4, 556,552, "Enteric Film- Coating Compositions," issued Dec. 3, 1985; U.S. Pat. No. 4,309,404, "Sustained Release Pharmaceutical Compositions," issued Jan. 5, 1982; and U.S. Pat. No. 4,309,406, "Sustained Release Pharmaceutical Compositions," issued Jan. 5, 1982.

Examples of solid carriers include starch, sugar, bentonite, silica, and other commonly used carriers. Further non-limiting examples of carriers and diluents which can be used in the formulations described include saline, syrup, dextrose, and water.

### Enteric Coated Formulation

As regards formulations for administering the small chemical entities for inhibitors of Lp-PLA₂ of the likes of formulas (I) - (IV) as described herein, one particularly useful example is a tablet formulation comprising the Lp-PLA inhibitor with an enteric polymer casing. An example of such a preparation can be found in WO2005/021002. The active material in the core can be present in a micronised or solubilised form. In addition to active materials the core can contain additives conventional to the art of compressed tablets. Appropriate additives in such a tablet can comprise diluents such as anhydrous lactose, lactose monohydrate, calcium carbonate, magnesium carbonate, dicalcium phosphate or mixtures thereof; binders such as microcrystalline cellulose, hydroxypropylmethylcellulose, hydroxypropyl-cellulose, polyvinylpyrrolidone, pre-gelatinised starch or gum acacia or mixtures thereof; disintegrants such as microcrystalline cellulose (fulfilling both binder and disintegrant functions) cross-linked polyvinylpyrrolidone, sodium starch glycollate, croscarmellose sodium or mixtures thereof; lubricants, such as magnesium stearate or stearic acid, glidants or flow aids, such as colloidal silica, talc or starch, and stabilisers such as desiccating amorphous silica, colouring agents, flavours etc. Preferably the tablet comprises lactose as diluent. When a binder is present, it is preferably hydroxypropylmethyl cellulose. Preferably, the tablet comprises magnesium stearate as lubricant. Preferably the tablet comprises croscarmellose sodium as disintegrant. Preferably, the tablet comprises microcrystalline cellulose.

The diluent can be present in a range of 10 - 80% by weight of the core. The lubricant can be present in a range of 0.25 - 2% by weight of the core. The disintegrant can be present in a range of 1 - 10% by weight of the core. Microcrystalline cellulose, if present, can be present in a range of 10 - 80% by weight of the core.

The active ingredient preferably comprises between 10 and 50% of the weight of the core, more preferably between 15 and 35% of the weight of the core. (calculated as free base equivalent). The core can contain any therapeutically suitable dosage level of the active ingredient, but preferably contains up to 150mg as free base of the active ingredient. Particularly preferably, the core contains 20, 30, 40, 50, 60, 80 or 100mg as free base of the active ingredient. The active ingredient can be present as the free base, or as any pharmaceutically acceptable salt. If the active ingredient is present as a salt, the weight is adjusted such that the tablet contains the desired amount of active ingredient, calculated as free base of the salt. Preferably, the active ingredient is present as a hydrochloride salt.

The core can be made from a compacted mixture of its components. The components can be directly compressed, or can be granulated before compression. Such granules can be formed by a conventional granulating process as known in the art. Alternatively, the granules can be individually coated with an enteric casing, and then enclosed in a standard capsule casing.

The core is surrounded by a casing which comprises an enteric polymer. Examples of enteric polymers are cellulose acetate phthalate, cellulose acetate succinate, methylcellulose phthalate, ethylhydroxycellulose phthalate, polyvinylacetate pthalate, polyvinylbutyrate acetate, vinyl acetate-maleic anhydride copolymer, styrene-maleic mono-ester copolymer, methyl acrylate-methacrylic acid copolymer or methacrylate-methacrylic acid-octyl acrylate copolymer. These can be used either alone or in combination, or together with other polymers than those mentioned above. The casing can also include insoluble substances which are neither decomposed nor solubilised in living bodies, such as alkyl cellulose derivatives such as ethyl cellulose, crosslinked polymers such as styrene-divinylbenzene copolymer, polysaccharides having hydroxyl groups such as dextran, cellulose derivatives which are treated with bifunctional crosslinking agents such as epichlorohydrin, dichlorohydrin or 1, 2-, 3, 4-diepoxybutane. The casing can also include starch and/or dextrin.

Preferred enteric coating materials are the commercially available Eudragit® enteric polymers such as Eudragit® L, Eudragit® S and Eudragit® NE used alone or with a plasticiser. Such coatings are normally applied using a liquid medium, and the nature of the plasticiser depends upon whether the medium is aqueous or non-aqueous. Plasticisers for use with aqueous medium include propylene glycol, triethyl citrate, acetyl triethyl citrate or Citroflex® or Citroflex® A2. Non-aqueous plasticisers include these, and also diethyl and dibutyl phthalate and dibutyl sebacate. A preferred plasticiser is Triethyl citrate. The quantity of plasticiser included will be apparent to those skilled in the art.

The casing can also include an anti-tack agent such as talc, silica or glyceryl monostearate. Preferably the anti-tack agent is glyceryl monostearate. Typically, the casing can include around 5-25 wt% Plasticiser and up to around 50 wt % of anti tack agent, preferably 1-10 wt % of anti-tack agent.

If desired, a surfactant can be included to aid with forming an aqueous suspension of the polymer. Many examples of possible surfactants are known to the person skilled in the art. Preferred examples of surfactants are polysorbate 80, polysorbate 20, or sodium lauryl sulphate. If present, a surfactant can form 0.1 - 10% of the casing, preferably 0.2 - 5% and particularly preferably 0.5 - 2%

There may be a seal coat included between the core and the enteric coating. A seal coat is a coating material which can be used to protect the enteric casing from possible chemical attack by any alkaline ingredients in the core. The seal coat can also provide a smoother surface, thereby allowing easier attachment of the enteric casing. A person skilled in the art would be aware of suitable coatings. Preferably the seal coat is made of an Opadry coating, and particularly preferably it is Opadry White OY-S-28876.

In one embodiment, the pharmaceutically active ingredient is 1-(N-(2-(diethylamino)ethyl)-N-(4-(4-trifluoromethylphenyl)benzyl)aminocarbonylmethyl)-2-(4-fluorobenzyl)thio-5,6-trimethylenepyrimidin-4-one, or a salt thereof.

One example of such an enteric-coated formulation, as described in WO2005/021002, comprises varying amounts of 1-(N-(2-(diethylamino)ethyl)-N-(4-(4-trifluoromethylphenyl)benzyl)aminocarbonylmethyl)-2-(4-fluorobenzyl)thio-5,6-trimethylenepyrimidin-4-one (called "active" in this example) as hydrochloride salt.

In that example, lactose monohydrate, microcrystalline cellulose, the active ingredient, the hydroxypropyl methyl cellulose and half of the croscarmellose sodium were screened into a 10 Litre Fielder high-shear blender (any suitable high shear blender could be used) and blended for 5 minutes at 300 rpm with the chopper off. The mixture was then granulated by the addition of about 750 ml water whilst continuing to blend. The granules were dried in a Glatt 3/5 fluid bed drier, screened by Comil into a Pharmatec 5 Litre bin blender and then blended with any lactose anhydrous given in the formula plus the remainder of the croscarmellose sodium over 5 minutes at 20 rpm. Magnesium stearate was screened into the blender and the mixing process continued for a further 1 minute at 10 rpm. The lubricated mix was compressed using a Riva Piccolla rotary tablet press fitted with 9.5mm round normal convex punches (any suitable tablet press could be used). The sealcoat, and subsequently the enteric coat, are applied by spraying of an aqueous suspension of the coat ingredients in a Manesty 10 coater using parameters for the coating process as recommended by the manufacturers of the coating polymers (again, any suitable coater could be used).

Other enteric-coated preparations of this sort can be prepared by one skilled in the art, using these materials or their equivalents.

### EXAMPLES

The examples presented herein relate to the methods and compositions for the prevention and/or treatment of neurodegenerative disorders, for example but not limited to vascular dementia and disorders of the blood brain barrier, for example Alzheimer's disease, Huntington's disease, Parkinson's disease by inhibition of Lp-PLA₂. Throughout this application, various publications are referenced. The following examples are not intended to limit the scope of the claims to the invention, but are rather intended to be exemplary of certain embodiments. Any variations in the exemplified methods which occur to the skilled artisan are intended to fall within the scope of the present invention.

### METHODS

*Animal model:* A diabetic/diabetic hypercholestrolemic pig model (DM/HC) was developed that mimics human-like atherosclerosis. Farm pigs weighing 25-30 kg and aged ~4 months were made diabetic with a single intravenous injection of 125 mg/kg of streptozotocin (Sicor Pharmaceuticals, Irvine, CA). After stabilization for 1-2 weeks, the animals with elevated levels of plasma glucose (> 150 mg/dl) were fed with atherogenic (high-fat) diet as shown in Table 1 (Animal Specialties, Quakertown, PA) to achieve a cholesterol level of approximately 250-800 mg/dl. Maintenance of the cholesterol level was determined by method as shown in Table 2.

**Table 1. Diet for 2.0% cholesterol diet the components are:**

| **Component** | **Weight/Weight %** |
|---|---|
| Purina* porcine grower meal | 47.5 % |
| Lard | 25.0 % |
| Casein | 11.1 % |
| Dried whole milk | 7.9 % |
| Peanut oil | 2.37 % |
| Cholesterol | 2.0 % |
| Wesson salt mix | 2.37 % |
| Purina* vitamin mix | 1.58 % |
| Sodium cholate | 1.58 % |
| Calcium carbonate | 0.4 % |
| Choline chloride | 0.2 % |

| | |
|---|---|
| *Purina Mills, LLC, Checkerboard Square, St. Louis, Missouri, 63164, USA. These feeds were prepared by Animal Specialties and Provisions, LLC, Quakertown, PA USA. | |

For the 0.5% cholesterol diet the components are similar with the exception of 0.5% cholesterol and 20% lard. The animals were Yorkshire pigs that were castrated males at the age of 3-5 and were obtained from Archer Farms, Darlington, MD. These feeds were prepared by Animal Specialties and Provisions, LLC, Quakertown, PA USA.

On days 1-2, animals were fed normal chow, followed by on days 3-14 animals were fed a diet of 0.5% cholesterol, 2% lard and on day 14, cholesterol levels were measured and the diet adjusted accordingly to increase to 2% cholesterol, 10% lard if cholesterol is <300 mg/dl. Following induction of DM/HC, cholesterol was measured until cholesterol levels are stable between 300 and 800 mg/dl, and following cholesterol stabilization, cholesterol was measured monthly. If cholesterol levels were unstable following initial stabilization phase, the diet of the animal was returned to the initial two-week measurement schedule. Monthly cholesterol levels were determined, including levels of total cholesterol, LDL, HDL, VLDL and triglycerides. Adjustment of the diet of the animal for a stable cholersteol level was determined according to the outlines shown in Table 2.

**Table 2. Cholesterol and Dietry adjustment.**

| **Cholesterol level** | **Dietary adjustment** | **Next measurement** | **Cholesterol level** | **Dietary adjustment** | **Next cholester ol measure ment** |
|---|---|---|---|---|---|
| <250 mg/dl | Change to 25% lard diet. | 2 weeks | <300 mg/dl | Continue 25% lard diet | 2 weeks |
| | | | 300-800 mg/dl | Change to 75% lard (25% lard): 25% normal diet | 2 weeks |
| | | | >800 mg/dl | Change to 100% 10% lard diet | 2 weeks |
| | | | >1000 mg/dl | Change to 50:50 mix of 10% lard diet | 2 weeks |
| | | | >1500 mg/dl | Change to normal diet | 2 weeks* |
| | | | | | |
| 300-800 mg/dl | No change. 10% lard diet | 2 weeks | < 300 mg/dl | Change to 25% lard diet | 2 weeks |
| | | | 300-800 mg/dl | No change | Regular schedule |
| | | | >800 mg/dl | Change to 50:50 mixture with 10% lard | 2 weeks |
| | | | >1000 mg/dl | Change to 25:75 mixture with 10% lard | 2 weeks |
| | | | >1500 mg/dl | Normal chow | 2 weeks |
| | | | | | |
| 800-1000 mg/dl | Change to 50:50 mix of 10% lard and normal chow diet. | 2 weeks | < 300 mg/dl | Change to 25:75 mixture with 10% lard diet | 2 weeks |
| | | | 300-800 mg/dl | No change in diet (50:50) 10% lard | 2 weeks |
| | | | >800 mg/dl | Change to 25:75 mixture (10% lard) | 2 weeks |
| | | | >1000 mg/dl | Change to 25:75 mixture (10% lard) | 2 weeks |
| | | | >1500 mg/dl | Normal chow | 2 weeks* |
| | | | | | |
| >1000 mg/dl | 25:75 mix of 10% lard and normal chow diet. | 2 weeks | < 300 mg/dl | Change to 50:50 mix of 10% lard and normal chow diet. | 2 weeks |
| | | | 300-800 mg/dl | No change in diet 25:75 mix of 10% lard and normal chow diet | 2 weeks |
| | | | >800 mg/dl | No change in diet | 2 weeks |
| | | | >1000 mg/dl | Normal chow | 2 weeks* |
| | | | >1500 mg/dl | Normal chow | 2 weeks* |
| | | | | | |
| >1500 mg/dl | Normal chow diet. | 2 weeks | < 300 mg/dl | Change to 100% of 10% lard diet. | 2 weeks |
| | | | 300-800 mg/dl | Change diet to 50:50 mix of 10% lard and normal chow diet | 2 weeks |
| | | | >800 mg/dl | Normal chow | 2 weeks* |
| | | | >1000 mg/dl | Normal chow | 2 weeks* |
| | | | >1500 mg/dl | Normal chow | 2 weeks* |

At one month after stabilization of cholesterol at 250-800mg/dl, the animals were randomized into two experimental groups, DM/HC (hyperglycemia and hypercholesterolemia) group with no treatment and treatment group (10 mg/kg/day of SB-480848, also referred to as 1-(N-(2-(diethylamino)ethyl)-N-(4-(4-trifluoromethylphenyl)benzyl)-aminocarbonylmethyl)-2-(4-fluorobenzyl)thio-5,6-trimethylenepyrimidin-4-one). The animals were sacrificed at 6-month after the randomization. The animal protocol has been approved by the Institutional Animal Care and Use Committee of University of Pennsylvania.

Two diabetic/hypercholesterolemic groups were evaluated: 1. DM/HC group and 2. DM/HC animals receiving Lp-PLA₂ inhibitors. The experiments included: the control group (DM/HC group - 17 pigs) and the experimental group (DM/HC animals receiving LP-PLA₂ inhibitors - 20 pigs). In addition blood cholesterol levels were maintained between 300 and 800 mg/dl in experimental animals, this range having been determined to provide a better test model. Blood cholesterol levels were monitored in all animals on a bimonthly basis, as shown in Table 4 and adjustments were made to the fat content of the feed accordingly, as shown in Table 2. The cholesterol and lard percent were in the range of 0.5-2% and 10-25%, respectively, and all animals received feed that contained cholesterol and lard concentration within that range. Blood samples were obtained at baseline, 1 month, 3 months, and 6 months. Less than 1 ml/kg of blood were obtained each time. At the bimonthly blood cholesterol levels tests, levels of total cholesterol, LDL, HDL, VLDL and triglycerides, blood glucose, Lp-PLA₂ and primary bone marrow cells (PBMCs) were tested (see Table 4).

The animal number, selected to justify the minimum requirement for statistical validity were 2 groups of animals per experiment as follows: 1. Control group (n=17); Diabetic and hyperlipidemic; 2. Experimental group (n=20) Diabetic, hyperlipidemic receiving 10 mg/kg Lp-PLA₂ inhibitor, as shown in Table 3.

Domestic farm pigs, Yorkshire boars, ranging in weight between 25-35 kg were purchased from a local farm and placed in indoor housing under the care of a veterinarian. They were castrated 3-5 days in advance of the study start date. Test pigs were made diabetic by infusing one dose of streptozocin (125 mg/kg) IV in a period of 30 min. If animals do not become diabetic a second dose of (50 mg/kg) was administered. To avoid the possible onset of initial hypoglycemia, 20 g of glucose powder was added to the feed for the first 2. The blood glucose was measured using a glucometer every day before feeding for the first 14 days and then once a week.

Test animals were housed separately from control animals to avoid inter-animal transfer of drug due to colcophagia. All animals were fed an atherogenic diet twice daily with free access to water. The custom-made diet contained 0.5 and 2% cholesterol and 10 and 25% lard, the components of which are shown in Table 1.

**Table 3. Schedule of animals and procedures (divided into 2 groups):**

| | Animal number | Timeline |
|---|---|---|
| Group 1: DM/HC | N=20 | 7 months |
| Group 2: DM/HC receiving LP-PLA₂ inhibitors | N=17 | 7 months |
| Total | N=37 | 7 months |

**Table 4. Summary of Proceedures**

| | **Start** | **28 d** | **57 d** | **85 d** | **113 d** | **141 d** | **168 d** | **196 d** |
|---|---|---|---|---|---|---|---|---|
| **Serum Glucose, LDL, HDL, Triglycerides** | X | X | X | X | X | X | X | X |
| **Lp-PLA₂ (frozen serum-EDTA)** | X | X | X | X | X | X | X | X |
| **PBMCs** | X | X | X | | X | | | X |
| **Tissue harvest** | | | | | | | | X |

Daily dosing began on Day 29, at which time each test animal was given a daily dose of 10 mg/kg SB-480848 (given as bolus equivalent in dog food). Animals were NPO from midnight the night before. Animals were euthanized on Day 196 and tissues harvested immediately.

*Brain tissue preparation.* The entire brain was removed within one hour after animals were euthanized. The right hemisphere was fixed in 10% formalin for at least one week. The brain tissues were then sliced and embedded in paraffin. From each animal, a total of ~11 tissue blocks were generated (see illustration) for immunohistochemistry (IHC). From the left hemisphere, the cerebral cortex (from multiples locations), hippocampus and brain stem were preserved for RNA and protein analyses.

*Post-mortem human brain tissue used to compare with pig brain tissue.* The hippocampus and entorhinal cortex from patients with clinically diagnosed, sporadic AD (n=21, age range=72-84) and control tissues from normal, age-matched, neurologically normal individuals (n=13, age range=69-81) were obtained from the Harvard Brain Tissue Resource Center (Belmont, MA) and the Cooperative Human Tissue Network (Philadelphia, PA). Post-mortem intervals for these brains were <24 h and pathological confirmation of AD for each brain specimen was carried out according to the criteria defined by the National Institute on Aging and the Reagan Institute Working Group on Diagnostic Criteria for the Neuropathological Assessment of AD (1997). Brain specimens from age-matched controls were screened using the same criteria. Tissues were characterized immunohistochemically for the presence of neuritic (amyloid) plaques and neurofibrillary tangles as described previously (D'Andrea et al., 2001; Nagele et al., 2002). Control tissues exhibited no gross pathology and minimal localized microscopic AD-like neuropathology. Tissues were processed for routine paraffin embedding and sectioning according to established protocols. Five-micrometer sections were serially cut, mounted onto SuperFrost Plus+ microslides (Fisher Scientific, Pittsburgh, PA), and dried overnight.

*Histology and Immunohistochemistry (IHC) of Human and Pig brain tissue.* Immunohistochemistry was carried out on paraffin-embedded tissues as described previously (D'Andrea et al., 2001; Nagele et al., 2002). Briefly, after removal of paraffin with xylene and rehydration through a graded series of decreasing concentrations of ethanol, protein antigenicity was enhanced by microwaving sections in Target Buffer (Dako, Carpenturia, CA) for 2 min. Alternatively, frozen sections were treated for 2 min in acetone, and again air-dried. Following a 30 min incubation in 0.3% H₂O₂, sections were treated for 30 min in normal blocking serum and then incubated with primary antibodies at appropriate dilutions for 1 h at room temperature. Following a thorough rinse in PBS, a secondary biotin-labeled antibody was applied for 30 min. Immunoreactions were treated with the avidin-peroxidase-labeled biotin complex (ABC, Vector Labs, Foster City, CA) and visualized by treatment of sections with 3-3-diaminobenzidine-4 HCl (DAB)/H₂O₂ (Biomeda, Foster City, CA). Sections were lightly counterstained with hematoxylin, dehydrated through a graded series of increasing concentrations of ethanols, cleared in xylene and mounted in Permount. Controls consisted of brain sections treated with either non-immune serum, pre-absorbed antibody or omission of the primary antibody. Specimens were examined and photographed with a Nikon FXA microscope, and digital images were recorded using a Nikon DXM1200F digital camera and processed using Image Pro Plus (Phase 3 Imaging, Glen Mills, PA) imaging software.

Serial histological sections from frontal cortex were stained as follows: Hematoxylin and eosin (H&E) to reveal vascular structure and hemorrhage; IHC to reveal BBB leakage (pig immunoglobulin as a marker for vascular leakage and BBB permeability), activation of astrocytes (glial fibrillary acidic protein) and the morphological appearance and structural integrity of neurons (microtubule-associated protein-2), Amyloid plaque (Abeta-42).

### EXAMPLE 1

### Inhibition of Lp-PLA₂ Activity Prevents Vascular Dementia & Alzheimer's Disease: DM/HC Induced Intracerebral Hemorrhage and Blood Brain Barrier (BBB) Compromise.

The primary function of the BBB is to maintain precise control over substances that enter or leave the brain so that the environment in which neurons function remains homeostatic. If the BBB is compromised, blood components that are normally excluded could enter the brain tissue, interfere with neuron function, trigger an immune response and elicit neuroinflammation, all of which contribute to vascular dementia and the development of Alzheimer's disease. As shown in Figure 1, in contrast to the control vessel (Left), hemorrhage (Top Right, H&E 200x) and BBB leakage (Bottom Right, IHC for pig immunoglobulin 100x) were noted in DM/HC brain. The black arrows indicate inflammatory cells outside the blood vessel (Top) and perivascular leak clouds (Bottom Right, BV: blood vessel). Additionally, there is increased adhesion of inflammatory cells on the surface of endothelial cells (white arrow, Top Right). IHC was used to (i) detect vascular leaks resulting from a defective BBB and (ii) track the fate of the leaked material in the brain tissue. Similar to what is observed in human Alzheimer's disease (AD) brain, leaked immunoglobulins (Ig) binds to neurons and causes collapse of the dendrite tree, as demonstrated by recoil and twisting of the main dendrite trunks (i.e., "corkscrew dendrite" appearance) (Figure 2).

During the study period, it was observed that animals administered the Lp-PLA₂ inhibitor were more responsive to external stimuli, demonstrated increased activity in the cage, and tended to respond more alertly to feeding and handling as compared to the control non-treated animals. Also, despite similar serum glucose and cholesterol levels, animals treated with the Lp-PLA₂ inhibitor demonstrated an increase in weight as compared to control animals (62.5 kg vs 50.9 kg for control animals) from a baseline of 26.9 kg and 30.3 kg weight respectively. Weight in animals administered the Lp-PLA₂ inhibitor is a direct reflection of their overall well-being, insofar as that more sickly animals (i.e. the control non-treated animals) do not eat. It was observed that inhibition of inflammation by the Lp-PLA₂ inhibitor results in greater well-being and health in the setting of systemic inflammation.

### EXAMPLE 2

### DM/HC Animals Demonstrated Early Stage Alzheimer's Disease Pathology

Activation of astrocytes and intracellular accumulation of Aβ42 within neurons have been recognized as early and key events in the pathogenesis of Alzheimer's disease (AD) and have been well documented in the brains of AD patients. As shown in Figure 3, as short as 7 months after exposure to CV risk factors, DM/HC induced activation of astrocytes, as indicated by elevated expression of glial fibrillary acidic protein (GFAP). Astrocytes exhibiting intense GFAP immunostaining ware seen in the immediate vicinity of neurons showing abnormal morphology. Furthermore, neurons and vascular smooth muscle cells (SMCs) demonstrated an increased immune reactivity with antibodies against Aβ42 (Figure 4). Of note, astrocytes showed negative immune reactivity against Aβ42, demonstrating Aβ42 accumulation occurs relatively selectively in neurons and vascular SMCs. These findings demonstrate that neurons and vascular SMCs are the cells which are most vulnerable to accumulate of Aβ42, which is comparable to what is observed in the brains of patients at early stages of Alzheimer's disease. It is important to note that Aβ42-positive neurons also showed characteristics of sick neurons, with some of them exhibiting "corkscrew dendrite trunks," an indication that the neuron's dendrite tree is in the process of collapsing (Figure 5). In contrast to the Aβ42(+) neurons, the Aβ42-negative neurons appeared healthy.

### EXAMPLE 3

### Inhibition of Lp-PLA₂ Activity Diminished DM/HC-induced BBB Leakage

As shown in Figure 5, DM/HC induced widespread BBB leakage in all 6 animals, whereas animals treated with Lp-PLA₂ inhibitor showed negligible BBB leakage. Brown stain indicates blood components (in this case Ig) have leaked out from small arteries into the surrounding brain tissue and extensive Ig binding to neurons. By contrast, in treated animals, there is no Ig leakage from blood vessels, no Ig binding to neurons, and no dendrite collapse (Figure 5 & Figure 6).

### EXAMPLE 4

### Inhibition of Lp-PLA₂ Activity Prevented DM/HC-induced Abeta-42 Accumulation in Neurons and Minimized Cerebrovascular Amyloidosis.

DM/HC animals demonstrated variable degree of Abeta 42 accumulation within neurons as well as neuron damage (Figure 7, left panel), whereas animals treated with Lp-PLA₂ inhibitor (Figure 7, right panel) showed no neuronal Abeta-42 immunoreactivity. Additionally, sick neurons were rarely observed in treated animals compared with non-treated animals. Similarly, Lp-PLA2 inhibitor also prevented Abeta42 accumulation in vascular SMCs and thus minimized cerebrovascular amyloidosis (Figure 8).

In summary, inhibition of Lp-PLA₂ prevents metabolic risk factors-induced BBB breakdown, Ig binding to neurons and Abeta42 accumulation in neurons. Inhibition of Lp-PLA₂ also reduced activated glia cells (for example astrocyte activation) and minimized the deposition of Abeta42 in cerebral blood vessels (i.e. cerebrovascular amyloidosis).

## Claims

1. A pharmaceutical composition comprising 1-(N-(2-(diethylamino)ethyl)-N-(4-(4-trifluoromethylphenyl)benzyl)-aminocarbonylmethyl)-2-(4-fluorobenzyl)thio-5,6-trimethylenepyrimidin-4-one (SB480848) or a pharmaceutically acceptable salt thereof, and a pharmaceutically acceptable carrier for use in the treatment and/or prevention of a neurodegenerative disorder or disease, wherein the neurodegenerative disorder is Vascular Dementia.

2. The pharmaceutical composition for use in the treatment according to claim 1, wherein the neurodegenerative disorder or disease is associated with an abnormal blood brain barrier.

3. The pharmaceutical composition for use in the treatment according to claim 2, wherein the abnormal blood brain barrier is a permeable blood brain barrier.

4. The pharmaceutical composition for use in the treatment according to any previous claim, wherein the Vascular Dementia comprises vascular mild cognitive impairment, multi-infarct dementia, vascular dementia due to a strategic single infarct, vascular dementia due to hemorrhagic lesions, small vessel disease, mixed Alzheimer's Disease with vascular dementia or subcortical ischemic vascular dementia.

5. The pharmaceutical composition for use in the treatment according to claim 1, wherein the treatment and/or prevention of the neurodegenerative disorder or disease further comprises the use of additional therapeutic agents selected from the group consisting of: donepezil; tacrine; rivastigmine; galantamine; anti-amyloid vaccine; and Abeta-lowering therapies.

6. The pharmaceutical composition for use in the treatment according to claim 1, wherein the treatment and/or prevention of the neurodegenerative disorder or disease further comprises the use of additional therapeutic agents selected from the group consisting of: M1 muscarinic receptor agonists or allosteric modulators; M2 muscarinic antagonists; acetylcholinesterase inhibitors including tetrahydroaminoacridine, donepezil, galantamine and rivastigmine; nicotinic receptor agonists or allosteric modulators including α7 agonists or allosteric modulators or α4β2 agonists or allosteric modulators; peroxisome proliferator-activated receptors (PPAR) agonists including PPARγ agonists; 5-HT4 receptor partial agonists; histamine H3 antagonists and inverse agonists; 5-HT6 receptor antagonists or 5HT1A receptor antagonists; AMPA positive modulators including alpha-amino-3-hydroxy-5-methylisoxazole-4-propionate, a glutamate receptor subtype; and N-methyl-D-aspartic acid (NMDA) receptor antagonists or modulators including memantine.

## Patentansprüche

1. Pharmazeutische Zusammensetzung, die 1-(N-(2-(Diethylamino)ethyl)-N-(4-(4-trifluormethyphenyl)benzyl)-aminocarbonylmethyl)-2-(4-fluorbenzyl)thio-5,6-trimethylenpyrimidin-4-on (SB480848) oder ein pharmazeutisch geeignetes Salz davon und einen pharmazeutisch geeigneten Träger umfasst, zur Verwendung bei der Behandlung und/oder Vorbeugung einer neurodegenerativen Störung oder Erkrankung, wobei die neurodegenerative Störung vaskuläre Demenz ist.

2. Pharmazeutische Zusammensetzung zur Verwendung bei der Behandlung nach Anspruch 1, wobei die neurodegenerative Störung oder Erkrankung mit einer abnormen Blut-Hirn-Schranke verknüpft ist.

3. Pharmazeutische Zusammensetzung zur Verwendung bei der Behandlung nach Anspruch 2, wobei die abnorme Blut-Hirn-Schranke eine durchlässige Blut-Hirn-Schranke ist.

4. Pharmazeutische Zusammensetzung zur Verwendung bei der Behandlung nach einem der vorhergehenden Ansprüche, wobei die vaskuläre Demenz vaskuläre leichte kognitive Beeinträchtigung, Multiinfarktdemenz, vaskuläre Demenz auf Grund eines strategischen Einzelinfarkts, vaskuläre Demenz auf Grund von hämorrhagischen Verletzungen, Mikroangiopathie, Alzheimer-Krankheit, gemischt mit vaskulärer Demenz, oder subkortikale ischämische vaskuläre Demenz umfasst.

5. Pharmazeutische Zusammensetzung zur Verwendung bei der Behandlung nach Anspruch 1, wobei die Behandlung und/oder Vorbeugung der neurodegenerativen Störung oder Erkrankung ferner die Verwendung von zusätzlichen therapeutischen Wirkstoffen umfasst, die ausgewählt sind aus der Gruppe, die als Folgendem besteht: Donepezil, Tacrin, Rivastigmin, Galantamin, Anti-Amyloid-Vakzinen und Abeta-Senkungstherapien.

6. Pharmazeutische Zusammensetzung zur Verwendung bei der Behandlung nach Anspruch 1, wobei die Behandlung und/oder Vorbeugung der neurodegenerativen Störung oder Erkrankung ferner die Verwendung von zusätzlichen therapeutischen Wirkstoffen umfasst, die ausgewählt sind aus der Gruppe, die als Folgendem besteht: M1-Muscarinrezeptorantagonisten oder allosterischen -Modulatoren, M2-Muscarinantagonisten, Acetylcholinesterasehemmern einschließlich von Tetrahydroaminoacridin, Donepezil, Galantamin und Rivastigmin, Nikotinrezeptoragonisten oder allosterischen -Modulatoren einschließlich von α7-Agonisten oder allosterischen -Modulatoren oder α4β2-Agonsiten oder allosterischen -Modulatoren, Agonisten von peroxisomproliferatoraktivierten Rezeptoren (PPAR) einschließlich von PPARγ-Agonisten, 5-HT4-Rezeptorpartialagonisten, Histamin-H3-Antagonisten und -Inversagonisten, 5-HT6-Rezeptorantagonisten oder 5HT1A-Rezeptorantagonisten, AMPA-Positivmodulatoren einschließlich von alpha-Amino-3-hydroxy-5-methylisoaxol-4-propionat, einem Glutamatrezeptor-Untertyp, und N-Methyl-D-Asparaginsäure- (NMDA-) Rezeptorantagonisten oder -Modulatoren einschließlich von Memantin.

## Revendications

1. Composition pharmaceutique comprenant la 1-(N-(2-(diéthylamino)éthyl)-N-(4-(4-trifluorométhylphényl)benzyl)-aminocarbonylméthyl)-2-(4-fluorobenzyl)thio-5,6-triméthylènepyrimidin-4-one (SB480848) ou sel pharmaceutiquement acceptable de celle-ci, et vecteur pharmaceutiquement acceptable, destinés à une utilisation dans le traitement et/ou la prévention d'une maladie ou d'un trouble neurodégénératif, le trouble neurodégénératif étant la démence vasculaire.

2. Composition pharmaceutique destinée à une utilisation dans le traitement selon la revendication 1, dans laquelle la maladie ou le trouble neurodégénératif est associé à une barrière hématoencéphalique anormale.

3. Composition pharmaceutique destinée à une utilisation dans le traitement selon la revendication 2, dans laquelle la barrière hématoencéphalique anormale est une barrière hématoencéphalique perméable.

4. Composition pharmaceutique destinée à une utilisation dans le traitement selon l'une quelconque des revendications précédentes, dans laquelle la démence vasculaire comprend un trouble cognitif vasculaire léger, la démence artériopathique, la démence vasculaire due à un seul infarctus stratégique, la démence vasculaire due à des lésions hémorragiques, la microangiopathie, la maladie d'Alzheimer mixte avec démence vasculaire ou la démence vasculaire ischémique sous-corticale.

5. Composition pharmaceutique destinée à une utilisation dans le traitement selon la revendication 1, dans laquelle le traitement et/ou la prévention de la maladie ou du trouble neurodégénératif comprend en outre l'utilisation d'agents thérapeutiques additionnels choisis dans le groupe consistant en : le donépézil ; la tacrine ; la rivastigmine ; la galantamine ; le vaccin antiamyloïde ; et les thérapies abaissant l'Abêta.

6. Composition pharmaceutique destinée à une utilisation dans le traitement selon la revendication 1, dans laquelle le traitement et/ou la prévention de la maladie ou du trouble neurodégénératif comprend en outre l'utilisation d'agents thérapeutiques additionnels choisis dans le groupe consistant en : les agonistes ou les modulateurs allostériques du récepteur muscarinique M1 ; les antagonistes muscariniques M2 ; les inhibiteurs de l'acétylcholinestérase y compris la tétrahydroaminoacridine, le donépézil, la galantamine et la rivastigmine ; les agonistes ou les modulateurs allostériques du récepteur nicotinique y compris les agonistes ou les modulateurs allostériques d'α7 ou les agonistes ou les modulateurs allostériques d'α4β2 ; les agonistes des récepteurs activés par les proliférateurs des peroxysomes (PPAR) y compris les agonistes du PPARγ ; les agonistes partiels du récepteur de la 5-HT4 ; les antagonistes et les agonistes inverses à l'histamine H3 ; les antagonistes au récepteur de la 5-HT6 ou les antagonistes au récepteur de la 5HT1A ; les modulateurs positifs d'AMPA y compris l'alpha-amino-3-hydroxy-5-méthylisoxazole-4-propionate, un sous-type du récepteur du glutamate ; et les antagonistes ou les modulateurs du récepteur de l'acide N-méthyl-D-aspartique (NMDA) y compris la mémantine.
